# EUROPÄISCHE PATENTSCHRIFT

(11) **EP 0 664 797 B1**
(45) Veröffentlichungstag und Bekanntmachung des Hinweises auf die Patenterteilung: **16.04.1997**
(21) Anmeldenummer: 93921910.1
(22) Anmeldetag: 04.10.1993
(51) Int. Cl.: C07D 249/12, A01N 47/38, C07D 249/14, C07F 9/6518, A01N 57/32

(54) **HERBIZIDE SULFONYLAMINOCARBONYTRIAZOLINONE**
SULPHONYL AMINO CARBONYL TRIAZOLINONES
SULFONYLAMINOCARBONYLTRAZOLINONES UTILISEES COMME HERBICIDES

(30) Priorität: 15.10.1992 DE 4234801
(43) Veröffentlichungstag der Anmeldung: 02.08.1995
(73) Patentinhaber: BAYER AG, 51368 Leverkusen (DE)
(72) Erfinder: FINDEISEN, Kurt, D-51375 Leverkusen (DE); LINKER, Karl-Heinz, D-51377 Leverkusen (DE); KLUTH, Joachim, D-40764 Langenfeld (DE); MÜLLER, Klaus-Helmut, D-40593 Düsseldorf (DE); RIEBEL, Hans-Jochem, D-42113 Wuppertal (DE); KÖNIG, Klaus, D-51519 Odenthal (DE); SANTEL, Hans-Joachim, D-51371 Leverkusen (DE); SCHMIDT, Robert, R., D-51467 Bergisch Gladbach (DE)
(86) Internationale Anmeldenummer: EP9302706
(87) Internationale Veröffentlichungsnummer: WO9408979

(56) Entgegenhaltungen:
- EP-A- 0 341 489

## Beschreibung

Die Erfindung betrifft neue Sulfonylaminocarbonyltriazolinone, mehrere Verfahren und neue Zwischenprodukte zu ihrer Herstellung und ihre Verwendung als Herbizide.

Es ist bereits bekannt, daß bestimmte Sulfonylaminocarbonyltriazolinone, wie z.B. 4,5-Dimethyl-2-(2-chlor-phenylsulfonyl-aminocarbonyl)-2,4-dihydro-3H-1,2,4-triazol-3-on, herbizide Eigenschaften aufweisen (vgl. EP-A 341489; vgl. auch EP-A 422469; EP-A 425948; EP-A 431291). Die Wirkung dieser Verbindungen ist jedoch nicht in allen Belangen zufriedenstellend.

Es wurden nun die neuen Sulfonylaminocarbonyltriazolinone der allgemeinen Formel (I), 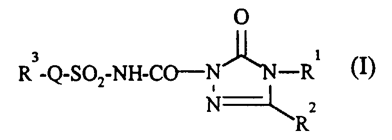 in welcher
- Q: für Sauerstoff, Schwefel oder eine der nachstehenden Gruppierungen steht 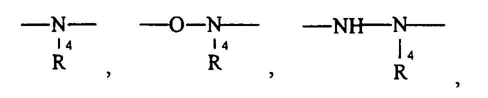
- R¹: für Wasserstoff, Hydroxy, Amino, Alkylidenamino oder für einen jeweils gegebenenfalls substituierten Rest aus der Reihe Alkyl, Alkenyl, Alkinyl, Cycloalkyl, Aralkyl, Aryl, Alkoxy, Alkenyloxy, Alkylamino, Cycloalkylamino, Dialkylamino, Alkanoylamino, Alkoxycarbonylamino steht,
- R²: für Wasserstoff, Hydroxy, Mercapto, Amino, Halogen oder für einen jeweils gegebenenfalls substituierten Rest aus der Reihe Alkyl, Cycloalkyl, Alkenyl, Cycloalkenyl, Aralkyl, Aryl, Alkoxy, Alkenyloxy, Alkinyloxy, Cycloalkyloxy, Aryloxy, Aralkyloxy, Alkylthio, Alkylsulfinyl, Alkylsulfonyl, Alkenylthio, Alkinylthio, Cycloalkylthio, Arylthio, Aralkylthio, Alkylamino, Alkenylamino, Arylamino, Aralkylamino, Dialkylamino, Aziridino, Pyrrolidino, Piperidino oder Morpholino steht,
- R³: für einen jeweils gegebenenfalls substituierten Rest aus der Reihe Alkyl, Alkenyl, Alkinyl, Cycloalkyl, Cycloalkylalkyl, Alkylsulfonyl, Alkylaminosulfonyl, Dialkylaminosulfonyl, Aryl, Aralkyl steht,
R⁴ für Wasserstoff, Hydroxy, Amino, Cyano, Alkoxycarbonyl, für jeweils gegebenenfalls substituiertes Alkyl, Alkenyl, Alkinyl, Alkoxy, Alkenyloxy, Aralkoxy, Aryloxy oder Dialkoxy(thio)phosphoryl steht oder für die nachstehende Gruppierung Q¹-R⁵ steht, worin
Q¹ für -CO- oder -SO₂- steht und
R⁵ für jeweils gegebenenfalls substituiertes Alkyl, Cycloalkyl oder Aryl steht,
sowie Salze von Verbindungen der Formel (I) gefunden.

Man erhält die neuen Sulfonylaminocarbonyltriazolinone der Formel (I), wenn man
(a) Chlorsulfonylaminocarbonyltriazolinone der allgemeinen Formel (II) 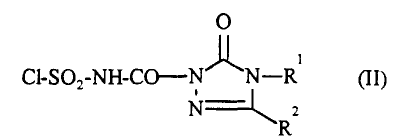 in welcher
   - R¹ und R²: die oben angegebene Bedeutung haben,
   mit nucleophilen Verbindungen der allgemeinen Formel (III)

   R³-Q-H (III)

   in welcher
   - Q und R³: die oben angegebene Bedeutung haben,
   gegebenenfalls in Gegenwart eines Säureakzeptors und gegebenenfalls in Gegenwart eines Verdünnungsmittels umsetzt,
   oder wenn man
(b) Triazolinone der allgemeinen Formel (IV) 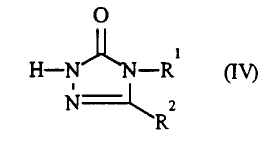 in welcher
   - R¹ und R²: die oben angegebene Bedeutung haben,

   mit Chlorsulfonylisocyanat, gegebenenfalls in Gegenwart eines Verdünnungsmittels umsetzt und die hierbei gebildeten Chlorsulfonylaminocarbonyltriazolinone der Formel (II) - oben - ohne Zwischenisolierung mit nucleophilen Verbindungen der allgemeinen Formel (III) - oben - gegebenenfalls in Gegenwart eines Säureakzeptors und gegebenenfalls in Gegenwart eines Verdünnungsmittels umsetzt,
   und gegebenenfalls die nach Verfahren (a) oder (b) erhaltenen Verbindungen der Formel (I) nach üblichen Methoden in Salze überführt.
   Weitere mögliche Herstellungsmethoden für die erfindungsgemäßen Verbindungen der Formel (I) sind nachstehend aufgeführt, wobei jeweils Q, R¹, R² und R³ die oben angegebene Bedeutung haben:
(c) Umsetzung von Isocyanaten der Formel (V) mit Triazolinonen der Formel (IV): 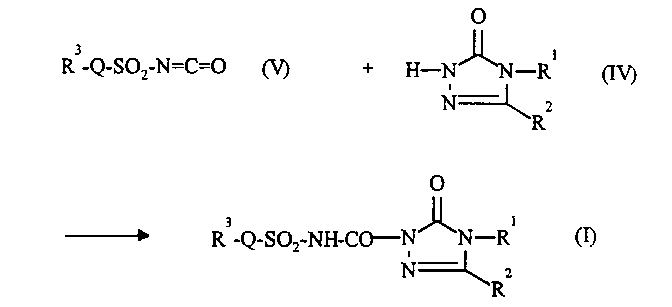
(d) Umsetzung von Aminosulfonylverbindungen der Formel (VI) mit Oxycarbonyltriazolinonen der Formel (VII) (R: Alkyl, Aralkyl, Aryl): 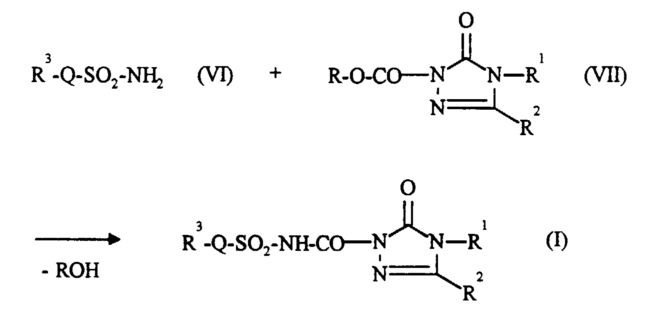
(e) Umsetzung von Sulfonylurethanen der Formel (VIII) mit Triazolinonen der Formel (IV) (R: Alkyl, Aralkyl, Aryl):

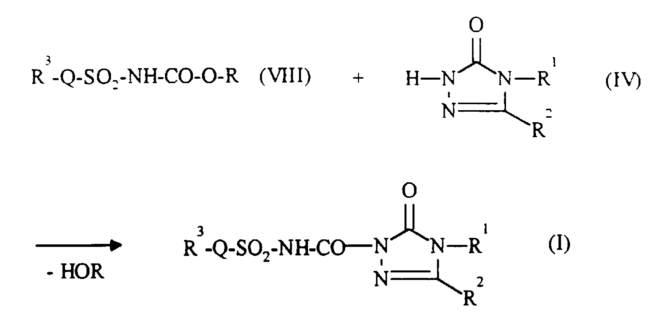

Die neuen Sulfonylaminocarbonyltriazolinone der allgemeinen Formel (I) zeichnen sich durch starke herbizide Wirksamkeit aus.

Überraschenderweise zeigen die neuen Verbindungen der Formel (I) erheblich stärkere herbizide Wirkung als das strukturell ähnliche bekannte 4,5-Dimethyl-2-(2-chlorphenylsulfonyl-aminocarbonyl)-2,4-dihydro-3H-1,2,4-triazol-3-on.

Gegenstand der Erfindung sind vorzugsweise Verbindungen der Formel (I), in welcher
- Q: für Sauerstoff Schwefel oder eine der nachstehenden Gruppierungen steht 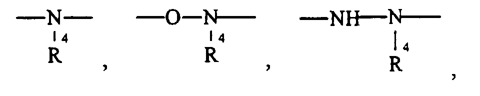
- R¹: für Wasserstoff, Hydroxy, Amino, für C₂-C₁₀-Alkylidenamino, für gegebenenfalls durch Fluor, Chlor, Brom, Cyano, C₁-C₄-Alkoxy, C₁-C₄-Alkylcarbonyl oder C₁-C₄-Alkoxy-carbonyl substituiertes C₁-C₆-Alkyl, für jeweils gegebenenfalls durch Fluor, Chlor und/oder Brom substituiertes C₃-C₆-Alkenyl oder C₃-C₆-Alkinyl, für gegebenenfalls durch Fluor, Chlor, Brom und/oder C₁-C₄-Alkyl substituiertes C₃-C₆-Cycloalkyl, für gegebenenfalls durch Fluor, Chlor, Brom, Cyano, Nitro, C₁-C₄-Alkyl, Trifluormethyl, C₁-C₄-Alkoxy und/oder C₁-C₄-Alkoxy-carbonyl substituiertes Phenyl-C₁-C₃-alkyl, für gegebenenfalls durch Fluor, Chlor, Brom, Cyano, Nitro, C₁-C₄-Akyl, Trifluormethyl, C₁-C₄-Alkoxy, Fluor- und/oder Chlor-substituiertes C₁-C₃-Alkoxy, C₁-C₄-Alkylthio, Fluor- und/oder Chlor-substituiertes C₁-C₃-Alkylthio, C₁-C₄-Alkylsulfinyl, C₁-C₄-Alkylsulfonyl und/oder C₁-C₄-Alkoxy-carbonyl substituiertes Phenyl, für gegebenenfalls durch Fluor, Chlor, Cyano, Phenyl, C₁-C₄-Alkoxy oder C₁-C₄-Alkoxy-carbonyl substituiertes C₁-C₆-Alkoxy, für C₃-C₆-Alkenyloxy, für gegebenenfalls durch Fluor, Cyano, C₁-C₄-Alkoxy oder C₁-C₄-Alkoxy-carbonyl substituiertes C₁-C₄-Alkylamino, C₃-C₆-Cycloalkylamino oder Di-(C₁-C₄-alkyl)-amino, für C₁-C₆-Alkanoylamino oder für C₁-C₆-Alkoxycarbonylamino steht,
- R²: für Wasserstoff, Hydroxy, Mercapto, Amino, Halogen, für gegebenenfalls durch Fluor, Chlor, Brom Cyano, C₃-C₆-Cycloalkyl, C₁-C₄-Alkoxy, C₁-C₄-Alkylthio oder C₁-C₄-Alkoxy-carbonyl substituiertes C₁-C₆-Alkyl oder C₂-C₆-Alkenyl, für gegebenenfalls durch Fluor, Chlor, Brom und/oder C₁-C₄-Alkyl substituiertes C₃-C₆-Cycloalkyl, für Cyclohexenyl, für gegebenenfalls durch Fluor, Chlor, Brom, Cyano, Nitro, C₁-C₄-Alkyl, Trifluormethyl, C₁-C₄-Alkoxy und/oder C₁-C₄-Alkoxy-carbonyl substituiertes Phenyl-C₁-C₃-alkyl, für gegebenenfalls durch Fluor, Chlor, Brom, Cyano, Nitro, C₁-C₄-Alkyl, Trifluormethyl, C₁-C₄-Alkoxy, Fluor- und/oder Chlor-substituiertes C₁-C₃-Alkoxy, C₁-C₄-Alkylthio, Fluor- und/oder Chlor-substituiertes C₁-C₃-Alkylthio, C₁-C₄-Alkyl-sulfinyl, C₁-C₄-Alkylsulfonyl und/oder C₁-C₄-Akoxy-carbonyl substituiertes Phenyl, für gegebenenfalls durch Fluor, Chlor, Cyano, C₁-C₄-Alkoxy oder C₁-C₄-Alkoxy-carbonyl substituiertes C₁-C₆-Alkoxy, für C₃-C₆-Alkenyloxy, C₃-C₆-Alkinyloxy oder C₃-C₆-Cycloalkyloxy, für jeweils gegebenenfalls durch Fluor, Chlor, Brom, Cyano, Nitro, C₁-C₄-Alkyl, Trifluormethyl, C₁-C₄-Alkoxy, Difluormethoxy oder Trifluormethoxy substituiertes Phenoxy oder Benzyloxy, für jeweils gegebenenfalls durch Fluor, Chlor, Cyano, C₁-C₄-Alkoxy oder C₁-C₄-Alkoxy-carbonyl substituiertes C₁-C₆-Alkylthio, C₁-C₆-Alkylsulfinyl, C₁-C₆-Akylsulfonyl, C₃-C₆-Alkenylthio, C₃-C₆-Alkinylthio oder C₃-C₆-Cycloalkylthio, für jeweils gegebenenfalls durch Fluor, Chlor, Brom, Cyano, Nitro, C₁-C₄-Alkyl, Trifluormethyl, C₁-C₄-Alkoxy, Difluormethoxy oder Trifluormethoxy substituiertes Phenylthio oder Benzylthio, für C₁-C₆-Alkylamino oder C₃-C₆-Alkenylamino, für jeweils gegebenenfalls durch Fluor, Chlor, Brom, Cyano, Nitro, C₁-C₄-Alkyl, Trifluormethyl, C₁-C₄-Alkoxy, Difluormethoxy oder Trifluormethoxy substituiertes Phenylamino oder Benzylamino, für Di-(C₁-C₄-alkyl)-amino, oder für jeweils gegebenenfalls durch C₁-C₄-Alkyl substituiertes Aziridino, Pyrrolidino oder Morpholino steht,
- R³: für jeweils gegebenenfalls durch Halogen, Cyano, Carboxy, C₁-C₄-Alkoxy, C₁-C₄-Alkylthio, C₁-C₄-Akylsulfinyl, C₁-C₄-Alkylsulfonyl oder C₁-C₄-Alkoxycarbonyl substituiertes C₁-C₆-Alkyl, C₃-C₆-Alkenyl oder C₃-C₆-Alkinyl, für jeweils gegebenenfalls durch Halogen, Cyano, Carboxy, C₁-C₄-Alkyl, C₁-C₄-Halogenalkyl oder C₁-C₄-Alkoxy-carbonyl substituiertes C₃-C₆-Cycloalkyl oder C₃-C₆-Cycloalkyl-C₁-C₄-alkyl, für jeweils gegebenenfalls durch Halogen substituiertes C₁-C₆-Alkylsulfonyl, C₁-C₆-Alkylaminosulfonyl oder Di-(C₁-C₄-alkyl)aminosulfonyl, für jeweils gegebenenfalls durch Halogen, Cyano, Carboxy, Nitro, durch C₁-C₄-Alkyl, C₁-C₄-Alkoxy, C₁-C₄-Alkylthio, C₁-C₄-Alkylsulfinyl oder C₁-C₄-Alkylsulfonyl (welche jeweils gegebenenfalls durch Halogen, Cyano, C₁-C₄-Alkoxy oder C₁-C₄-Alkoxy-carbonyl substituiert sind), durch C₁-C₄-Alkylsulfonyloxy, C₁-C₄-Alkylaminosulfonyloxy, Di-(C₁-C₄-alkyl)aminosulfonyloxy, C₁-C₄-Halogenalkylsulfonyloxy, Di-(C₁-C₄-alkyl)-aminocarbonyl oder C₁-C₄-Alkylaminocarbonyloxy, durch C₃-C₆-Cycloalkyl oder C₃-C₆-Cycloalkyl-C₁-C₄-alkyl (welche jeweils gegebenenfalls durch Halogen oder C₁-C₄-Alkyl substituiert sind), durch Phenyl, Phenoxy, Phenylthio, Phenylsulfinyl, Phenylsulfonyl, Phenylsulfonyloxy, Phenylamino, Phenylcarbonyl oder Phenyl-C₁-C₄-alkyl (welche jeweils gegebenenfalls durch Halogen, Cyano, Nitro, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkoxy-carbonyl oder C₁-C₄-Alkoxy-amino substituiert sind), durch C₁-C₄-Alkyl-carbonyl, C₃-C₆-Cycloalkylcarbonyl oder C₁-C₄-Alkoxy-carbonyl (welche jeweils gegebenenfalls durch Halogen, C₃-C₆-Cycloalkyl oder C₁-C₄-Alkoxy substituiert sind) substituiertes Phenyl, Naphthyl, Tetralinyl, Phenyl-C₁-C₄-alkyl, Naphthyl-C₁-C₄-alkyl oder Tetralinyl-C₁-C₄-alkyl steht,
- R⁴: für Wasserstoff, Hydroxy, Amino, Cyano, für jeweils gegebenenfalls durch Halogen, Cyano, Carboxy, C₁-C₄-Alkoxy, C₁-C₄-Akylthio, C₁-C₄-Alkylsulfinyl, C₁-C₄-Alkylsulfonyl, C₁-C₄-Alkylamino, Di-(C₁-C₄-Alkyl)-amino oder C₁-C₄-Alkoxy-carbonyl substituiertes C₁-C₆-Alkyl, C₃-C₆-Alkenyl oder C₃-C₆-Alkinyl, für C₁-C₄-Alkoxy-carbonyl, für C₁-C₆-Alkoxy, C₃-C₆-Alkenyloxy, Benzyloxy, Phenoxy oder Di-(C₁-C₄-alkoxy)-(thio)phosphoryl steht oder für die nachstehende Gruppierung Q¹-R⁵ steht, worin
Q¹ für -CO- oder -SO₂- steht und
R⁵ für gegebenenfalls durch Halogen oder C₁-C₄-Alkoxy substituiertes C₁-C₆-Alkyl, für gegebenenfalls durch Halogen oder C₁-C₄-Alkyl substituiertes C₃-C₆-Cycloalkyl oder für gegebenenfalls durch Halogen, C₁-C₄-Alkyl, C₁-C₄-Halogenalkyl oder C₁-C₄-Alkoxy substituiertes Phenyl steht.
Gegenstand der Erfindung sind weiter vorzugsweise Natrium-, Kalium-, Magnesium-, Calcium-, Ammonium-, C₁-C₄-Alkyl-ammonium-, Di-(C₁-C₄-alkyl)-ammonium-, Tri-(C₁-C₄-alkyl)-ammonium-, C₅- oder C₆-Cycloalkyl-ammonium- und Di-(C₁-C₂-alkyl)-benzyl-ammonium-Salze von Verbindungen der Formel (I), in welcher Q, R¹, R² und R³ die oben vorzugsweise angegebenen Bedeutungen haben.

Gegenstand der Erfindung sind insbesondere Verbindungen der Formel (I), in welcher
- Q: für Sauerstoff oder die Gruppierung 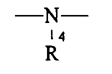 steht,
- R¹: für Wasserstoff, Hydroxy, Amino, für C₃-C₈-Alkylidenamino, für Methyl, Ethyl, n- oder i-Propyl, n-, i-, s- oder t-Butyl, Dichlormethyl, Fluorethyl, Chlorethyl, Difluorethyl, Dichlorethyl, Trifluorethyl, Trichlorethyl, Chlordifluorethyl, Tetrafluorethyl, Cyanomethyl, Cyanoethyl, Methoxymethyl, Ethoxymethyl, Methoxyethyl, Ethoxyethyl, für Allyl, Chlorallyl, Dichlorallyl, Propargyl, für Cyclopropyl, Benzyl oder Phenyl, für Methoxy, Ethoxy, n- oder i-Propoxy, für Allyloxy, n- oder i- oder s-Butoxy, für Methylamino, Ethylamino, n- oder i-Propylamino, für Cyclopropylamino, Dimethylamino, Diethylamino, Acetylamino, Methoxycarbonylamino oder Ethoxycarbonylamino steht,
- R²: für Wasserstoff, Hydroxy, Mercapto, Amino, Fluor, Chlor, Brom, für Methyl, Ethyl, n- oder i-Propyl, n-, i-, s- oder t-Butyl, Difluormethyl, Trifluormethyl, Dichlormethyl, Trichlormethyl, Cyanomethyl, Cyanoethyl, Cyclopropylmethyl, Methoxymethyl, Ethoxymethyl, Methoxyethyl, Ethoxyethyl, Methylthiomethyl, Ethylthiomethyl, Methylthioethyl oder Ethylthioethyl, für Cyclopropyl, Difluorcyclopropyl oder Dichlorcyclopropyl, für Phenyl oder Benzyl, für Methoxy, Ethoxy, n- oder i-Propoxy, für Methoxymethoxy, Ethoxymethoxy, Methoxyethoxy oder Ethoxyethoxy, für Phenoxy oder Benzyloxy, für Methylthio, Ethylthio, n- oder i-Propylthio, Allylthio, Propargylthio, Cyclopropylmethylthio, Methylsulfinyl, Ethylsulfinyl, Methylsulfonyl, Ethylsulfonyl, Phenylthio oder Benzylthio, für Methylamino, Ethylamino, n- oder i-Propylamino, Phenylamino oder Benzylamino, für Dimethylamino, Diethylamino, Dipropylamino, Methylethylamino, Methylpropylamino, für Aziridino oder für jeweils gegebenenfalls durch Methyl oder Ethyl substituiertes Pyrrolidino, Piperidino oder Morpholino steht,
- R³: für jeweils gegebenenfalls durch Fluor, Chlor, Cyano, Methoxy, Ethoxy, Methoxycarbonyl oder Ethoxycarbonyl substituiertes Methyl, Ethyl, n- oder i-Propyl, n-, i-, s- oder t-Butyl, für jeweils gegebenenfalls durch Fluor, Chlor, Cyano, Methyl, Ethyl, Trifluormethyl, Methoxycarbonyl oder Ethoxycarbonyl substituiertes Cyclopropyl, Cyclobutyl, Cyclopentyl, Cyclohexyl, Cyclopropylmethyl, Cyclobutylmethyl, Cyclopentylmethyl, Cyclohexylmethyl, Cyclopropylethyl, Cyclobutylethyl, Cyclopentylethyl oder Cyclohexylethyl, für gegebenenfalls durch Fluor oder Chlor substituiertes Methylsulfonyl, Ethylsulfonyl, Propylsulfonyl oder Butylsulfonyl, für gegebenenfalls durch Fluor, Chlor, Brom, Cyano, Carboxy, Nitro, durch Methyl, Ethyl, Methoxy, Ethoxy, Methylthio, Ethylthio, Methylsulfinyl, Ethylsulfinyl, Methylsulfonyl oder Ethylsulfonyl (welche jeweils gegebenenfalls durch Fluor, Chlor, Cyano, Methoxy, Ethoxy, Methoxycarbonyl oder Ethoxycarbonyl substituiert sind), durch Methylsulfonyloxy, Ethylsulfonyloxy, Methylaminosulfonyloxy, Ethylaminosulfonyloxy, Dimethylaminosulfonyloxy, Diethylaminosulfonyloxy, Trifluormethylsulfonyloxy, Dimethylaminocarbonyl oder Diethylaminocarbonyl, durch Cyclohexyl oder Cyclohexylmethyl (welche jeweils gegebenenfalls durch Chlor, Methyl oder Ethyl substituiert sind) durch Phenyl, Phenoxy, Phenylthio, Phenylsulfonyl, Phenylsulfonyloxy, Phenylamino, Phenylcarbonyl, Phenylmethyl oder Phenylethyl (welche jeweils gegebenenfalls durch Fluor, Chlor, Cyano, Nitro, Methyl, Ethyl, Trifluormethyl, Methoxy, Ethoxy, Difluormethoxy, Trifluormethoxy, Methylthio, Ethylthio, Trifluormethylthio, Methoxycarbonyl oder Ethoxycarbonyl substituiert sind), durch Acetyl, Cyclopropylcarbonyl, Methoxycarbonyl oder Ethoxycarbonyl (welche gegebenenfalls durch Fluor, Chlor, Cyclopentyl, Cyclohexyl, Methoxy oder Ethoxy substituiert sind) substituiertes Phenyl, für jeweils gegebenenfalls durch Fluor, Chlor, Cyano, Nitro, Methyl, Ethyl, Methoxy oder Ethoxy substituiertes Naphthyl oder Tetralinyl, oder für jeweils gegebenenfalls durch Fluor, Chlor, Methyl, Ethyl, Methoxy oder Ethoxy substituiertes Naphthylmethyl, Naphthylethyl, Tetralinylmethyl oder Tetralinylethyl steht,
R⁴ für Wasserstoff, Cyano, Cyanomethyl, Cyanoethyl, Difluormethyl, Methoxymethyl, Ethoxymethyl, Methoxyethyl, Ethoxyethyl, Methoxycarbonylmethyl, Ethoxycarbonylmethyl, Methoxycarbonylethyl, Ethoxycarbonylethyl, Allyl, Propargyl, Methoxycarbonyl, Ethoxycarbonyl, Methoxy, Ethoxy, Allyloxy oder Benzyloxy steht oder für die Gruppierung Q¹-R⁵ steht, worin
Q¹ für -CO- oder -SO₂- steht und
R⁵ für jeweils gegebenenfalls durch Fluor, Chlor, Methoxy oder Ethoxy substituiertes Methyl, Ethyl oder Propyl, für jeweils gegebenenfalls durch Fluor, Chlor, Methyl oder Ethyl substituiertes Cyclopropyl, Cyclobutyl, Cyclopentyl oder Cyclohexyl, oder für gegebenenfalls durch Fluor, Chlor, Brom, Methyl, Ethyl, Trifluormethyl, Methoxy oder Ethoxy substituiertes Phenyl steht.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- bzw. Zwischenprodukte. Diese Restedefinitionen können untereinander, also auch zwischen den angegebenen bevorzugten Bereichen beliebig kombiniert werden.

Die bei den Restedefinitionen genannten Kohlenwasserstoffreste, wie Alkyl, Alkenyl oder Alkinyl, auch in Kombinationen mit Heteroatomen, wie in Alkoxy, Alkylthio oder Alkylamino, sind auch dann, wenn dies nicht ausdrücklich angegeben ist, geradkettig oder verzweigt.

Halogen steht im allgemeinen für Fluor, Chlor, Brom oder Iod, vorzugsweise für Fluor, Chlor oder Brom, insbesondere für Fluor oder Chlor.

Beispiele für die erfindungsgemäßen Verbindungen der Formel (I) sind in der nachstehenden Tabelle 1 aufgeführt.

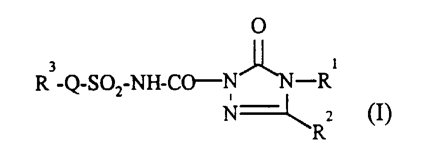

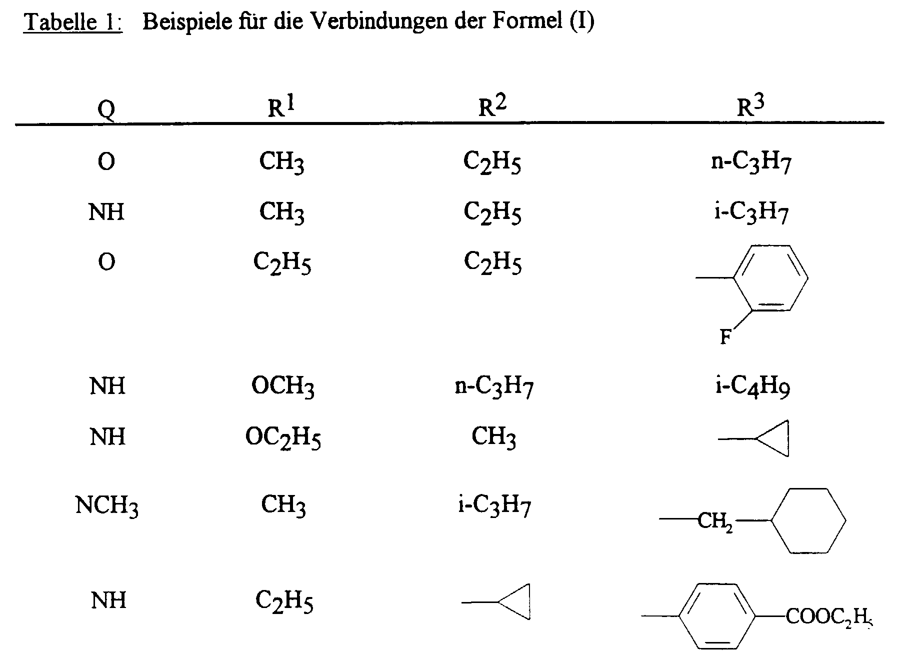

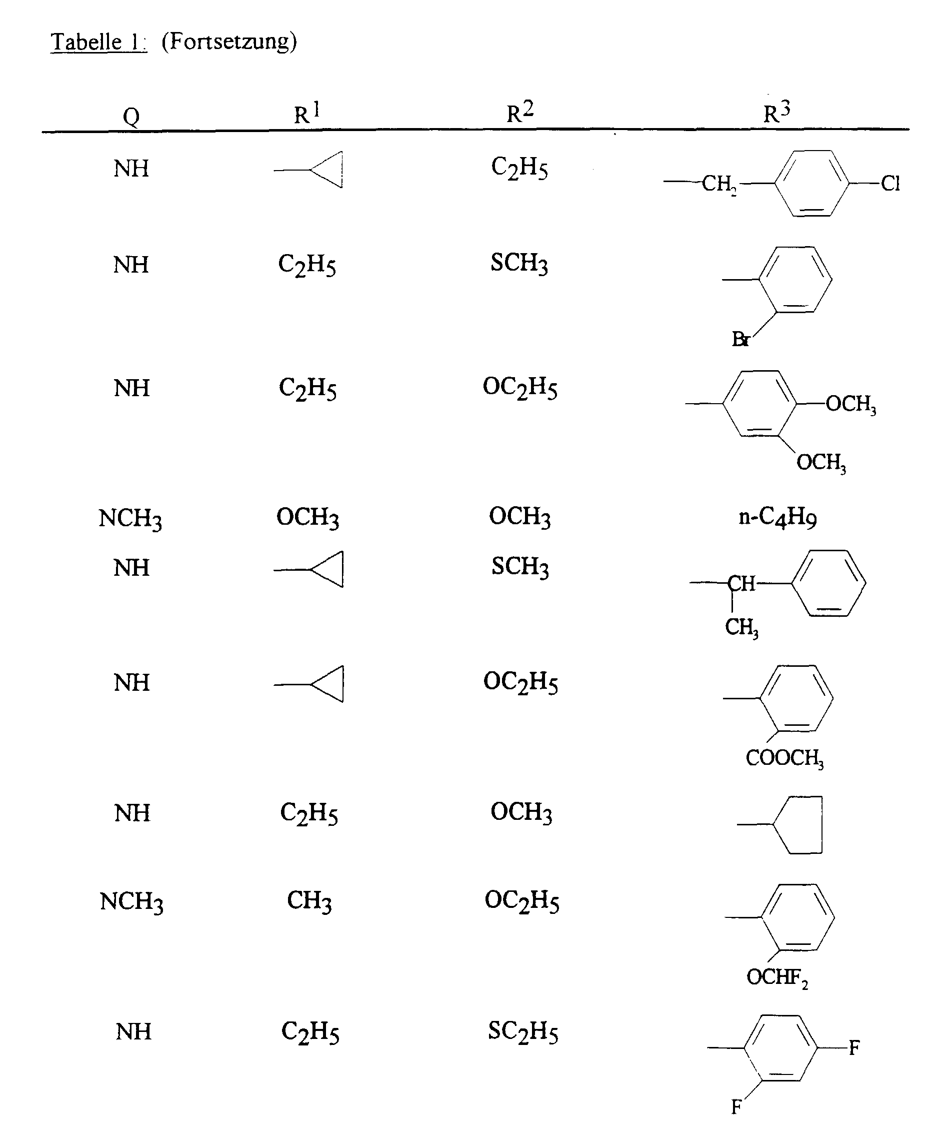

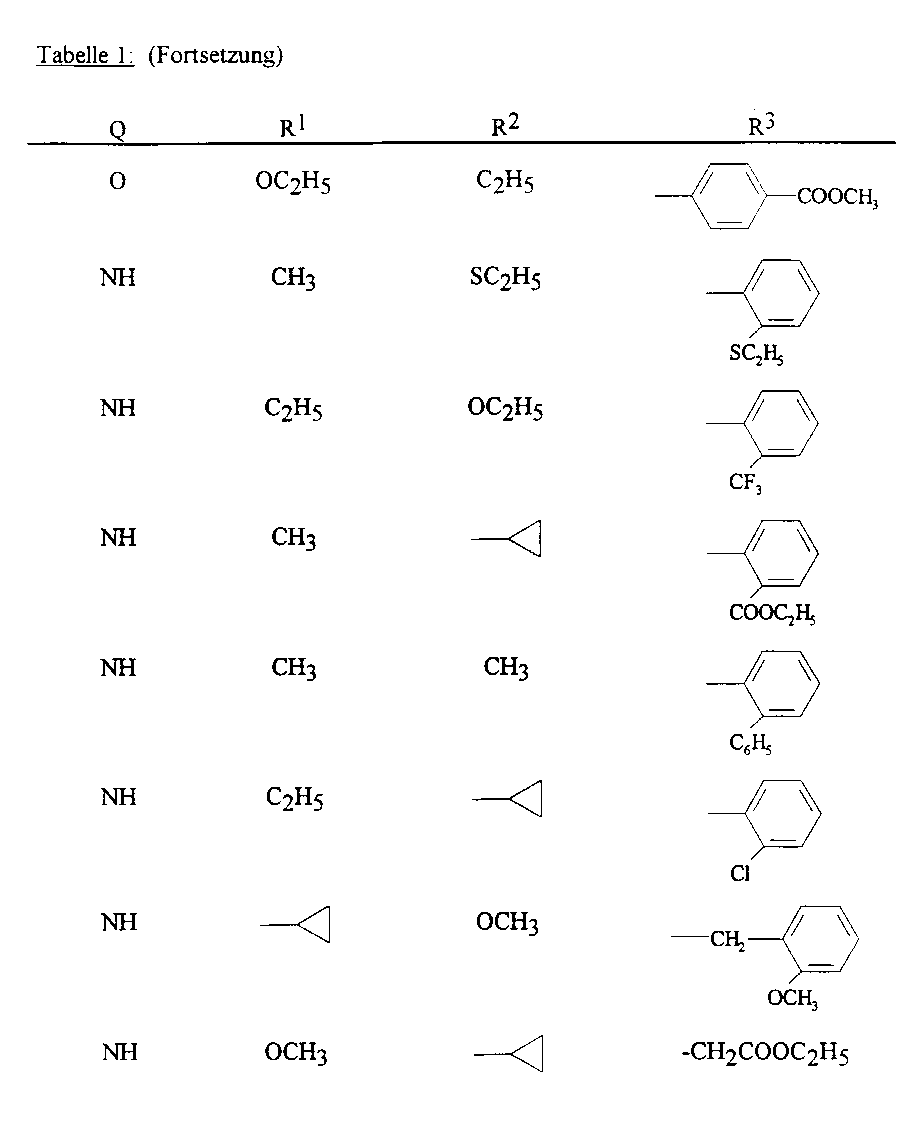

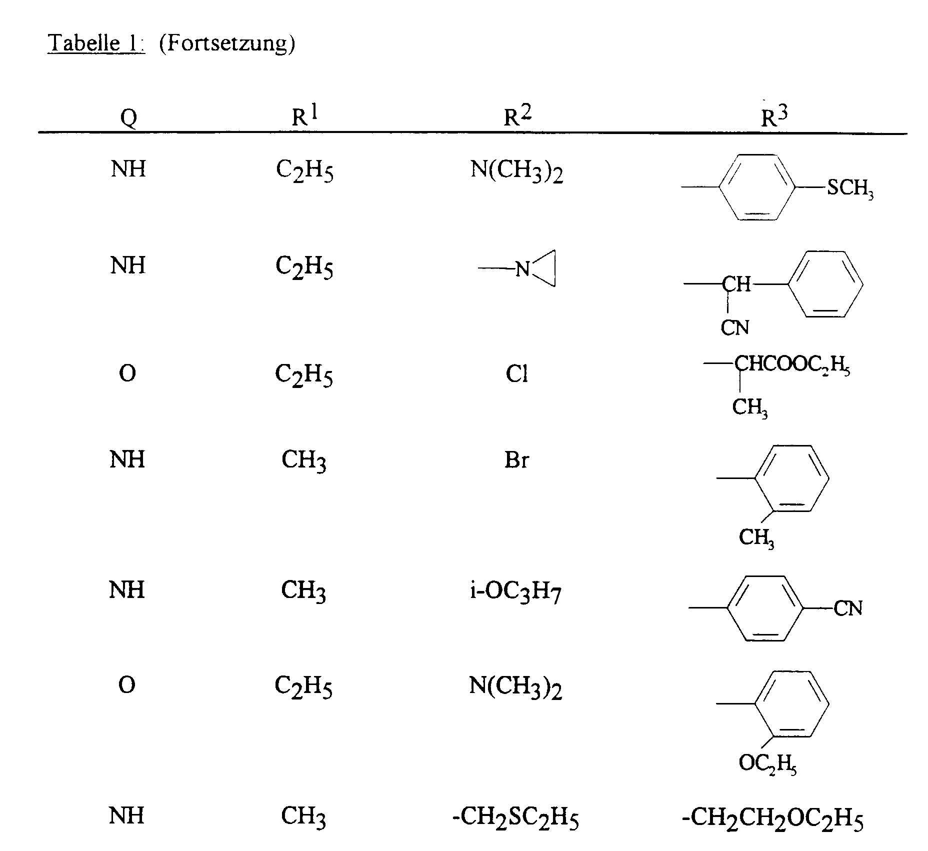

Verwendet man beispielsweise 2-Chlorsulfonylaminocarbonyl-4-ethoxy-5-ethyl-2,4-dihydro-3H-1,2,4-triazol-3-on und 2-Fluor-phenol als Ausgangsstoffe, so kann der Reaktionsablauf beim erfindungsgemäßen Verfahren (a) durch das folgende Formelschema skizziert werden:

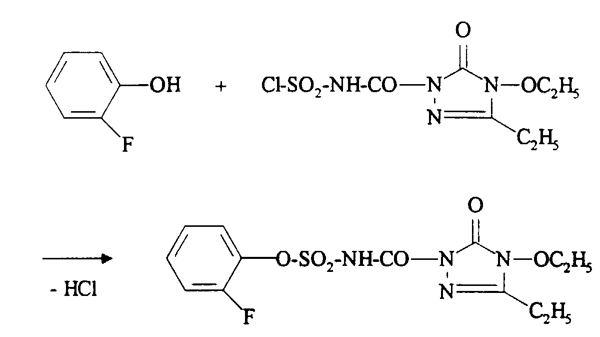

Verwendet man beispielsweise 4-Ethyl-5-ethylthio-2,4-dihydro-3H-1,2,4-triazol-3-on und Chlorsulfonylisocyanat sowie anschließend 2-Amino-benzoesäuremethylester als Ausgangsstoffe, so kann der Reaktionsablauf beim erfindungsgemäßen Verfahren (b) durch das folgende Formelschema skizziert werden:

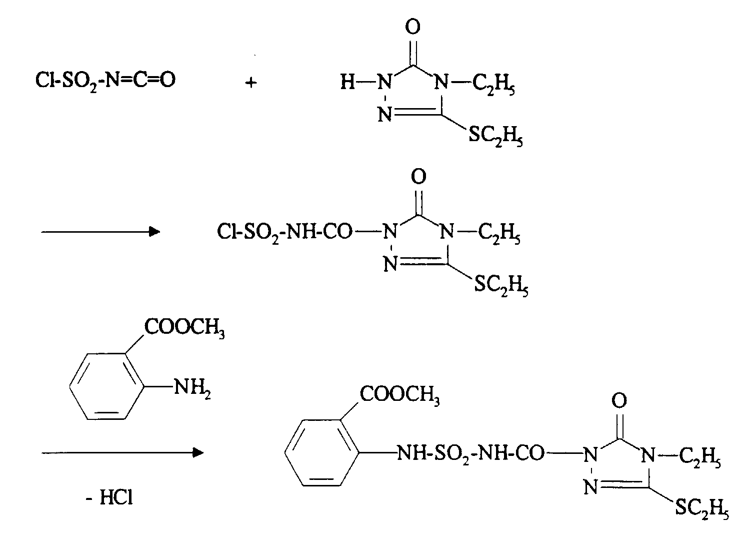

Die beim erfindungsgemäßen Verfahren (a) zur Herstellung von Verbindungen der Formel (I) als Ausgangsstoffe zu verwendenden Chlorsulfonylaminocarbonyltriazolinone sind durch die Formel (II) allgemein definiert.

In Formel (II) haben R¹ und R² vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R¹ und R² angegeben wurden.

Als Beispiele für die Ausgangsstoffe der Formel (II) seien genannt:

4,5-Dimethyl-, 4,5-Diethyl-, 4-Ethyl-5-methyl-, 5-Ethyl-4-methyl-, 4-Methyl-5-propyl-, 4-Ethyl-5-propyl-, 4-Cyclopropyl-5-ethyl-, 5-Cyclopropyl-4-methyl-, 5-Cyclopropyl-4-ethyl-, 4-Methyl-5-chlor-, 4-Ethyl-5-chlor-, 4-Methyl-5-brom-, 4-Ethyl-5-brom-, 4-Cyclopropyl-5-chlor-, 4-Cyclopropyl-5-brom-, 4-Methoxy-5-methyl-, 4-Ethoxy-5-methyl-, 4-Ethoxy-5-ethyl-, 4-Methoxy-5-ethyl-, 4-Methyl-5-methoxy-, 4-Ethyl-5-methoxy-, 4-Methyl-5-ethoxy, 4-Ethyl-5-ethoxy-, 4-Methyl-5-methylthio-, 4-Methyl-5-ethylthio-, 4-Ethyl-5-methylthio-, 4-Ethyl-5-ethylthio, 4-Cyclopropyl-5-methoxy-, 4-Cyclopropyl-5-ethoxy-, 4-Cyclopropyl-5-methylthio-, 4-Cyclopropyl-5-ethylthio-, 4-Methoxy-5-cyclopropyl-, 4-Ethoxy-5-cyclopropyl-, 4-Methoxy-5-ethoxy-, 4,5-Dimethoxy-, 4,5-Diethoxy-, 4-Methyl-5-dimethylamino-, 4-Ethyl-5-dimethylamino- und 4-Cyclopropyl-5-dimethylamino-2-chlorsulfonylaminocarbonyl-2,4-dihydro-3H-1,2,4-triazol-3-on.

Die Chlorsulfonylaminocarbonyltriazolinone der allgemeinen Formel (II) sind noch nicht aus der Literatur bekannt und sind als neue Stoffe ebenfalls Gegenstand der vorliegenden Erfindung.

Man erhält die neuen Verbindungen der Formel (II), wenn man Triazolinone der allgemeinen Formel (IV) 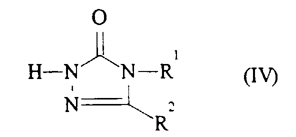 in welcher
- R¹ und R²: die oben angegebene Bedeutung haben,
mit Chlorsulfonylisocyanat, gegebenenfalls in Gegenwart eines Verdünnungsmittels, wie z.B. Methylenchlorid, bei Temperaturen zwischen -20°C und +50°C umsetzt und auf übliche Weise aufarbeitet (vgl. die Herstellungsbeispiele).

Die bei den erfindungsgemäßen Verfahren (a) und (b) zur Herstellung von Verbindungen der Formel (I) als Ausgangsstoffe zu verwendenden nucleophilen Verbindungen sind durch die Formel (III) allgemein definiert.

In Formel (III) haben Q und R³ vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für Q und R³ angegeben wurden.

Die Ausgangsstoffe der Formel (III) sind bekannte organische Synthesechemikalien.

Die bei den erfindungsgemäßen Verfahren (a) und (b) zur Herstellung von Verbindungen der Formel (I) als Ausgangsstoffe zu verwendenden Triazolinone sind durch die Formel (IV) allgemein definiert.

In Formel (IV) haben R¹ und R² vorzugsweise bzw. insbesondere diejenigen Bedeutungen, die bereits oben im Zusammenhang mit der Beschreibung der erfindungsgemäßen Verbindungen der Formel (I) vorzugsweise bzw. als insbesondere bevorzugt für R¹ und R² angegeben wurden.

Die Ausgangsstoffe der Formel (IV) sind bekannt und/oder können nach an sich bekannten Verfahren hergestellt werden (vgl. EP-A 283876, EP-A 294666, EP-A 301946, EP-A 298371, EP-A 341489, EP-A 399294, EP-A 398096, EP-A 422469, EP-A 425948, EP-A 431291, EP-A 477646, DE-OS 4110795).

Die erfindungsgemäßen Verfahren (a) und (b) zur Herstellung der neuen Verbindungen der Formel (I) werden vorzugsweise unter Verwendung von Verdünnungsmitteln durchgeführt. Als Verdünnungsmittel kommen dabei praktisch alle inerten organischen Lösungsmittel infrage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methylethyl-, Methyl-isopropyl- und Methyl-isobutyl-keton, Ester wie Essigsäuremethylester und -ethylester, Nitrile wie z.B. Acetonitril und Propionitril, Amide wie z.B. Dimethylformamid, Dimethylacetamid und N-Methylpyrrolidon sowie Dimethylsulfoxid, Tetramethylensulfon und Hexamethylphosphorsäuretriamid.

Als Säureakzeptoren können bei den erfindungsgemäßen Verfahren (a) und (b) alle üblicherweise für derartige Umsetzungen verwendbaren Säurebindemittel eingesetzt werden. Vorzugsweise infrage kommen Alkalimetallhydroxide wie z.B. Natrium- und Kaliumhydroxid, Erdalkalihydroxide wie z.B. Calciumhydroxid, Alkalicarbonate und - alkoholate wie Natrium- und Kaliumcarbonat, Natrium- und Kalium-tert-butylat, ferner basische Stickstoffverbindungen, wie Trimethylamin, Triethylamin, Tripropylamin, Tributylamin, Diisobutylamin, Dicyclohexylamin, Ethyldiisopropylamin, Ethyldicyclohexylamin, N,N-Dimethylbenzylamin, N,N-Dimethyl-anilin, Pyridin, 2-Methyl-, 3-Methyl-, 4-Methyl-, 2,4-Dimethyl-, 2,6-Dimethyl-, 2-Ethyl-, 4-Ethyl- und 5-Ethyl-2-methyl-pyridin, 1,5-Diazabicyclo[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei den erfindungsgemäßen Verfahren (a) und (b) in einem größeren Bereich variiert werden. Im allgemeinen arbeitet man bei Temperaturen zwischen -20°C und +80°C, vorzugsweise bei Temperaturen zwischen -10°C und +50°C.

Die erfindungsgemäßen Verfahren (a) und (b) werden im allgemeinen unter Normaldruck durchgeführt. Es ist jedoch auch möglich, unter erhöhtem oder vermindertem Druck zu arbeiten.

Zur Durchführung der erfindungsgemäßen Verfahren (a) und (b) werden die jeweils benötigten Ausgangsstoffe im allgemeinen in angenähert äquimolaren Mengen eingesetzt. Es ist jedoch auch möglich, eine der jeweils eingesetzten Komponenten in einem größeren Überschuß zu verwenden. Die Reaktionen werden im allgemeinen in einem geeigneten Verdünnungsmittel in Gegenwart eines Säureakzeptors durchgeführt, und das Reaktionsgemisch wird mehrere Stunden bei der jeweils erforderlichen Temperatur gerührt. Die Aufarbeitung erfolgt bei den erfindungsgemäßen Verfahren (a) und (b) jeweils nach üblichen Methoden (vgl. die Herstellungsbeispiele).

Aus den erfindungsgemäßen Verbindungen der allgemeinen Formel (I) können gegebenenfalls Salze hergestellt werden. Man erhält solche Salze in einfacher Weise nach üblichen Salzbildungsmethoden, beispielsweise durch Lösen oder Dispergieren einer Verbindung der Formel (I) in einem geeigneten Lösungsmittel, wie z.B. Methylenchlorid, Aceton, tert-Butyl-methylether oder Toluol, und Zugabe einer geeigneten Base. Die Salze können dann - gegebenenfalls nach längerem Rühren - durch Einengen oder Absaugen isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur selektiven Bekämpfung von dikotylen Unkräutern in monokotylen Kulturen sowohl im Vorauflauf- als auch im Nachauflauf-Verfahren.

Darüberhinaus zeigen die erfindungsgemäßen Wirkstoffe (I) auch interessante Nebenwirkungen, nämlich eine blattinsektizide Wirkung sowie fungizide Wirkungen insbesondere gegen Pyricularia oryzae, z.B. am Reis, und gegen Erysiphe graminis.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxy-alkansäureester, wie z.B. Diclofop-methyl, Fenoxaprop-ethyl, Fluazifop-butyl, Haloxyfop-methyl und Quizalofopethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuronmethyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin, Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 10 g und 10 kg Wirkstoff pro Hektar Bodenfläche, vorzugsweise zwischen 50 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

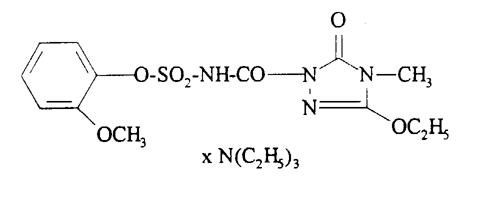

### (Verfahren (a))

2,84 g (0,01 Mol) 2-Chlorsulfonylaminocarbonyl-4-methyl-5-ethoxy-2,4-dihydro-3H-1,2,4-triazol-3-on werden in 100 ml Methylenchlorid vorgelegt und zu dieser Mischung werden bei 20°C 1,24 g (0,01 Mol) 2-Methoxy-phenol und 2,02 g (0,02 Mol) Triethylamin gegeben. Das Reaktionsgemisch wird 60 Minuten bei 20°C gerührt. Dann wird es dreimal mit je 150 ml Wasser gewaschen, mit Natriumsulfat getrocknet und filtriert. Das Filtrat wird im Wasserstrahlvakuum eingeengt und der Rückstand aus Isopropanol umkristallisiert.

Man erhält 2,5 g (53% der Theorie) 2-(2-Methoxy-phenoxy)-sulfonylaminocarbonyl-4-methyl-5-ethoxy-2,4-dihydro-3H-1,2,4-triazol-3-on-Triethylammoniumsalz vom Schmelzpunkt 107°C.

### Beispiel 2

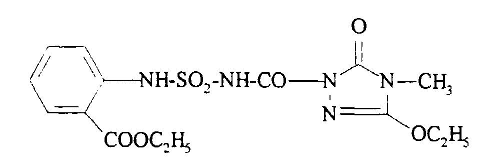

### (Verfahren (a))

5,69 g (0,02 Mol) 2-Chlorsulfonylaminocarbonyl-4-methyl-5-ethoxy-2,4-dihydro-3H-1,2,4-triazol-3-on werden in 150 ml Methylenchlorid vorgelegt und zu dieser Mischung wird bei 20°C eine Lösung von 6,6 g (0,04 Mol) 2-Amino-benzosäure-ethylester in 20 ml Methylenchlorid gegeben. Das Reaktionsgemisch wird 60 Minuten bei 20°C gerührt. Dann wird es dreimal mit je 150 ml Wasser gewaschen, mit Natriumsulfat getrocknet und filtriert. Das Filtrat wird im Wasserstrahlvakuum eingeengt und der Rückstand aus Isopropanol umkristallisiert.

Man erhält 5,7 g (69% der Theorie) 2-(2-Ethoxy-carbonyl-phenylamino)-sulfonylaminocarbonyl-4-methyl-5-ethoxy-2,4-dihydro-3H-1,2,4-triazol-3-on vom Schmelzpunkt 135°C.

### Beispiel 3

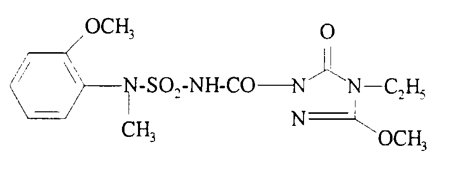

### (Verfahren (a))

5,69 g (0,02 Mol) 2-Chlorsulfonylminocarbonyl-4-ethyl-5-methoxy-2,4-dihydro-3H-1,2,4-triazol-3-on werden in 150 ml Methylenchlorid vorgelegt und zu dieser Mischung wird bei 20°C eine Lösung von 2,74 g (0,02 Mol) 2-Methoxy-N-methyl-anilin und 2,02 g (0,02 Mol) Triethylamin in 20 ml Methylenchlorid gegeben. Das Reaktionsgemisch wird 60 Minuten bei 20°C gerührt. Dann wird es dreimal mit je 150 ml Wasser gewaschen, mit Natriumsulfat getrocknet und filtriert. Das Filtrat wird im Wasserstrahlvakuum eingeengt und der Rückstand aus Isopropanol umkristallisiert.

Man erhält 3,2 g (40% der Theorie) 2-(2-Methoxy-N-methyl-phenylamino)-sulfonyl-aminocarbonyl-4-ethyl-5-methoxy-2,4-dihydro-3H-1,2,4-triazol-3-on vom Schmelzpunkt 134°C.

### Beispiel 4

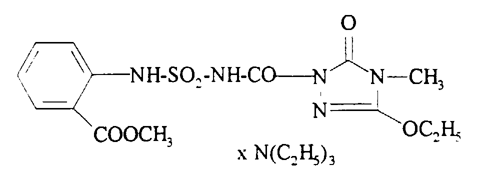

### (Verfahren (b))

4,3 g (0,03 Mol) 4-Methyl-5-ethoxy-2,4-dihydro-3H-1,2,4-triazol-3-on werden in 150 ml Methylenchlorid vorgelegt und auf -10°C abgekühlt. Dann wird eine Lösung von 4,3 g (0,03 Mol) Chlorsulfonylisocyanat in 20 ml Methylenchlorid dazu gegeben und das Gemisch wird 30 Minuten gerührt. Dann wird bei 20°C eine Lösung von 4,53 g (0,03 Mol) 2-Amino-benzosäure-methylester und 6,06 g (0,06 Mol) Triethylamin in 20 ml Methylenchlorid zugetropft und das Reaktionsgemisch wird weitere 30 Minuten gerührt. Anschließend wird es dreimal mit je 150 ml Wasser gewaschen, mit Natriumsulfat getrocknet und filtriert. Das Filtrat wird im Wasserstrahlvakuum eingeengt, der Rückstand mit Essigsäureethylester verrührt und das kristallin angefallene Produkt durch Absaugen isoliert.

Man erhält 11 g (73% der Theorie) 2-(2-Methoxycarbonyl-phenylamino)-sulfonylaminocarbonyl-4-methyl-5-ethoxy-2,4-dihydro-3H-1,2,4-triazol-3-on-Triethylammoniumsalz vom Schmelzpunkt 142°C (Zersetzung).

Analog zu den Herstellungsbeispielen 1 bis 4 sowie entsprechend der allgemeinen Beschreibung der erfindungsgemäßen Herstellungsverfahren können beispielsweise auch die in der nachstehenden Tabelle 2 aufgeführten Verbindungen der Formel (I) hergestellt werden.

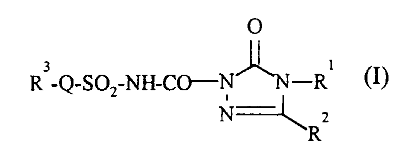

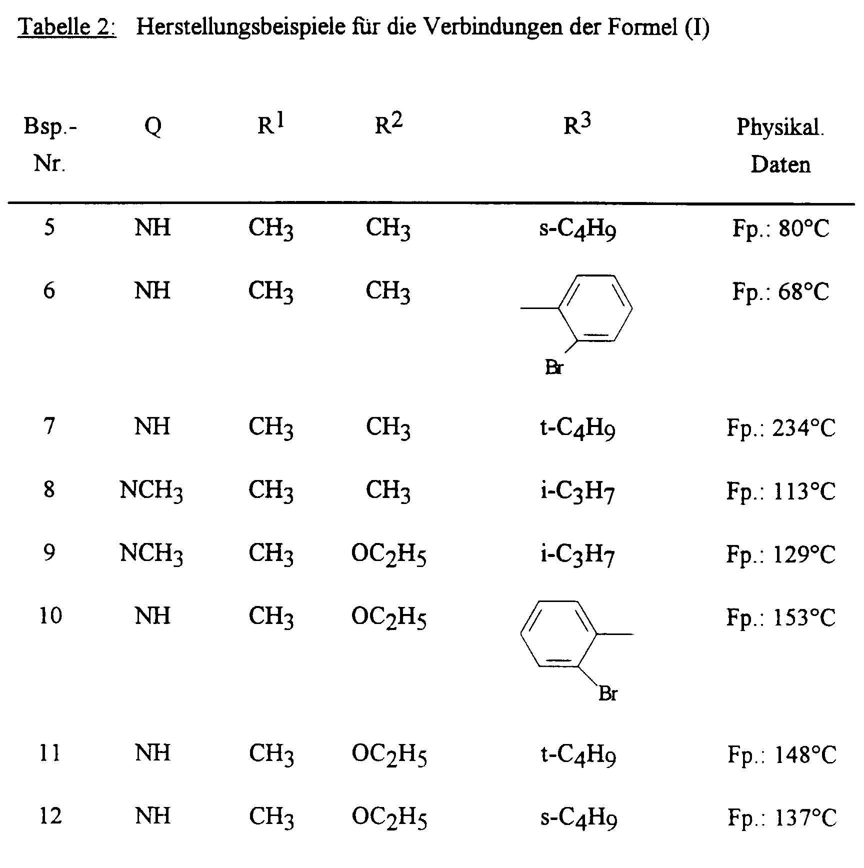

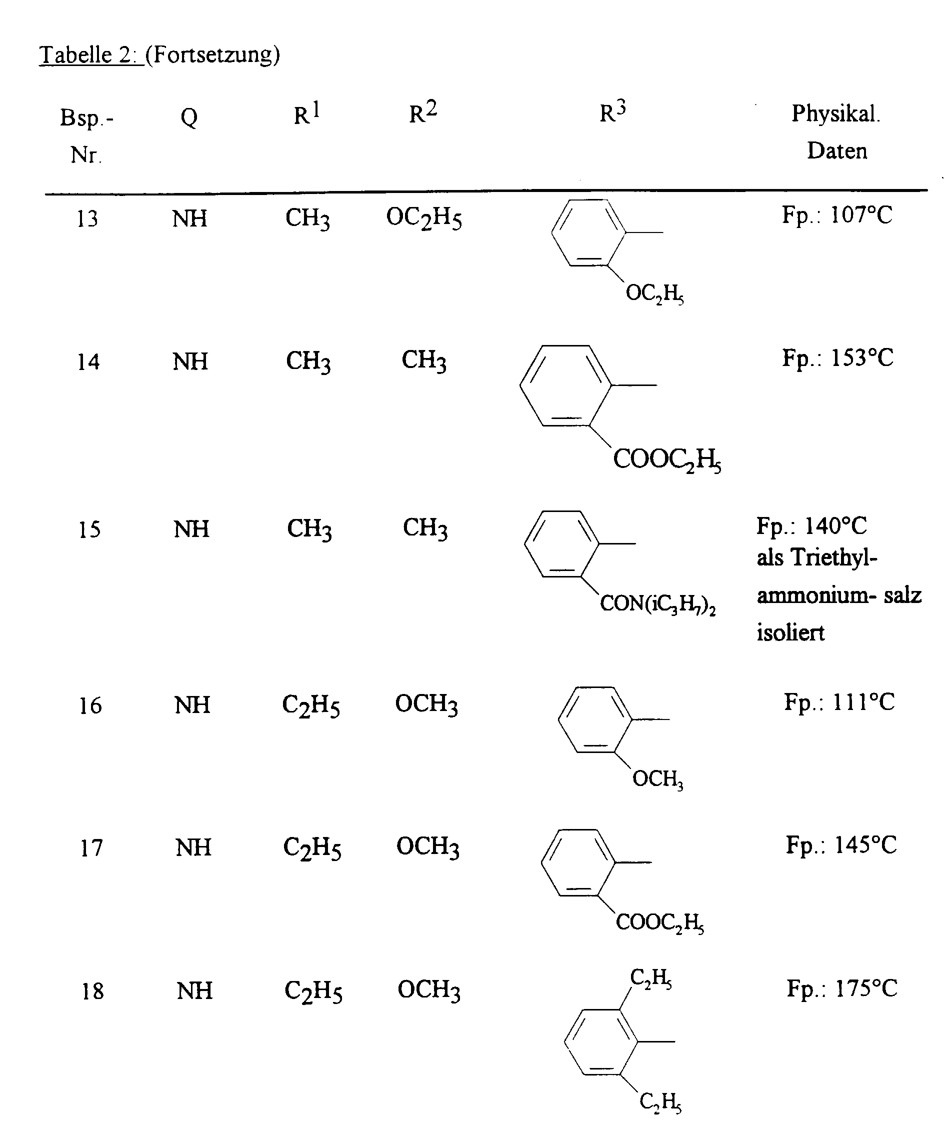

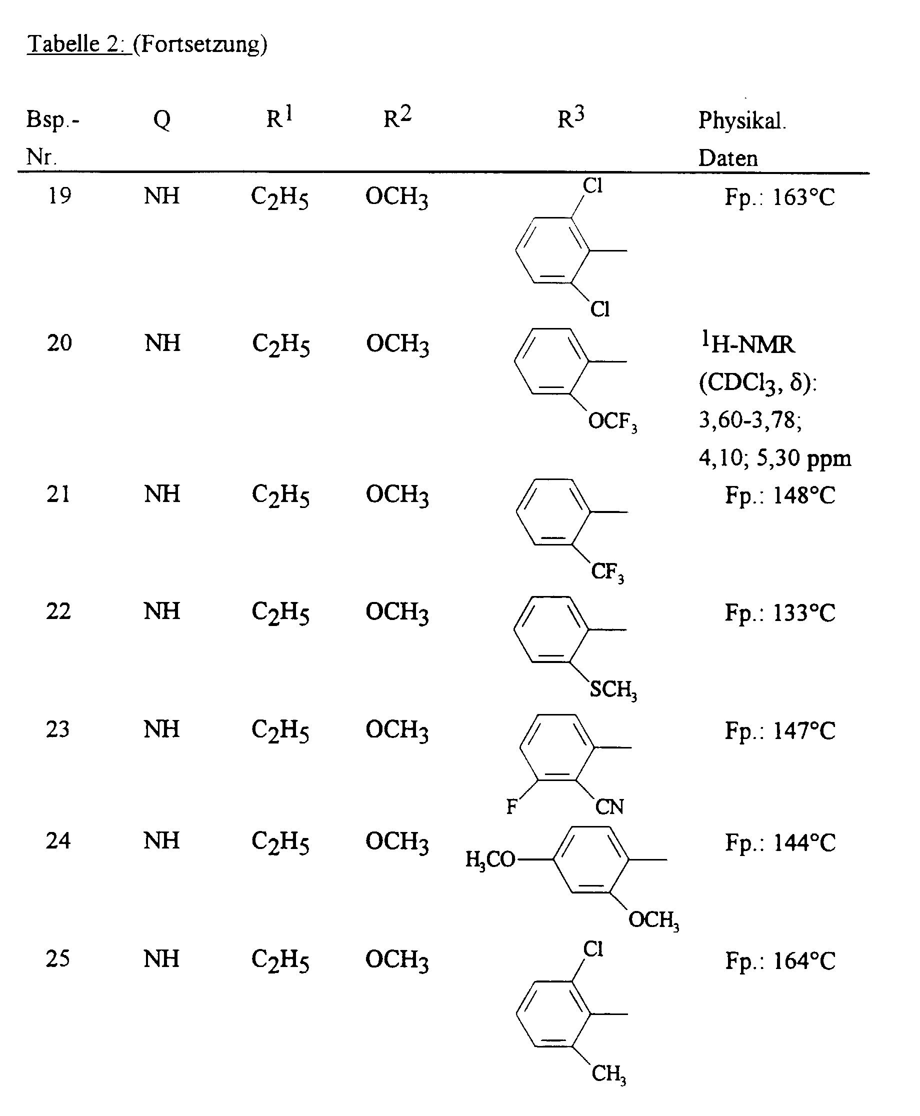

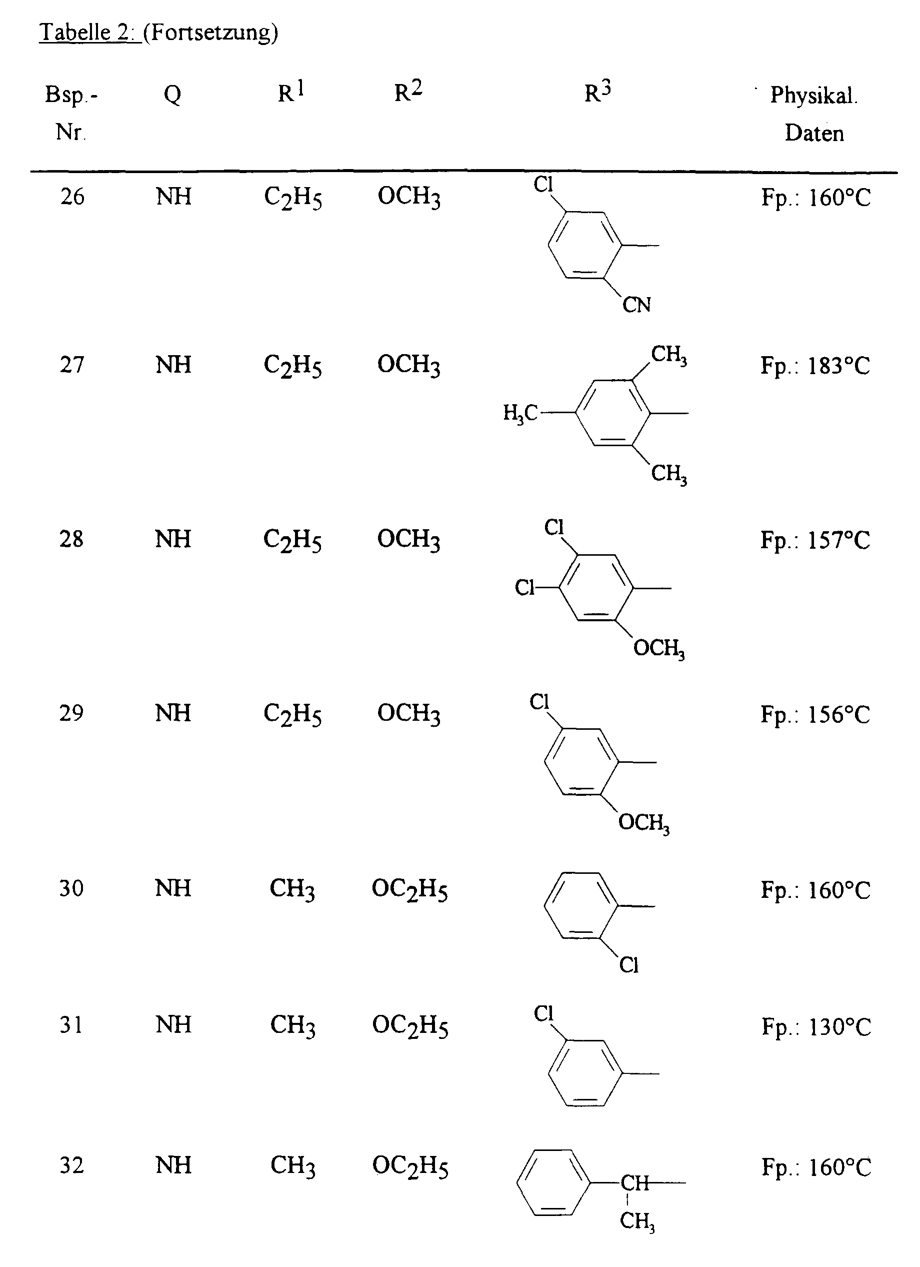

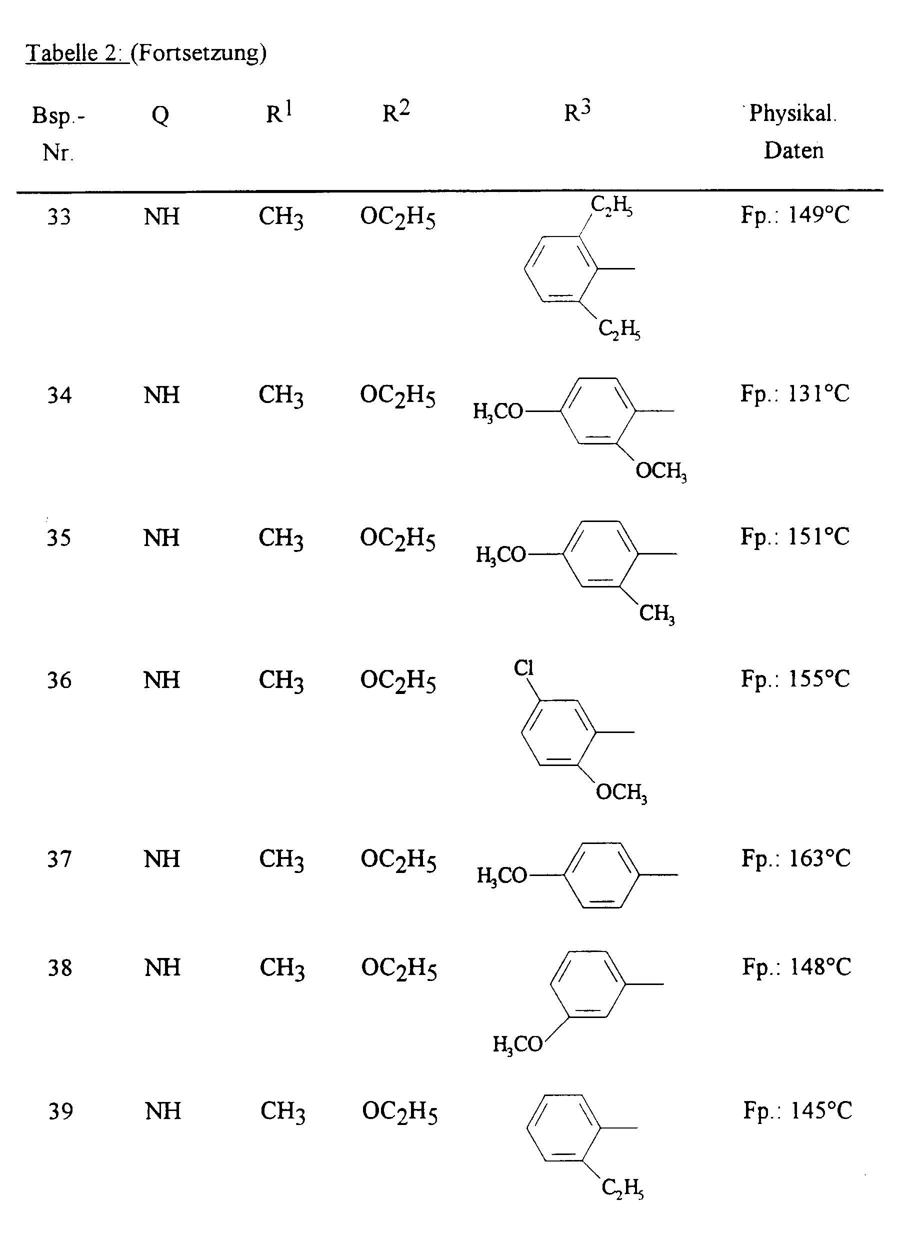

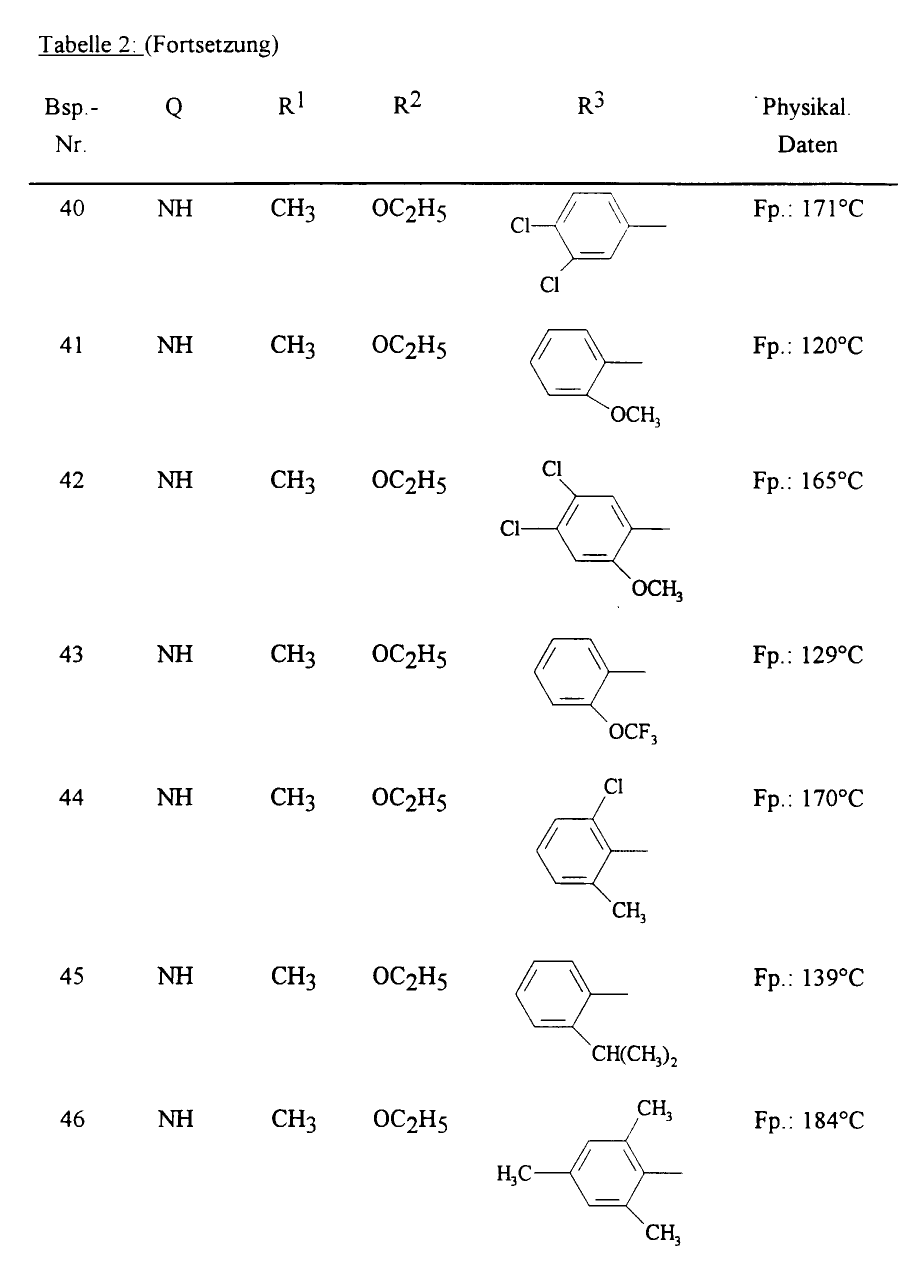

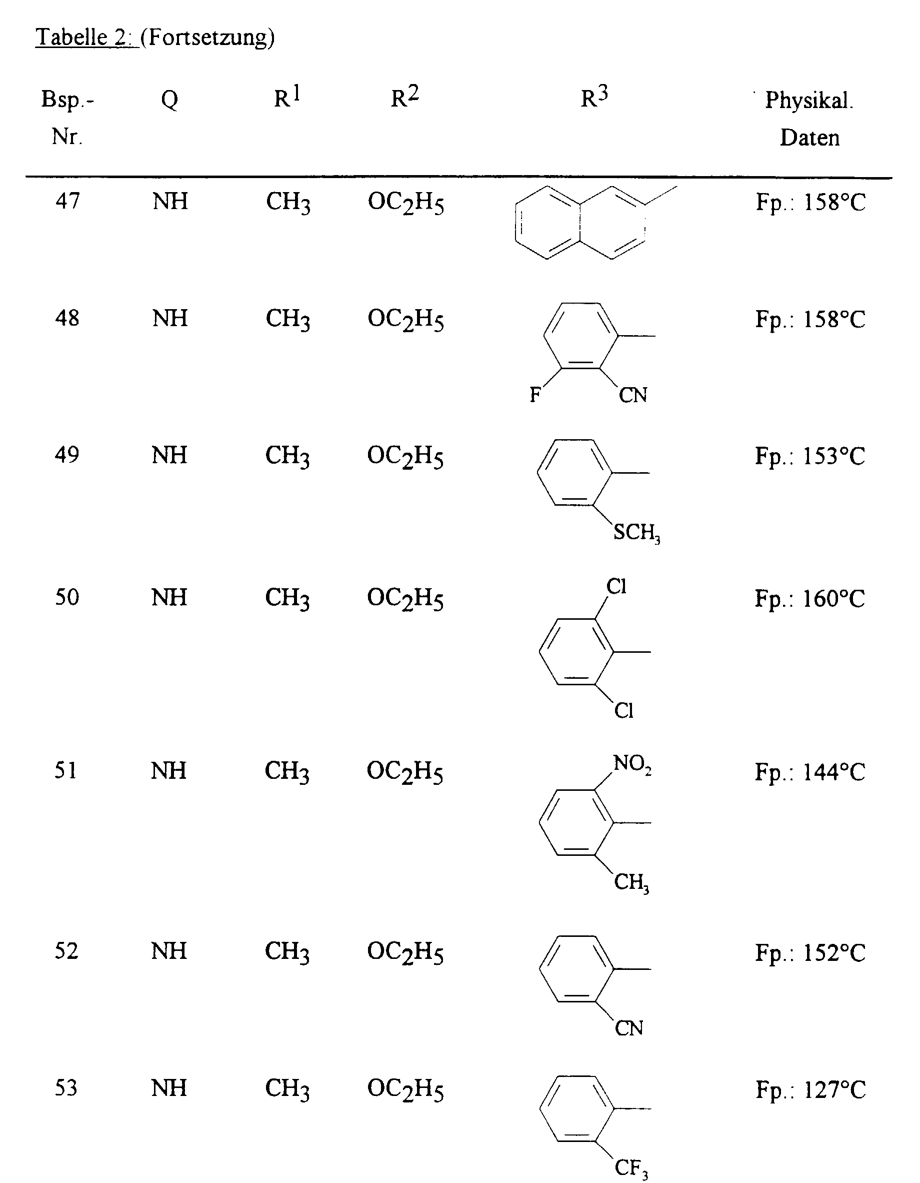

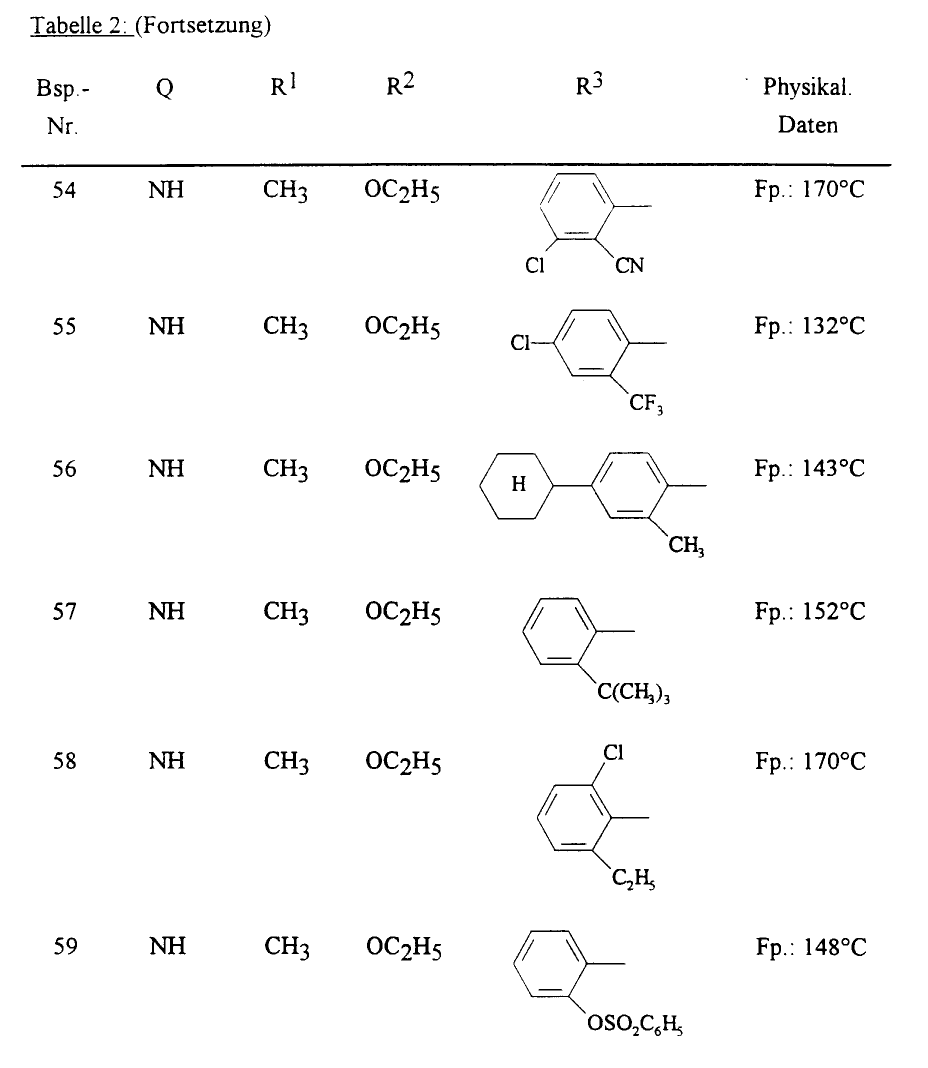

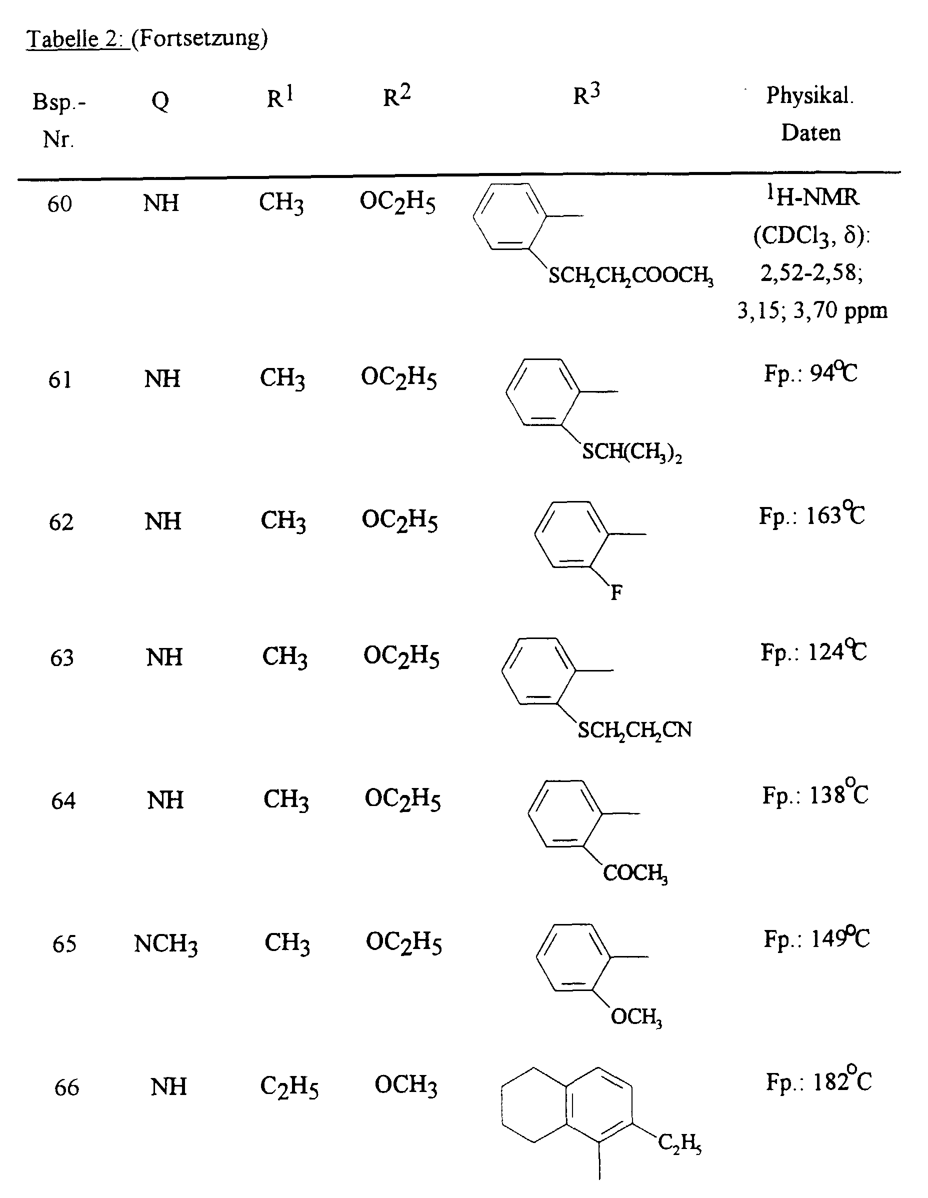

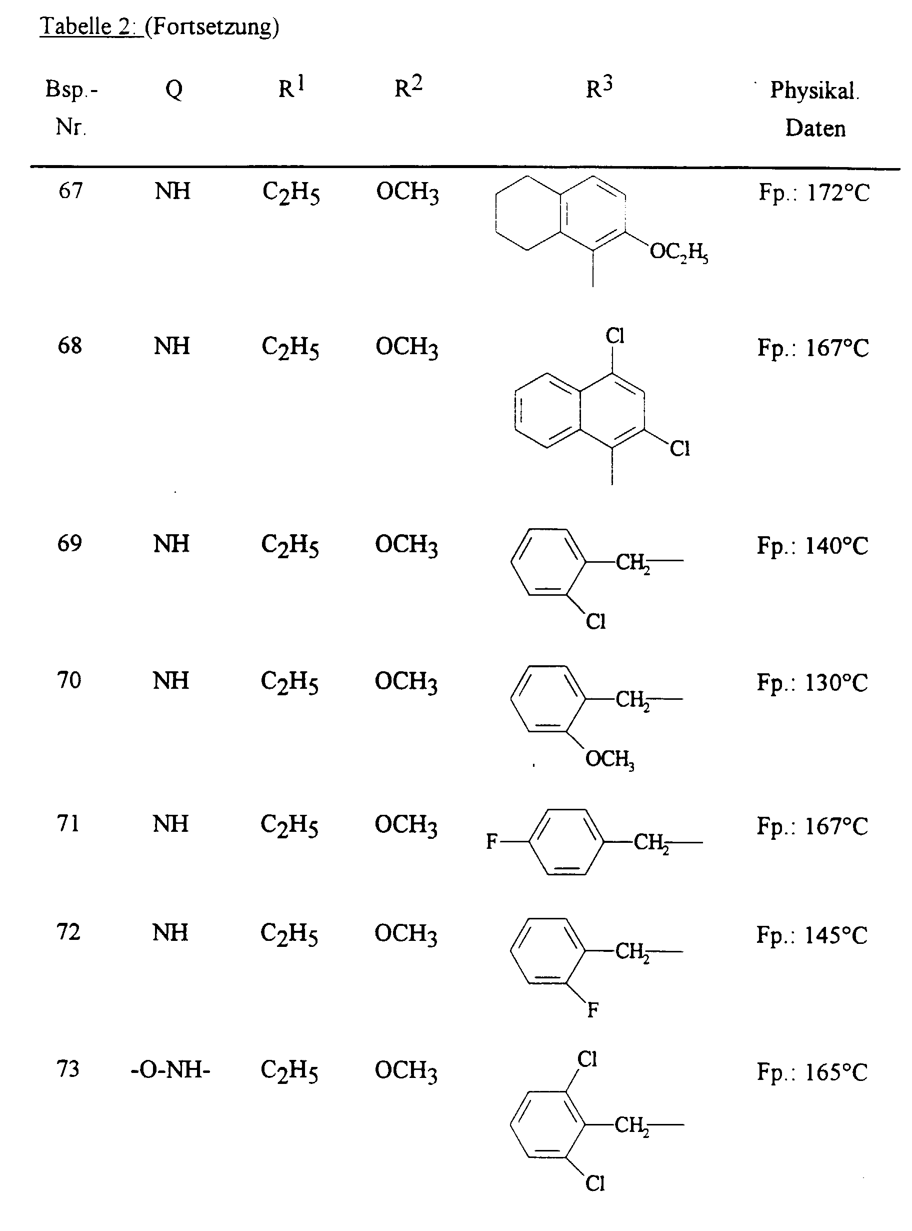

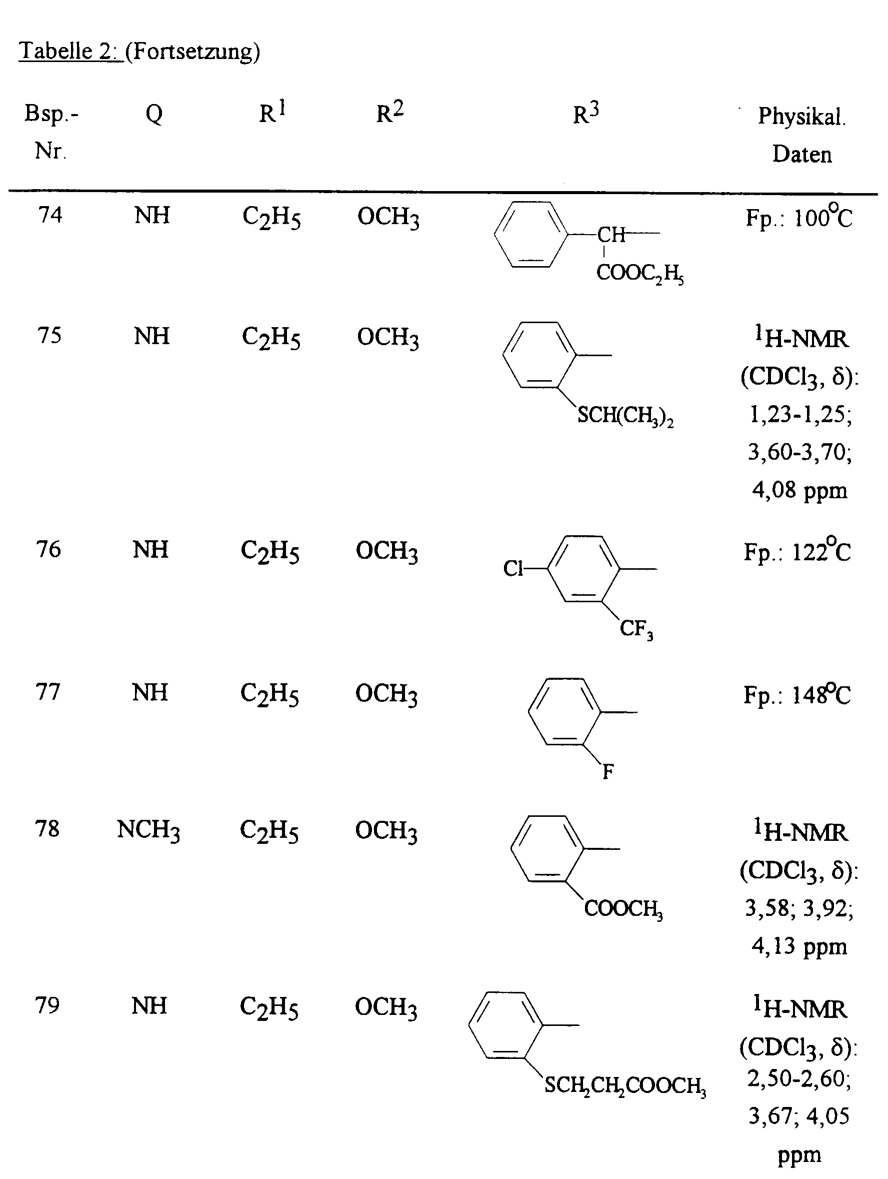

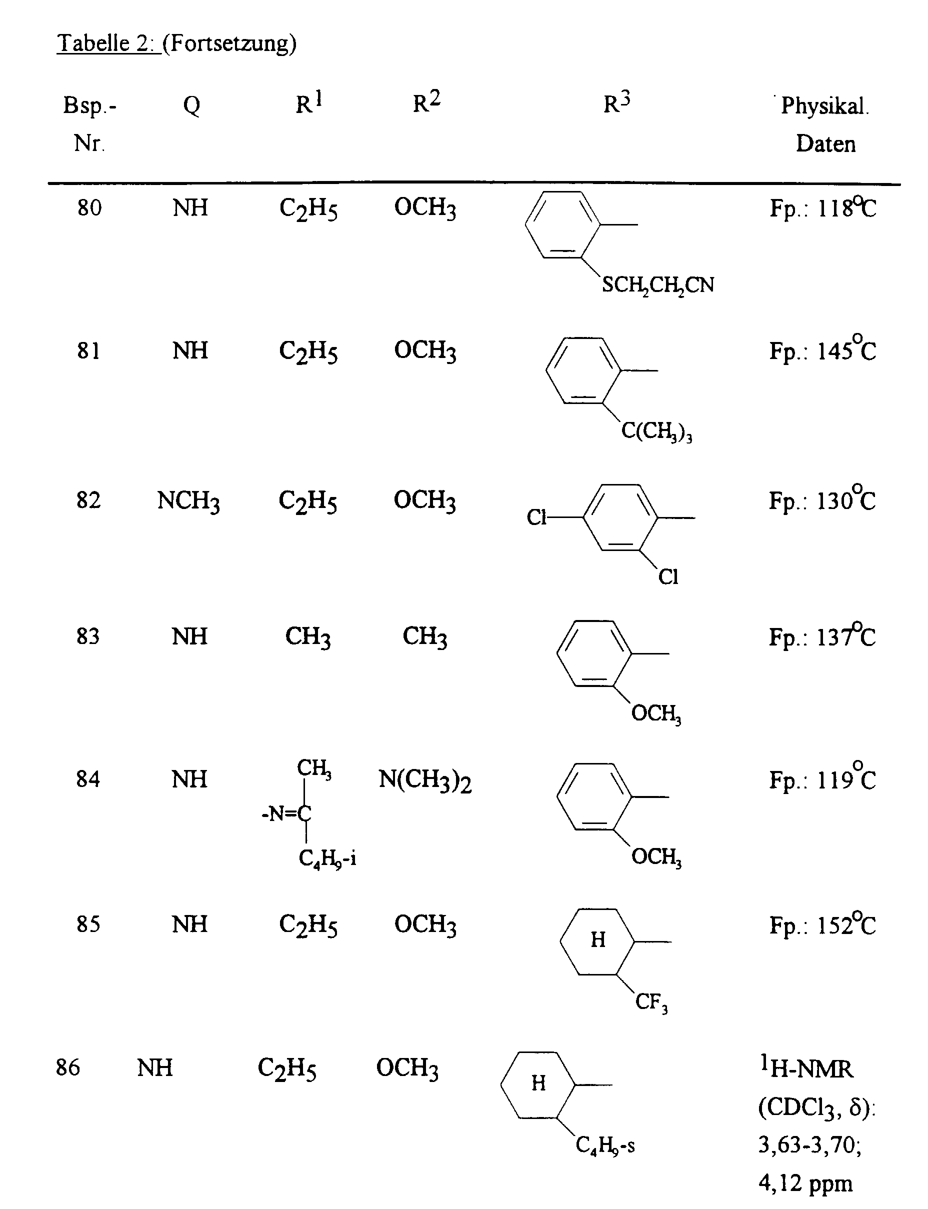

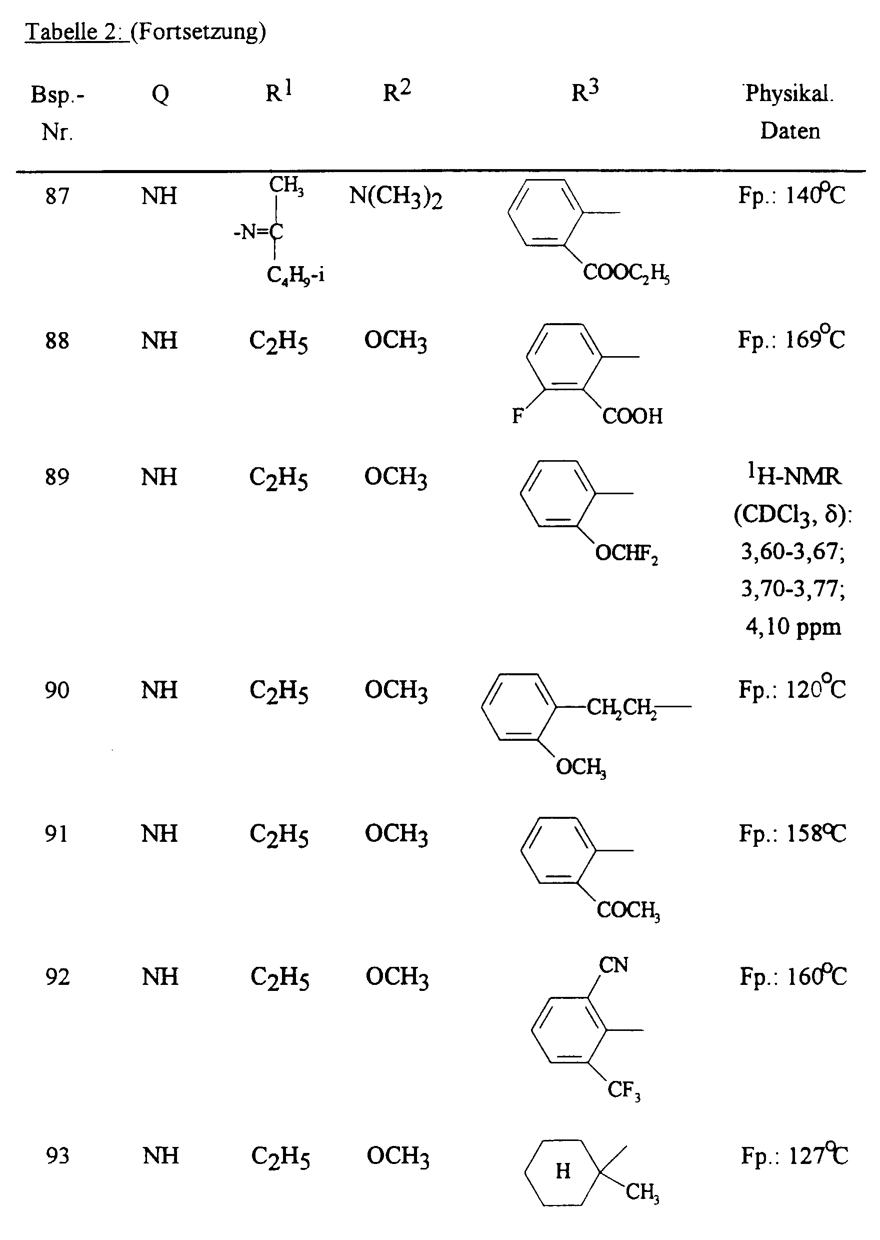

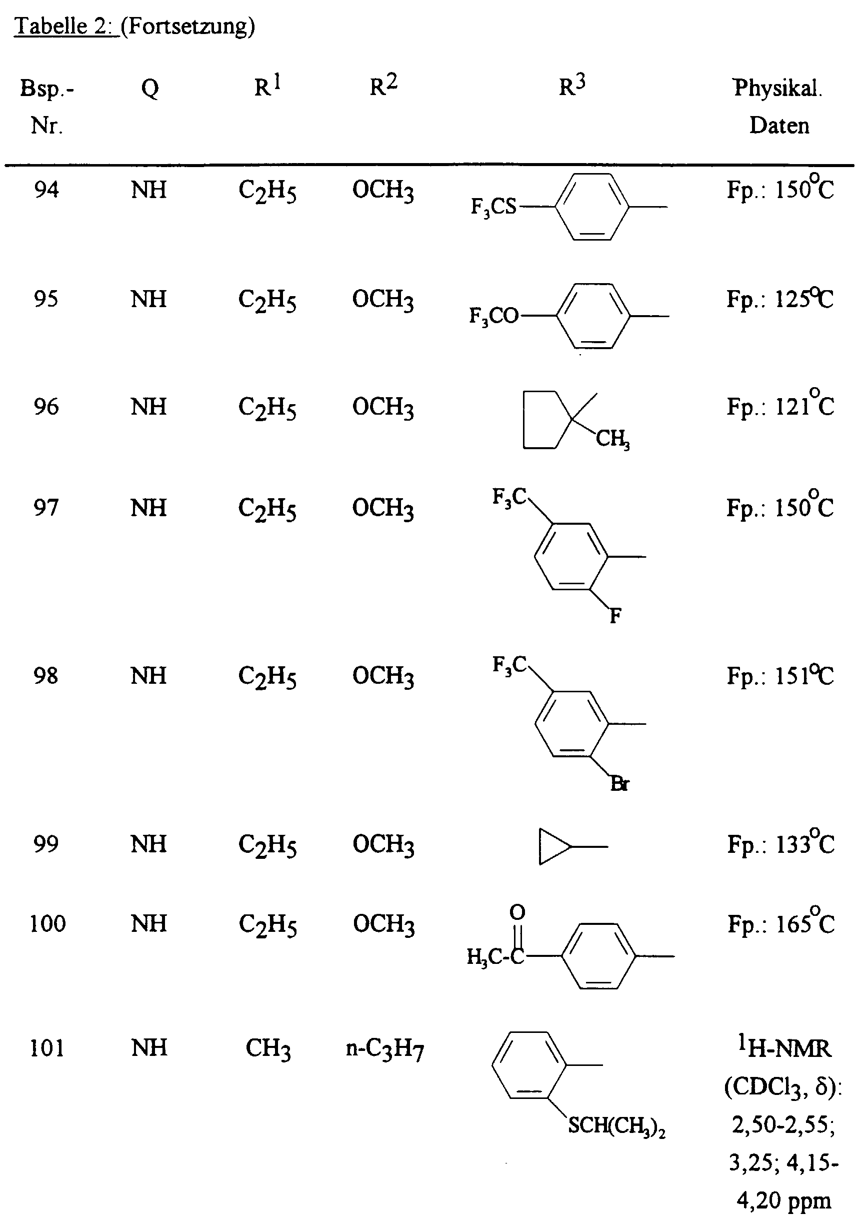

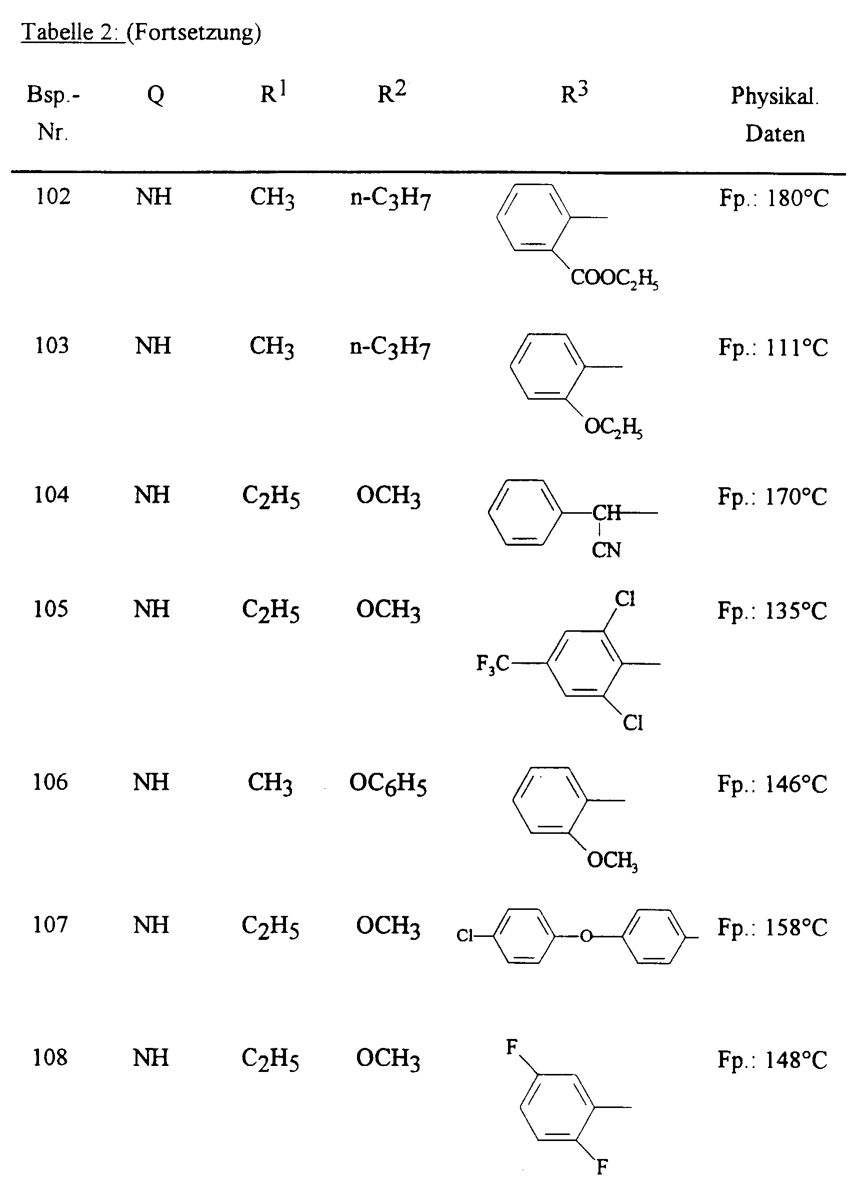

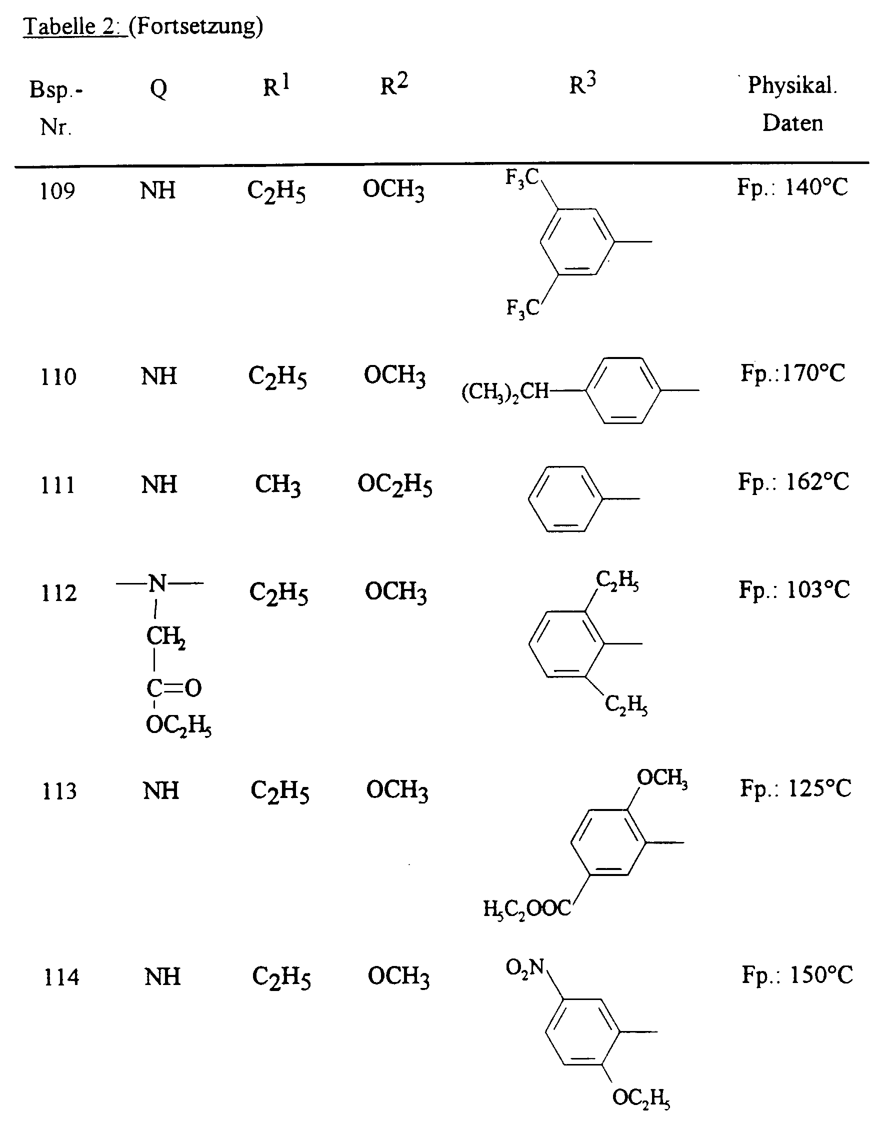

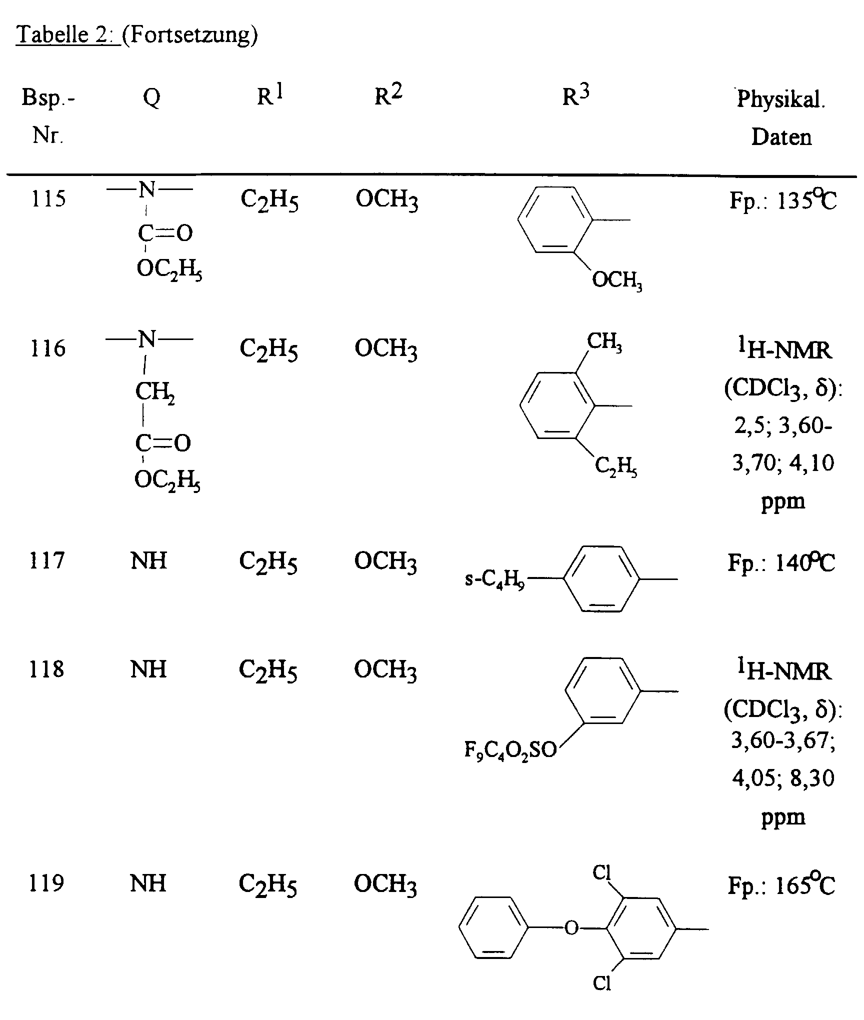

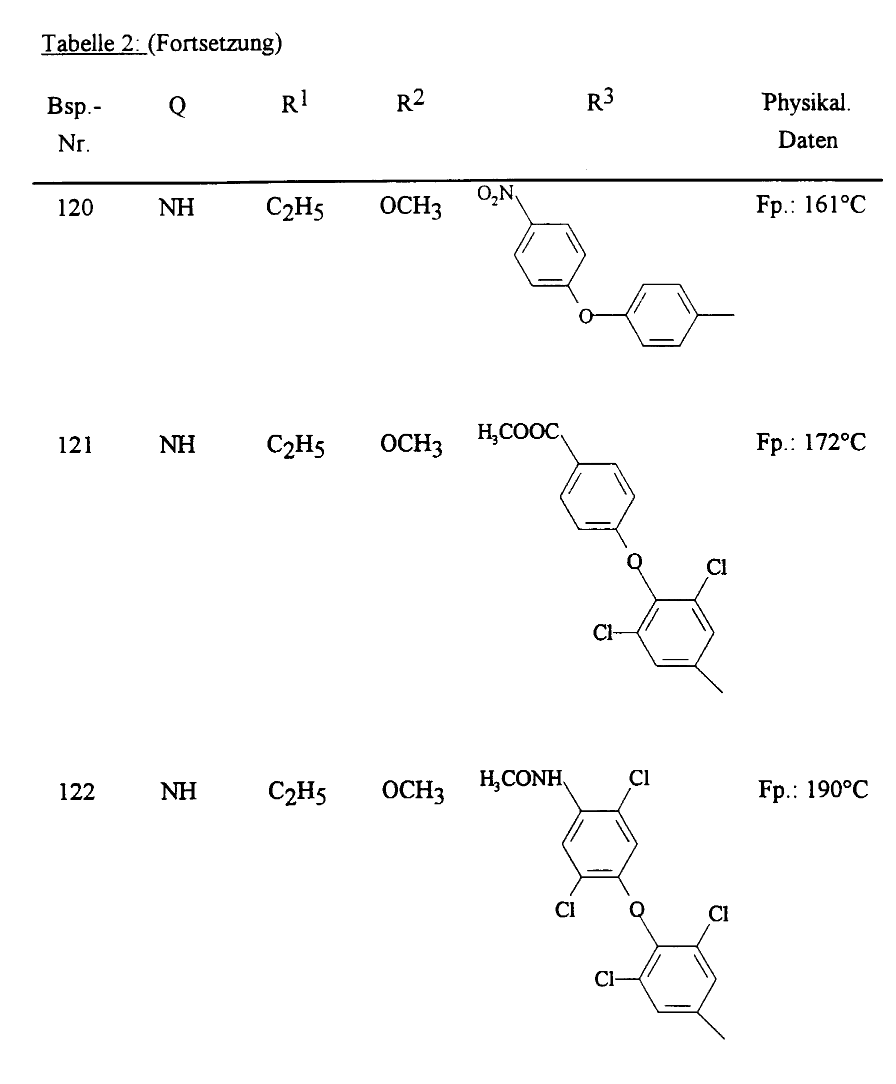

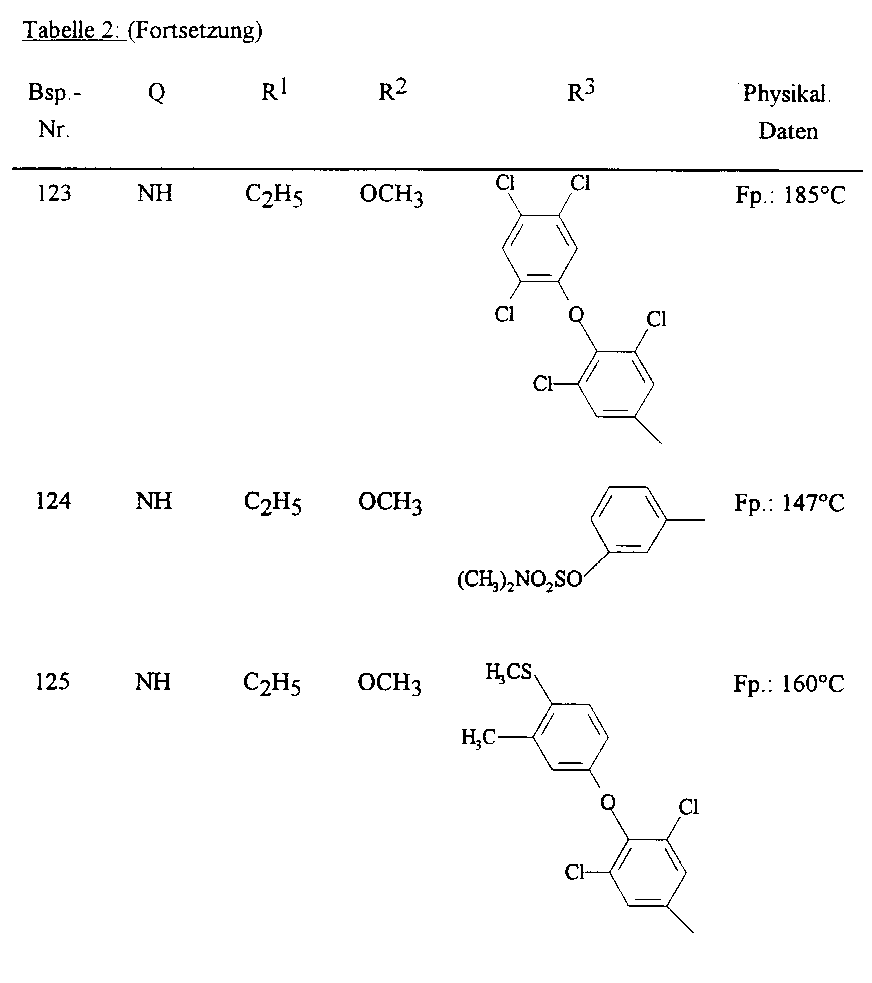

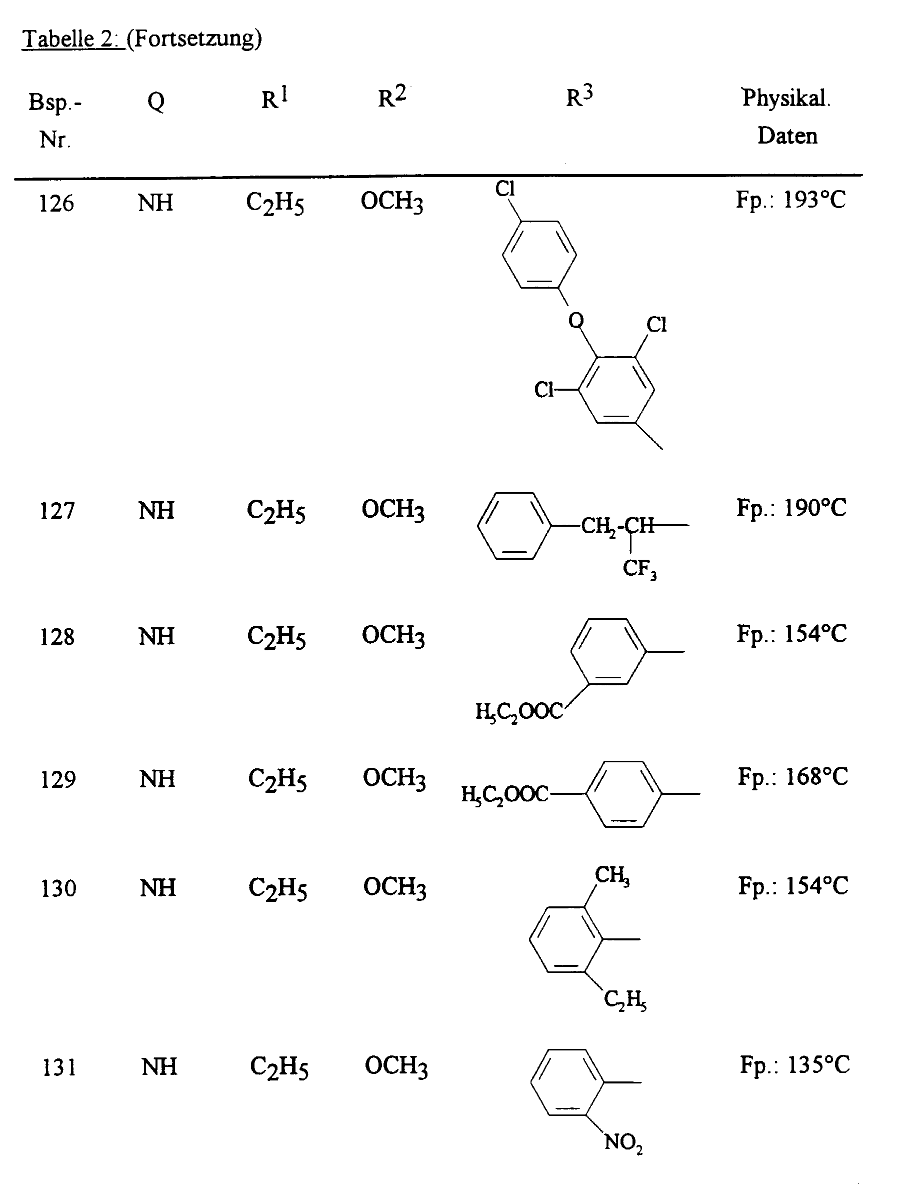

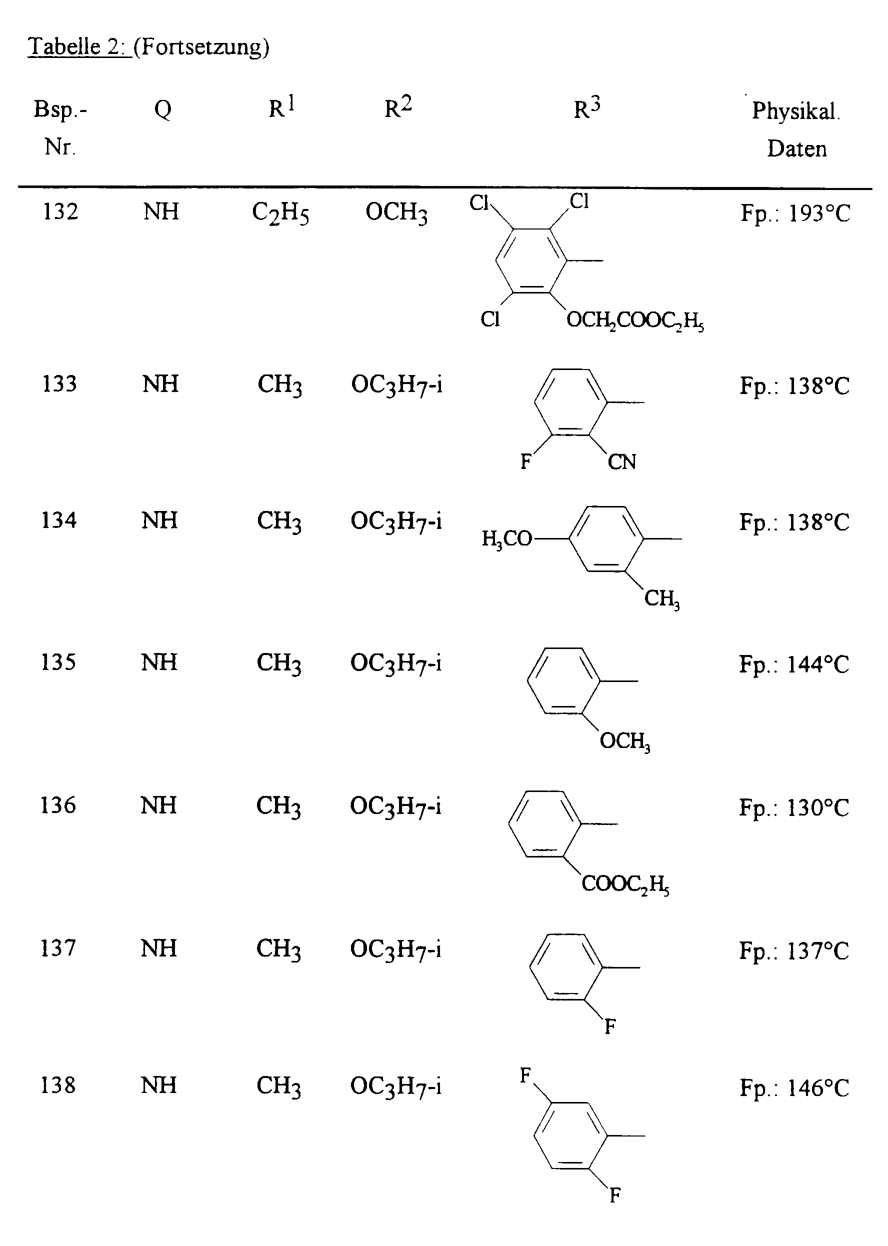

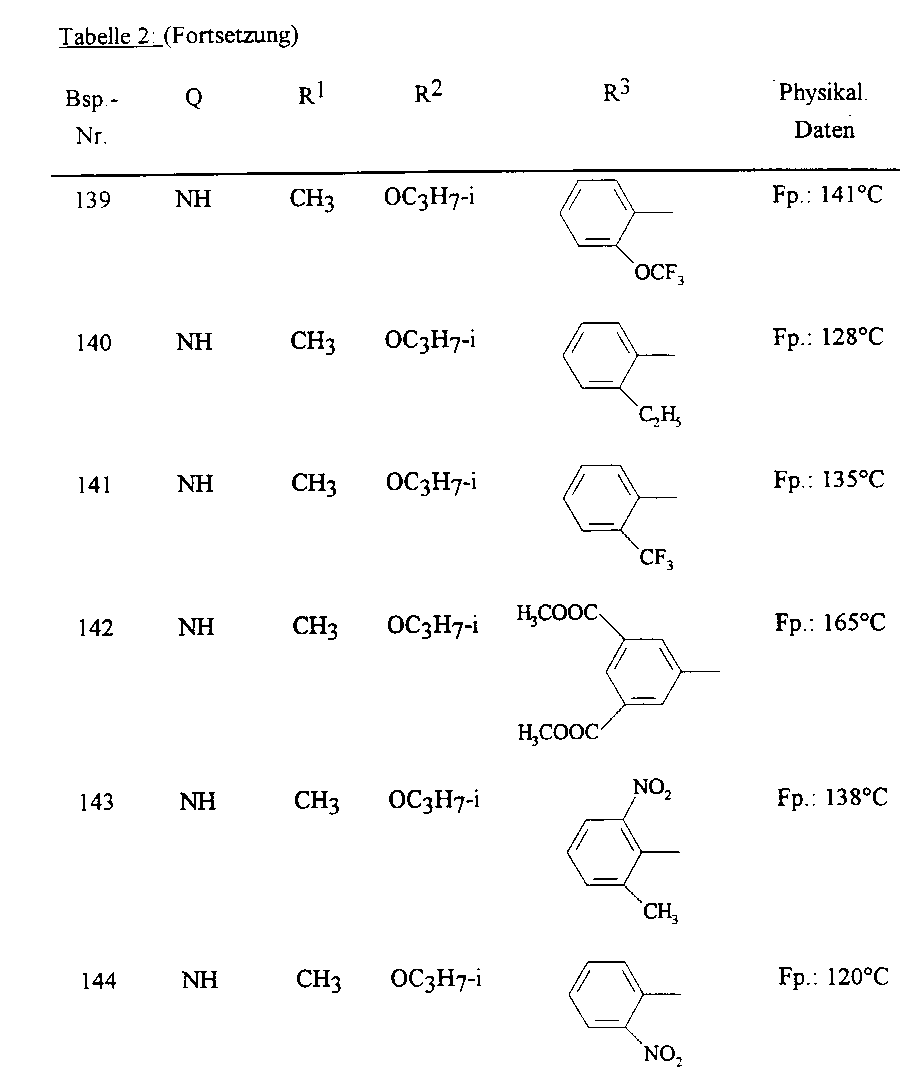

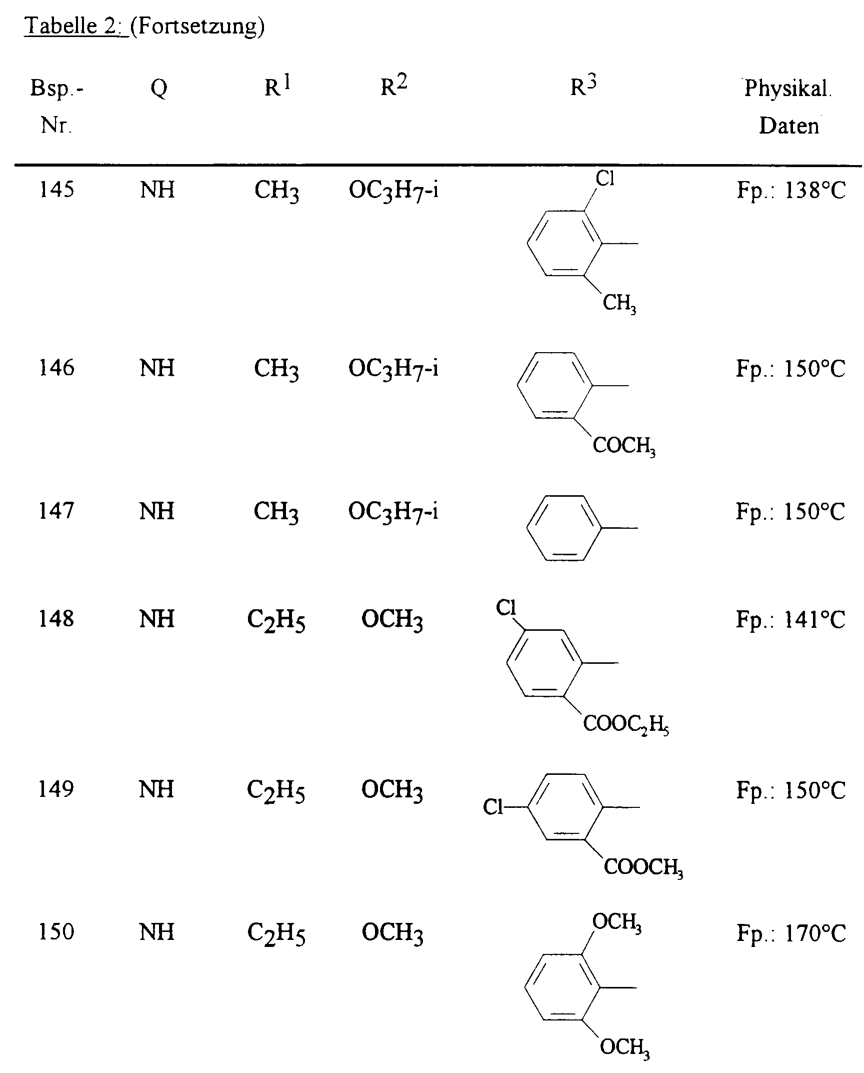

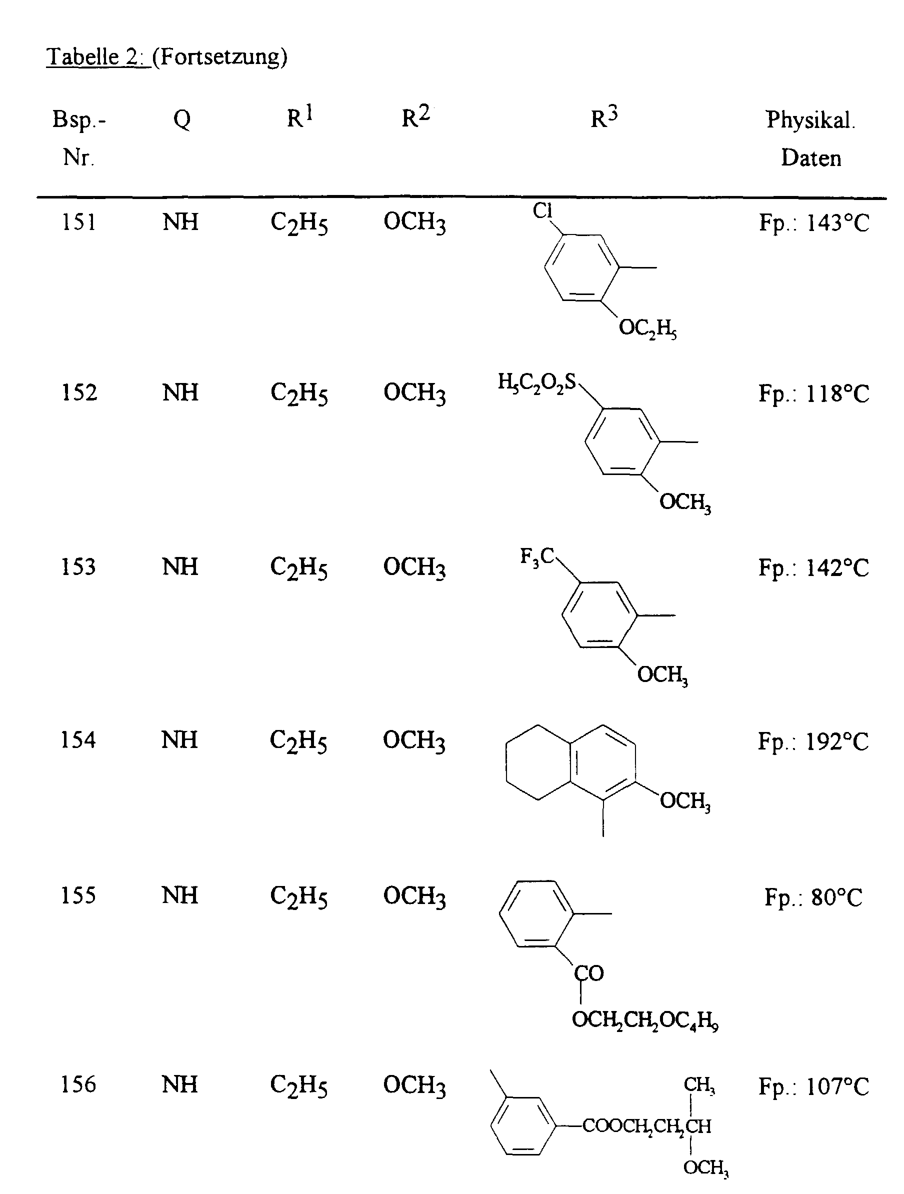

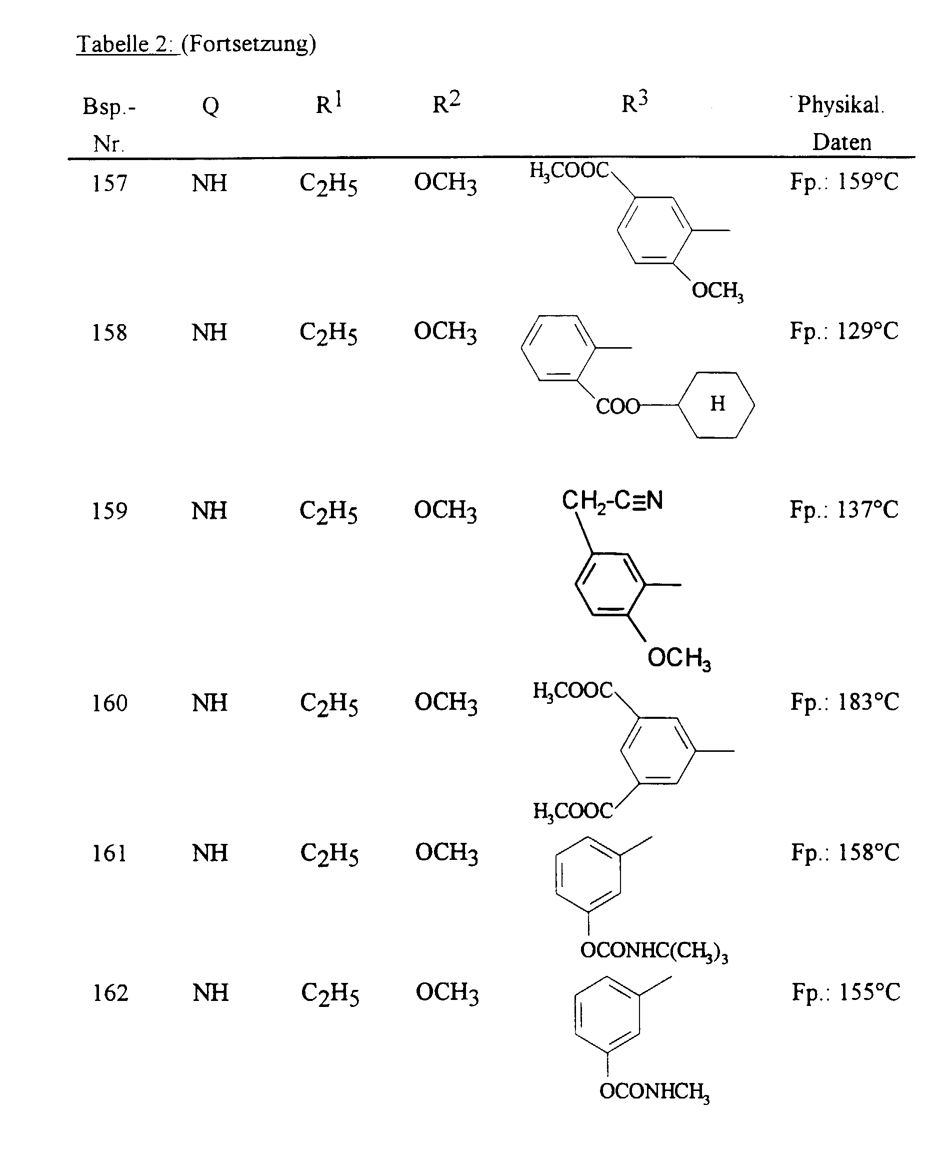

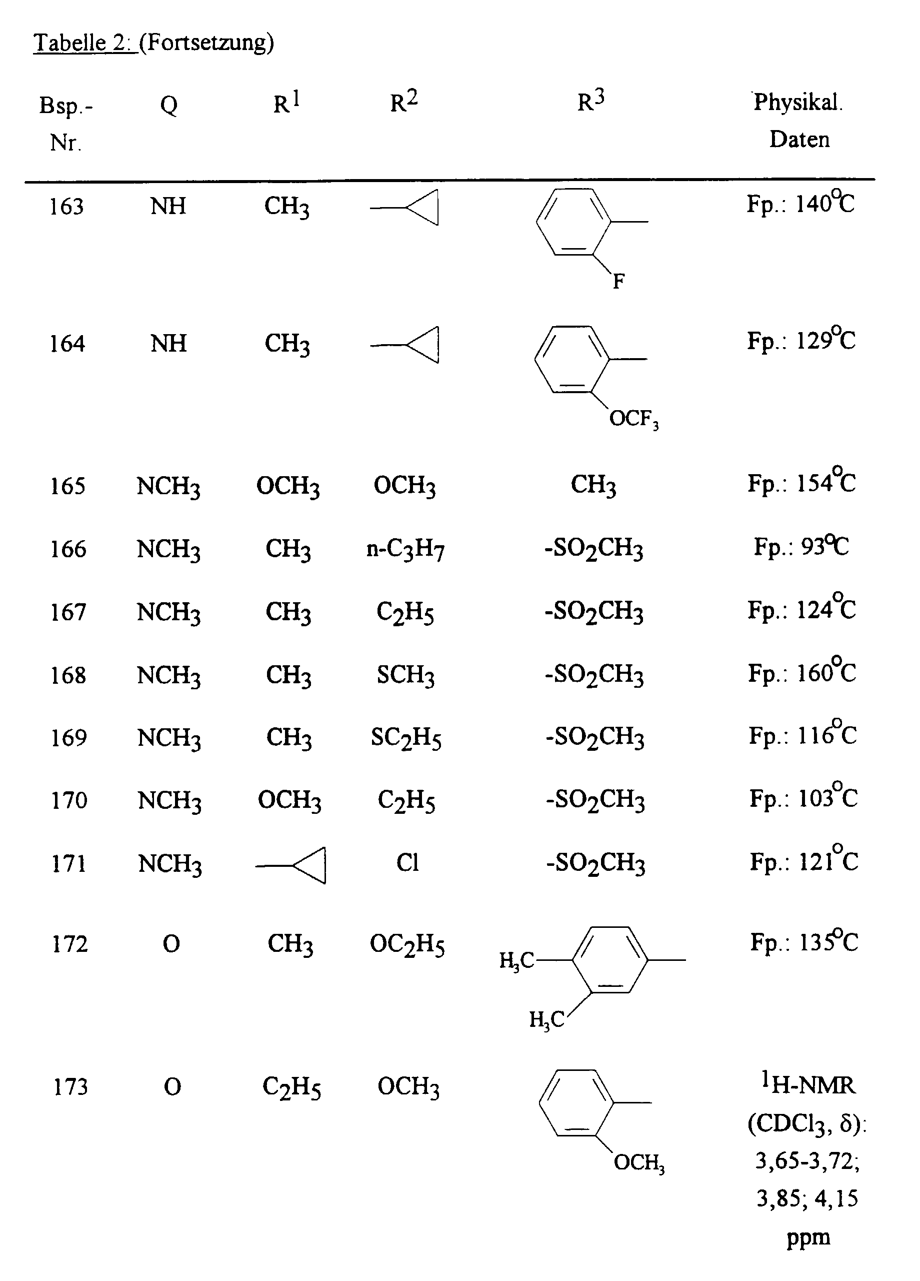

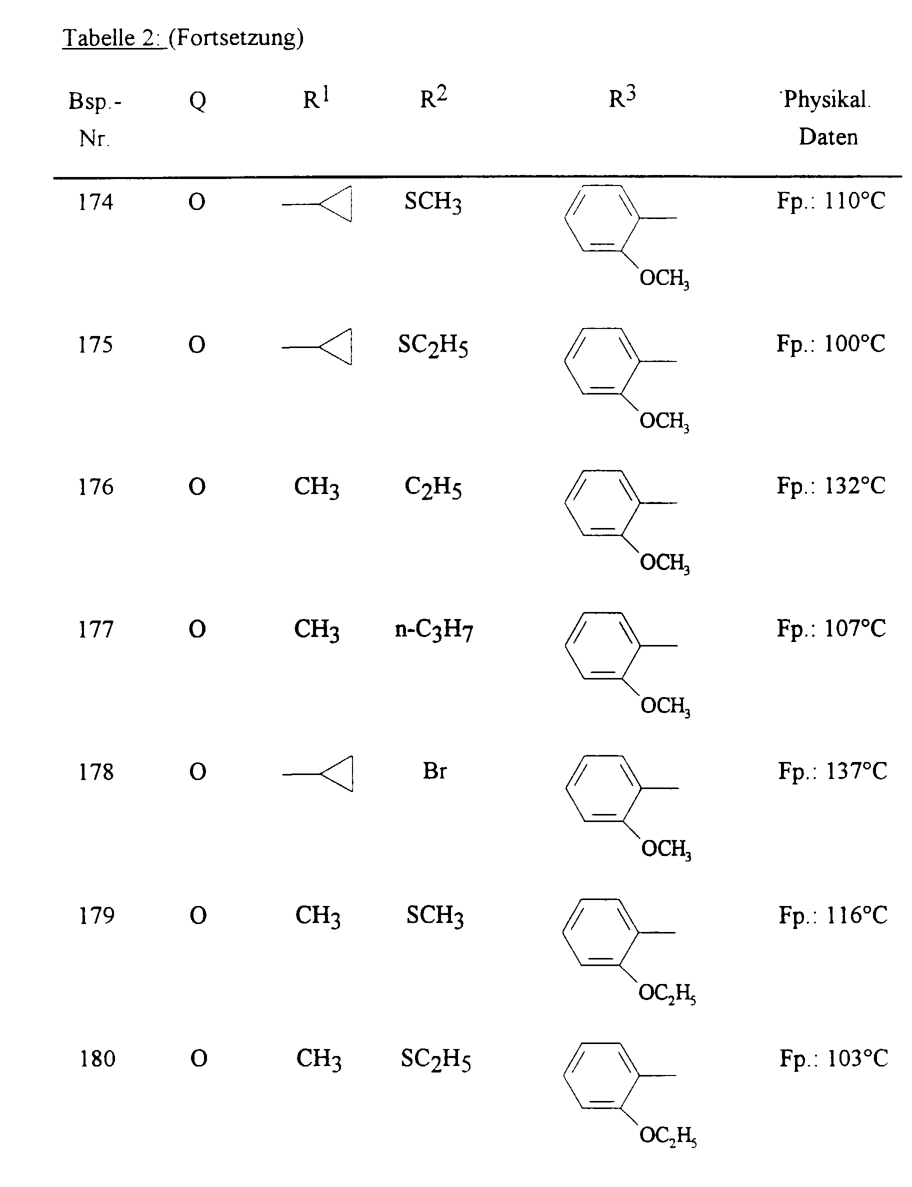

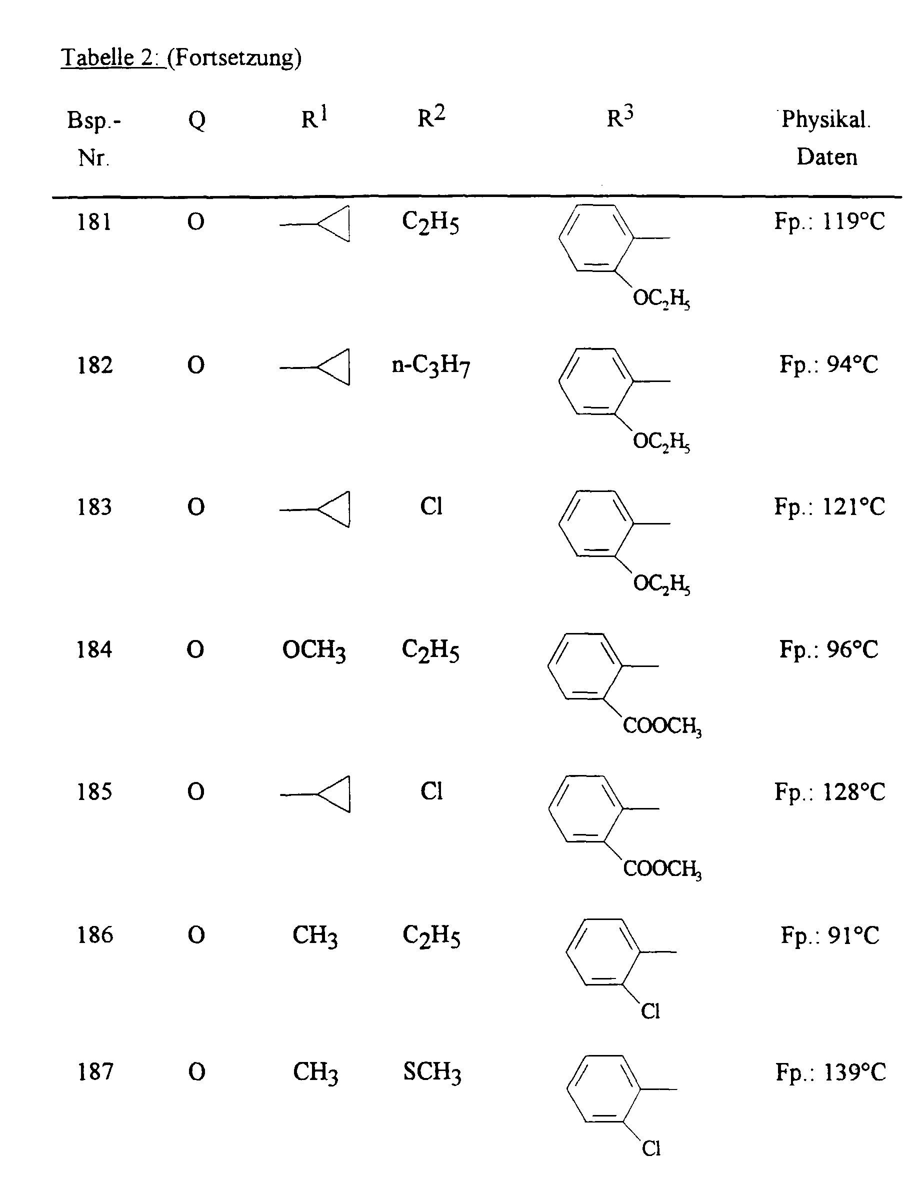

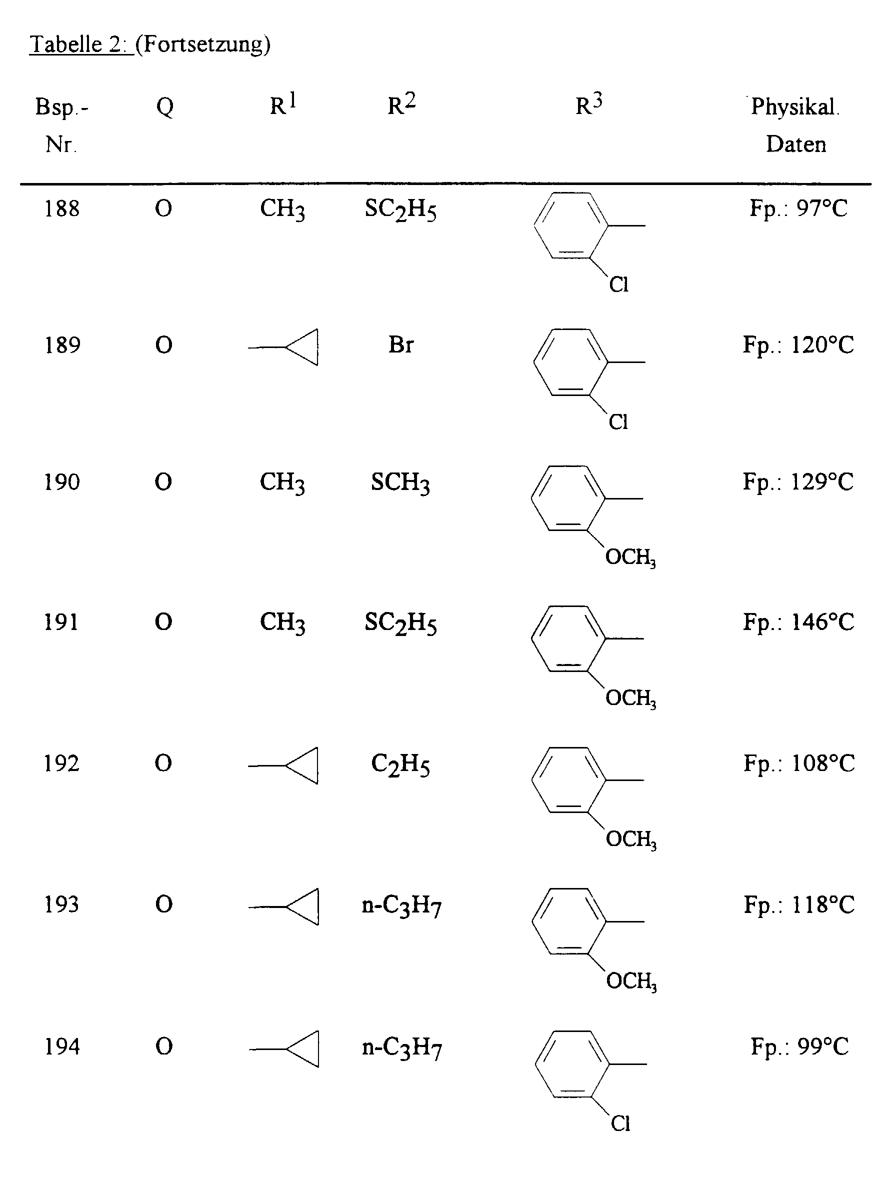

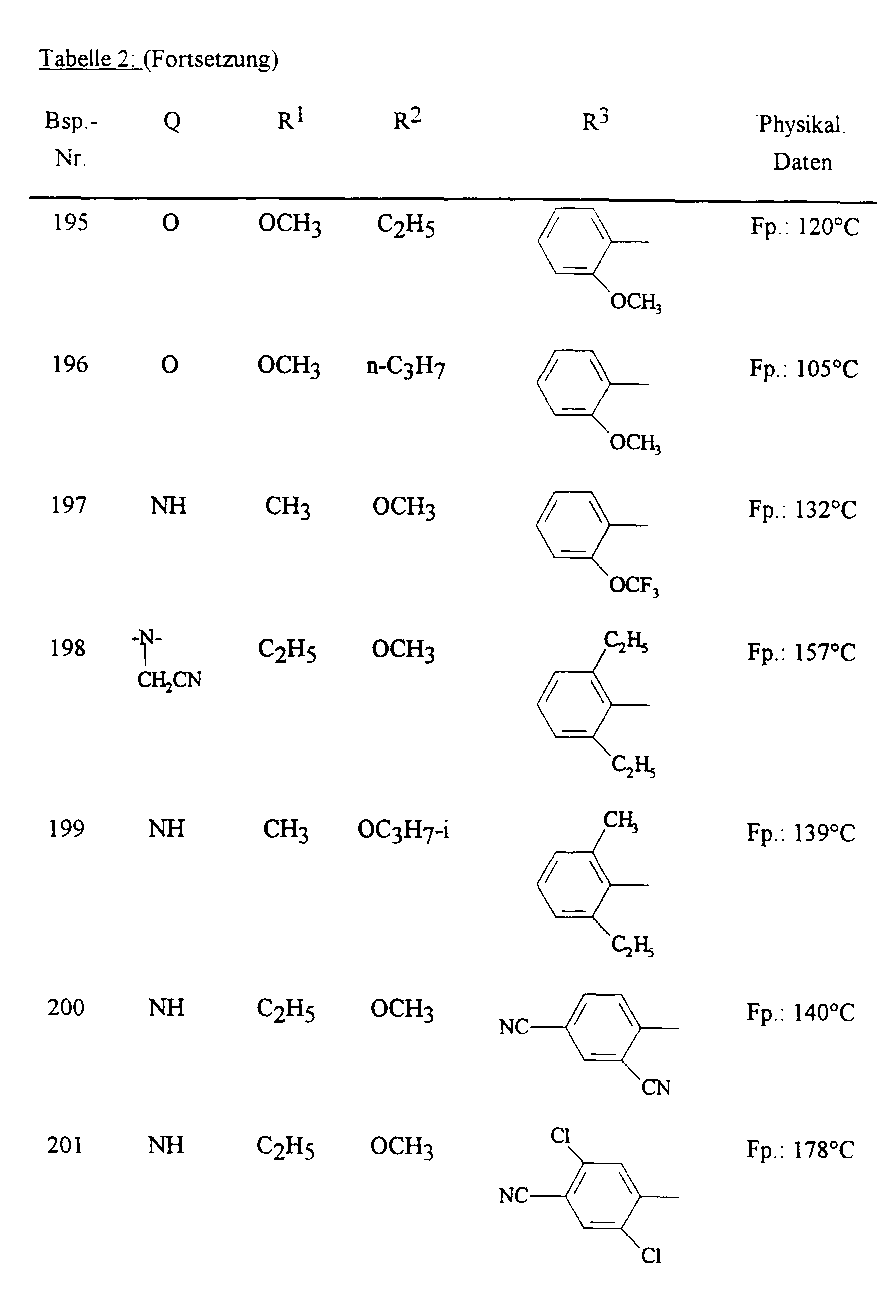

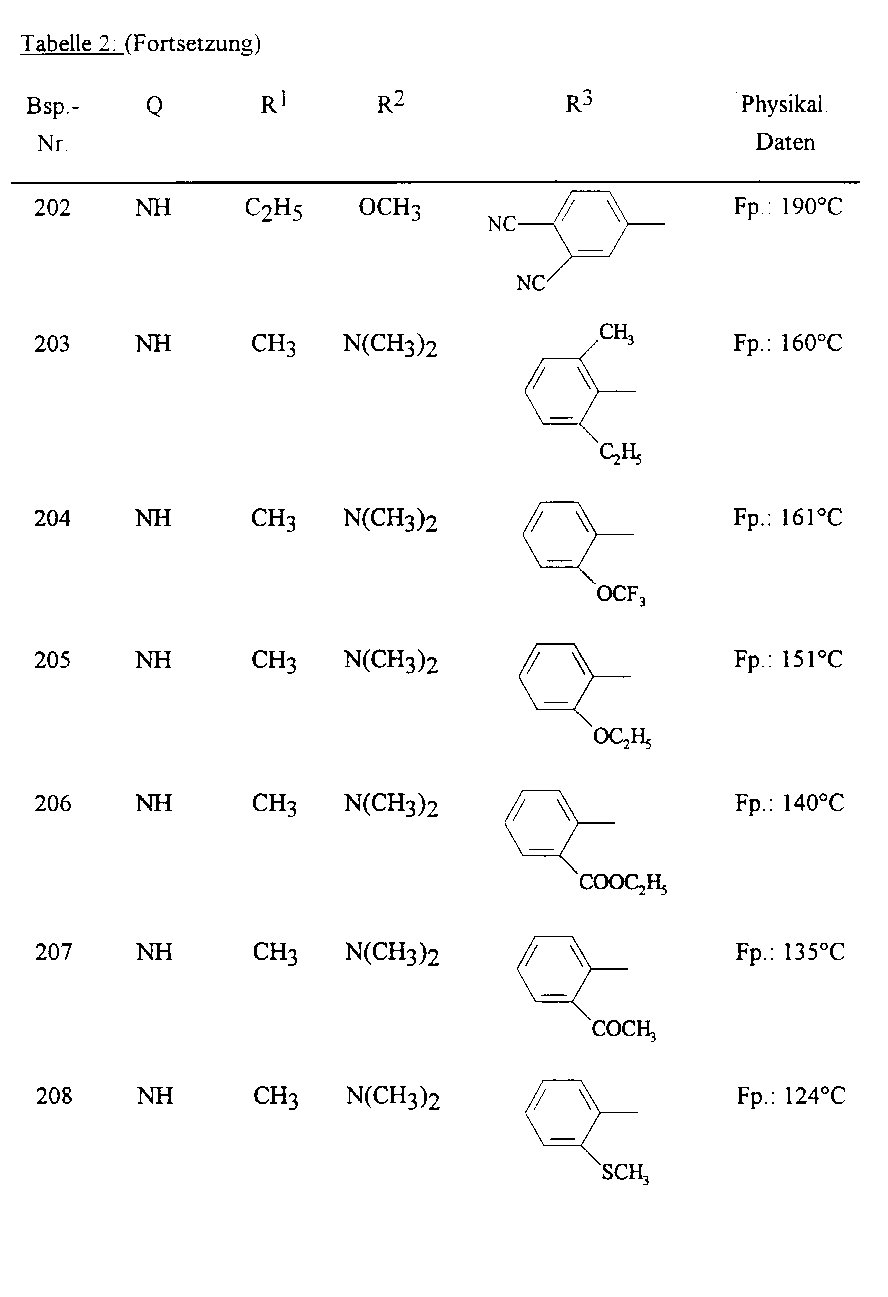

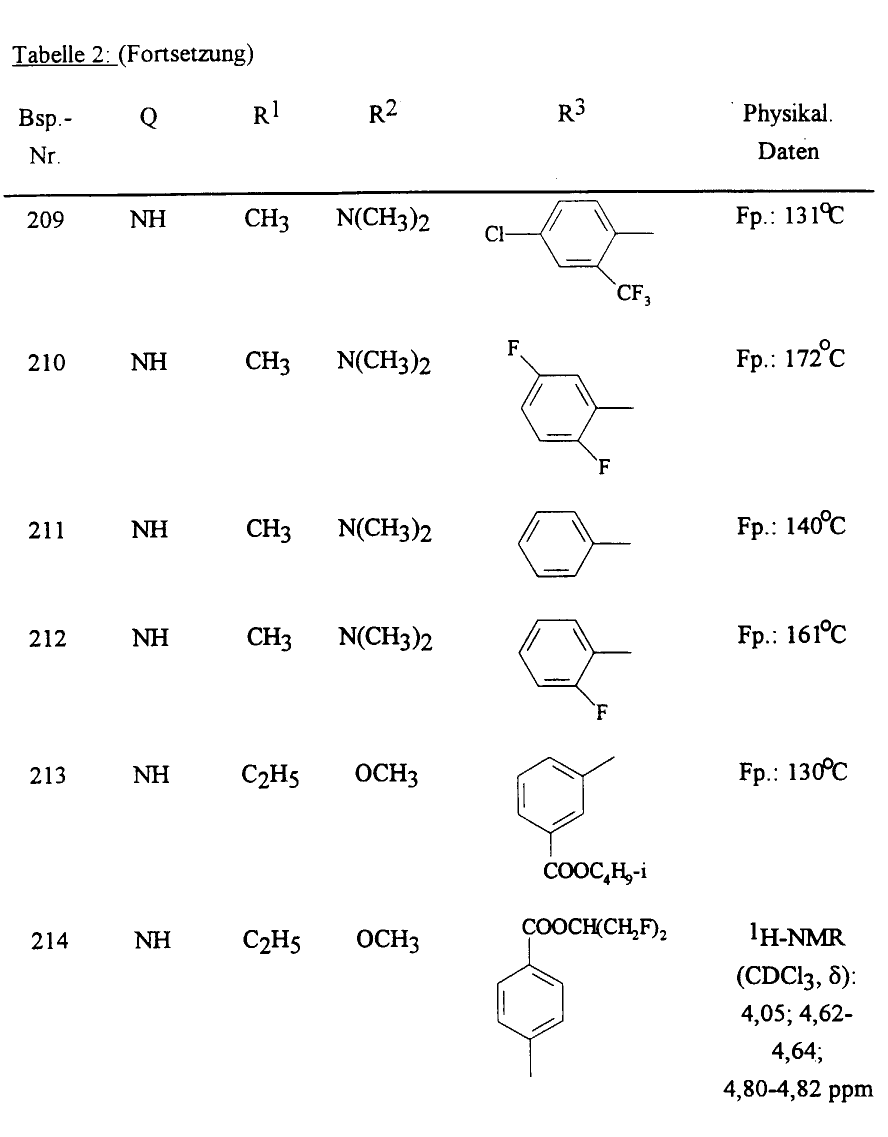

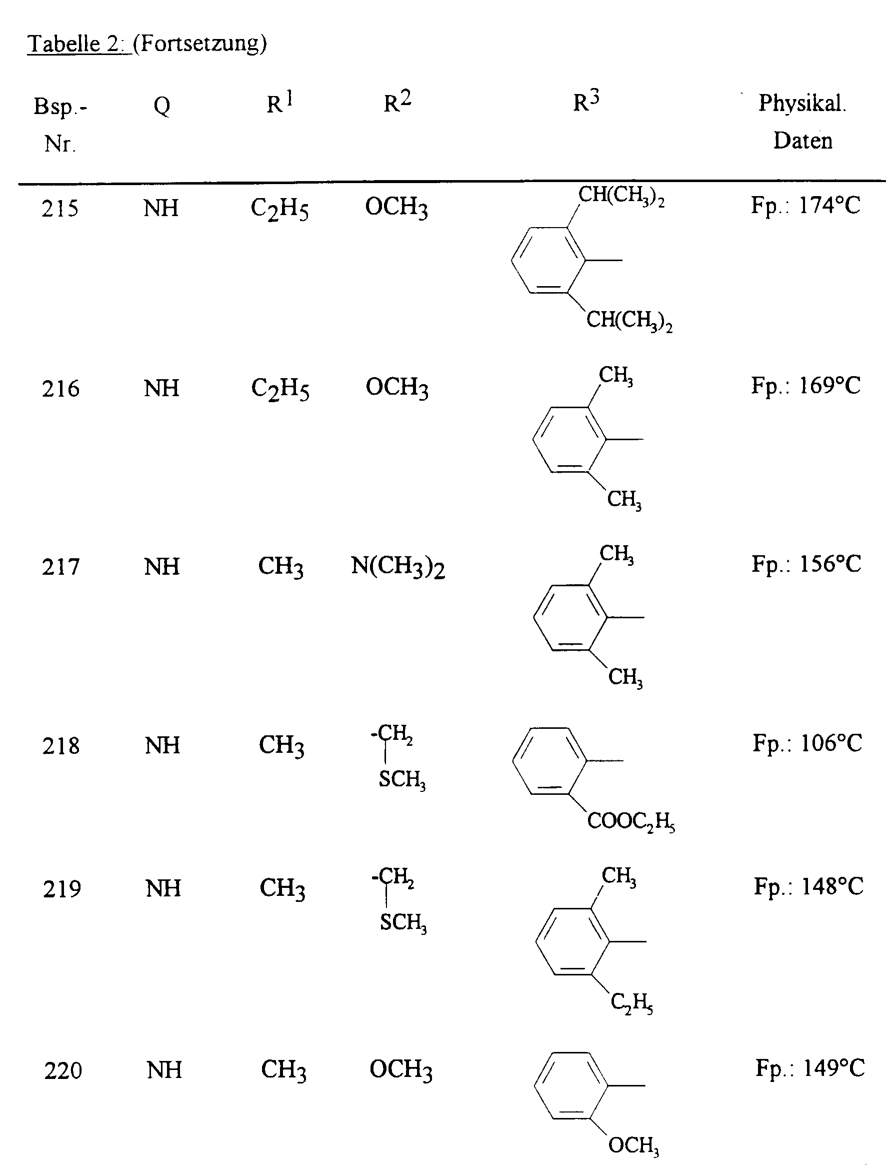

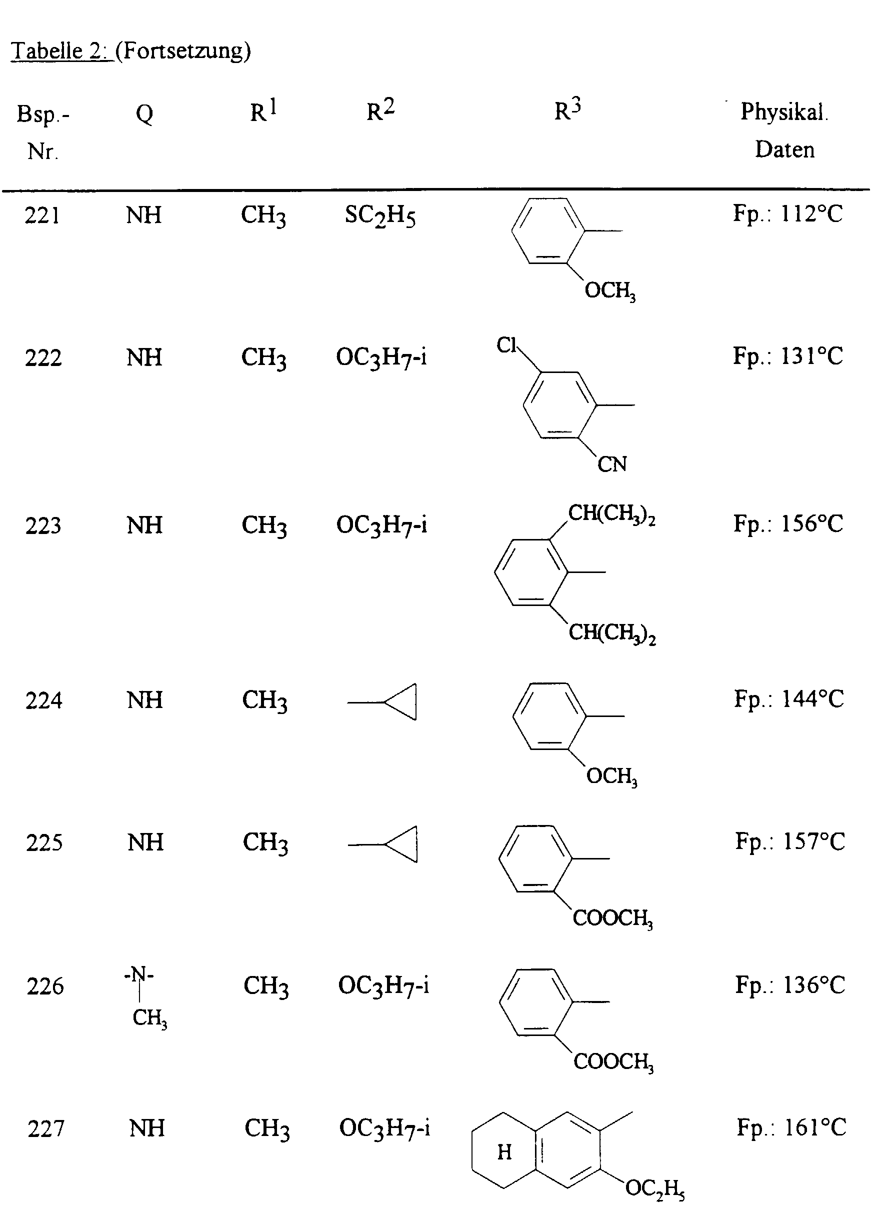

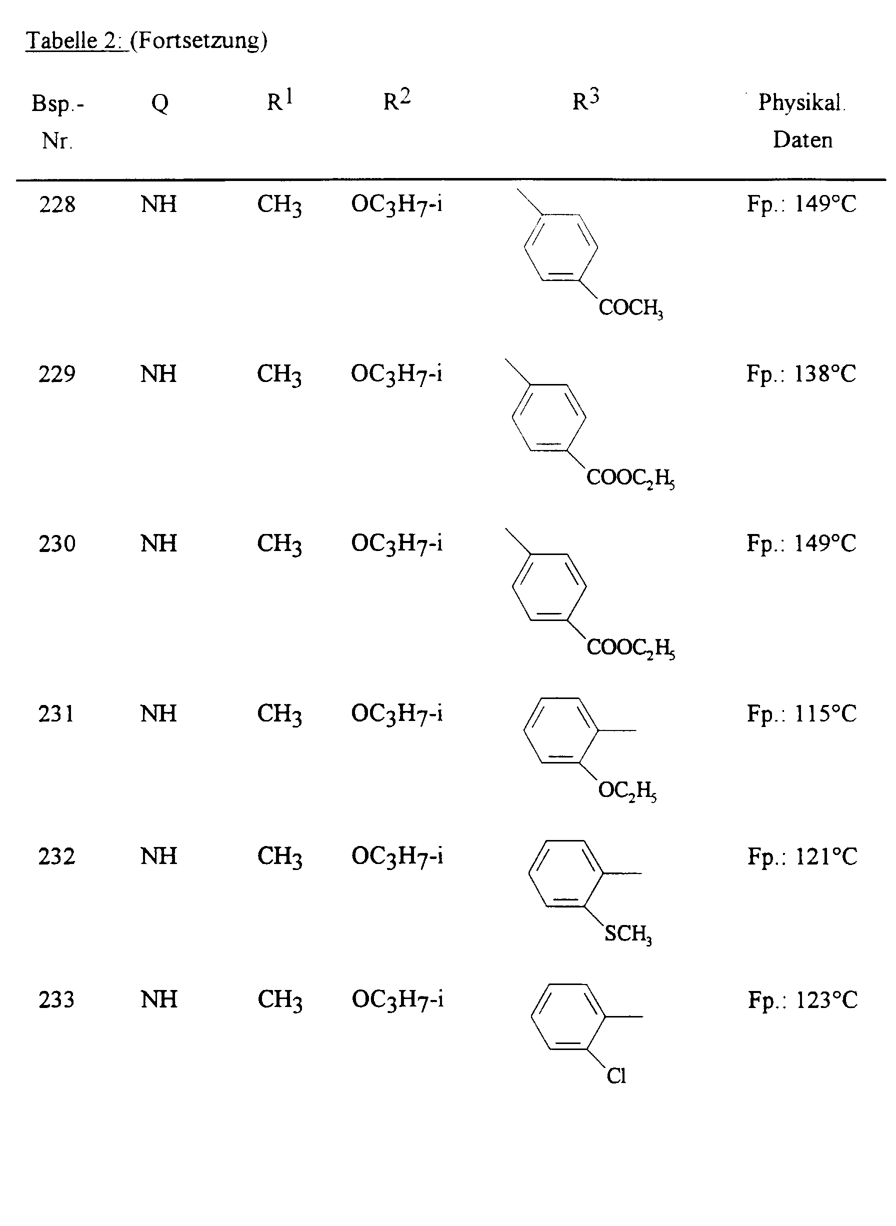

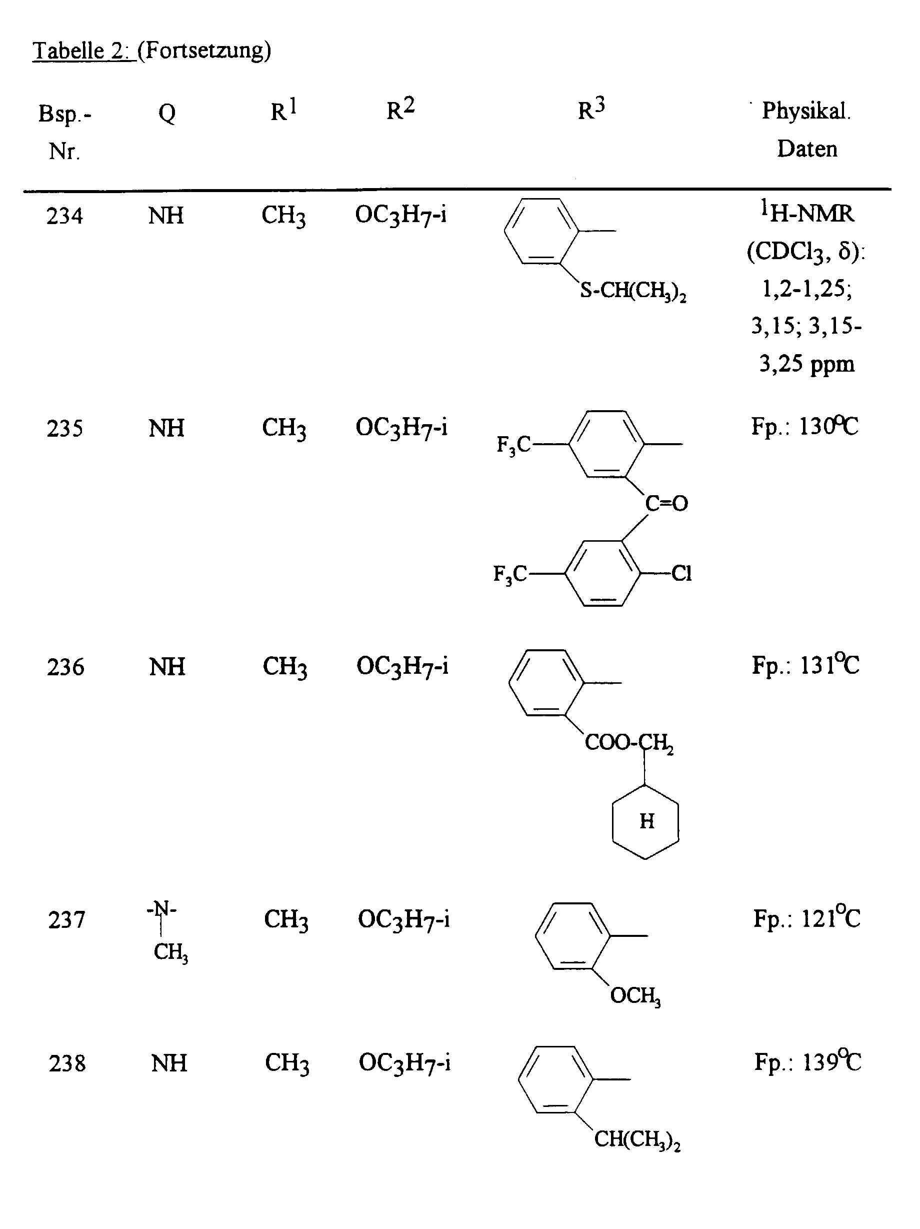

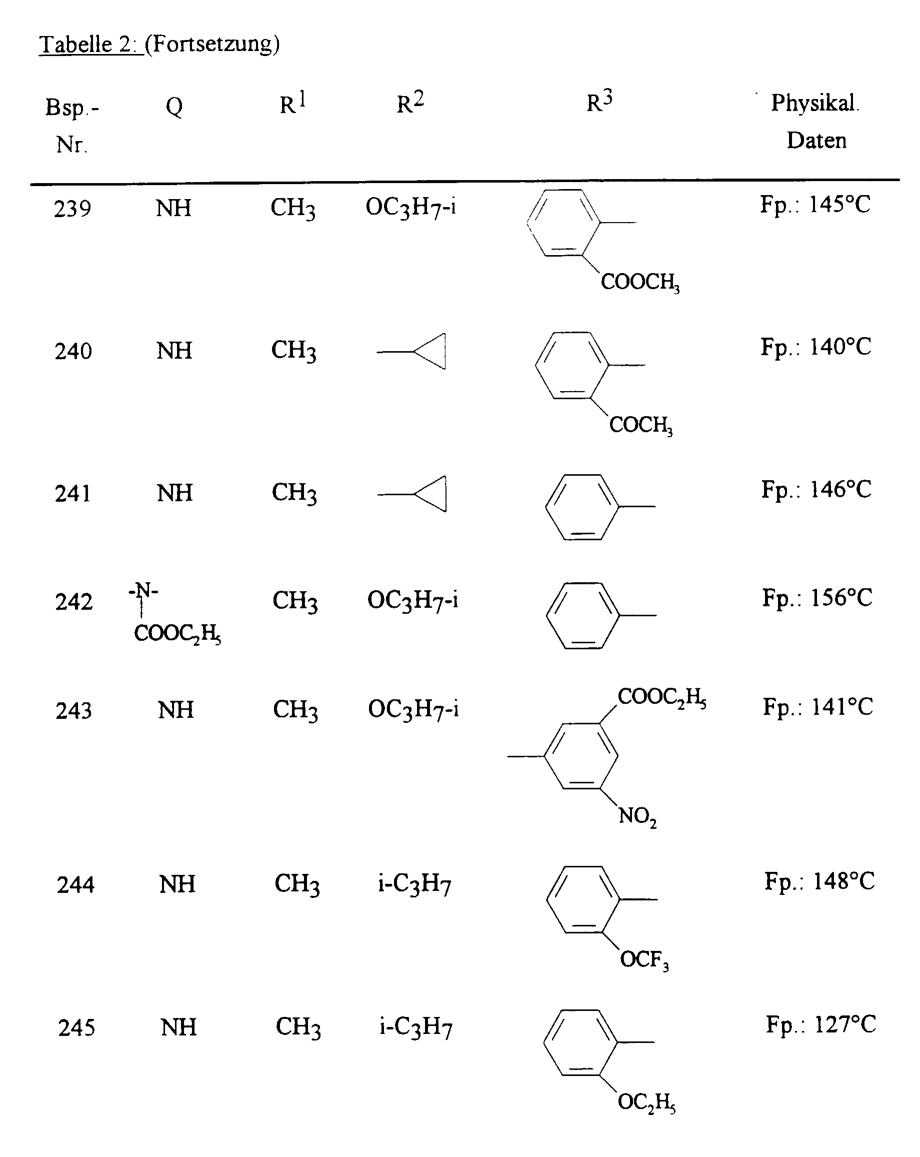

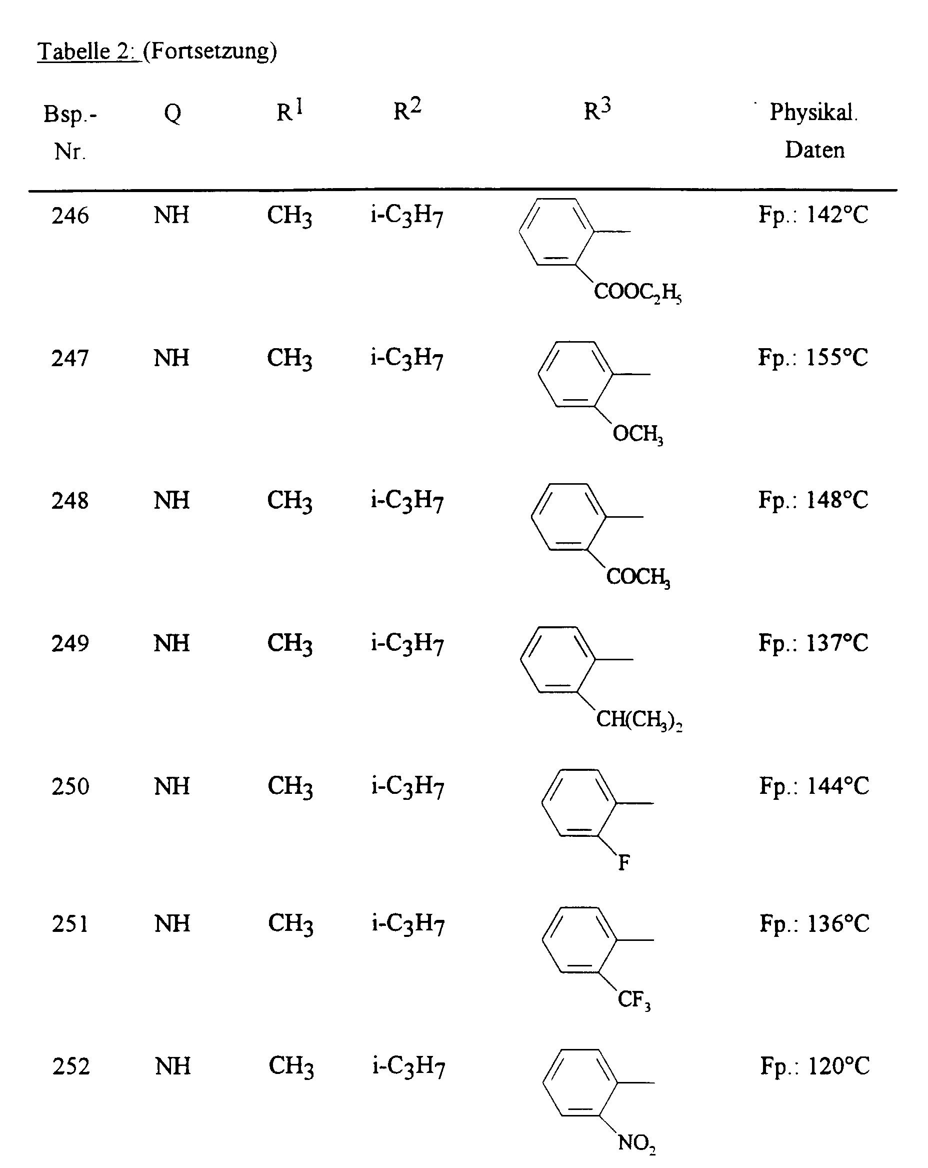

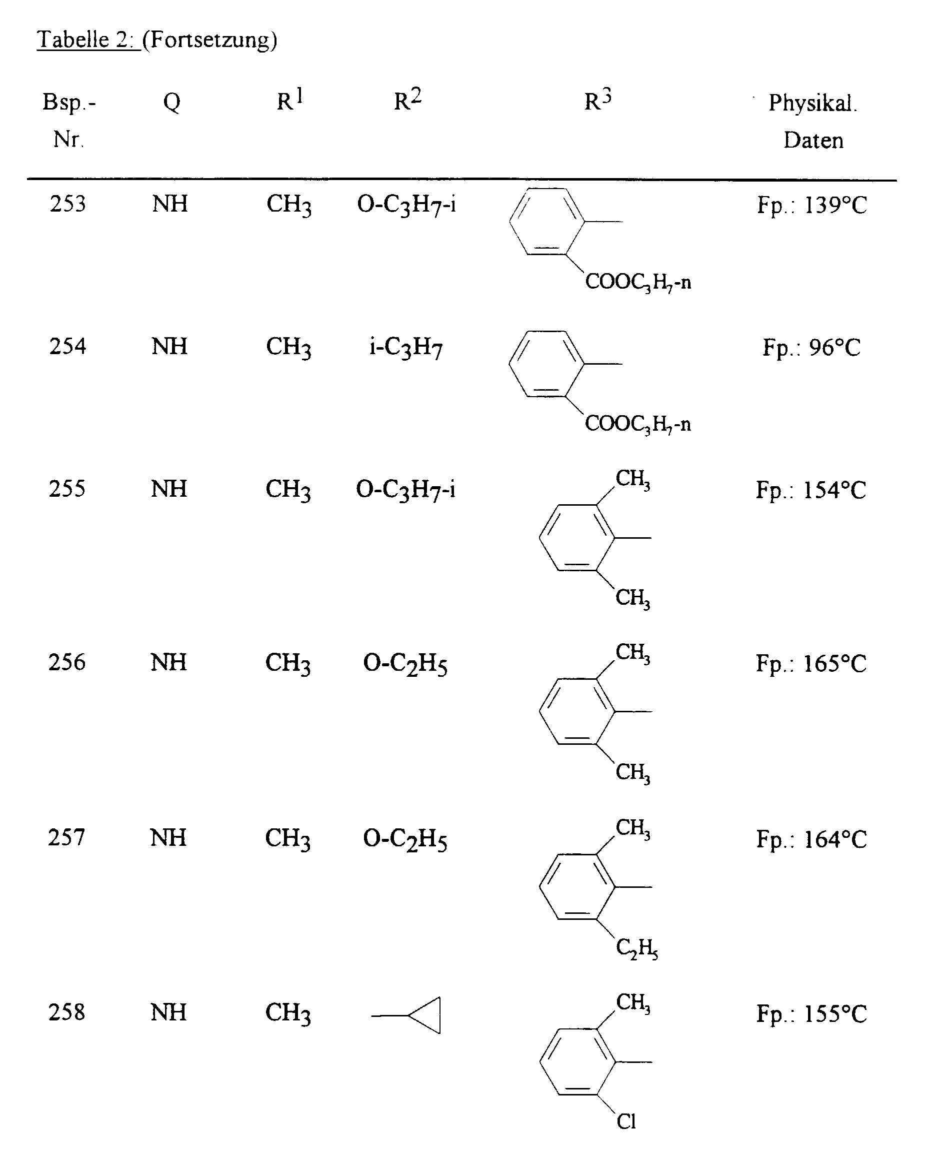

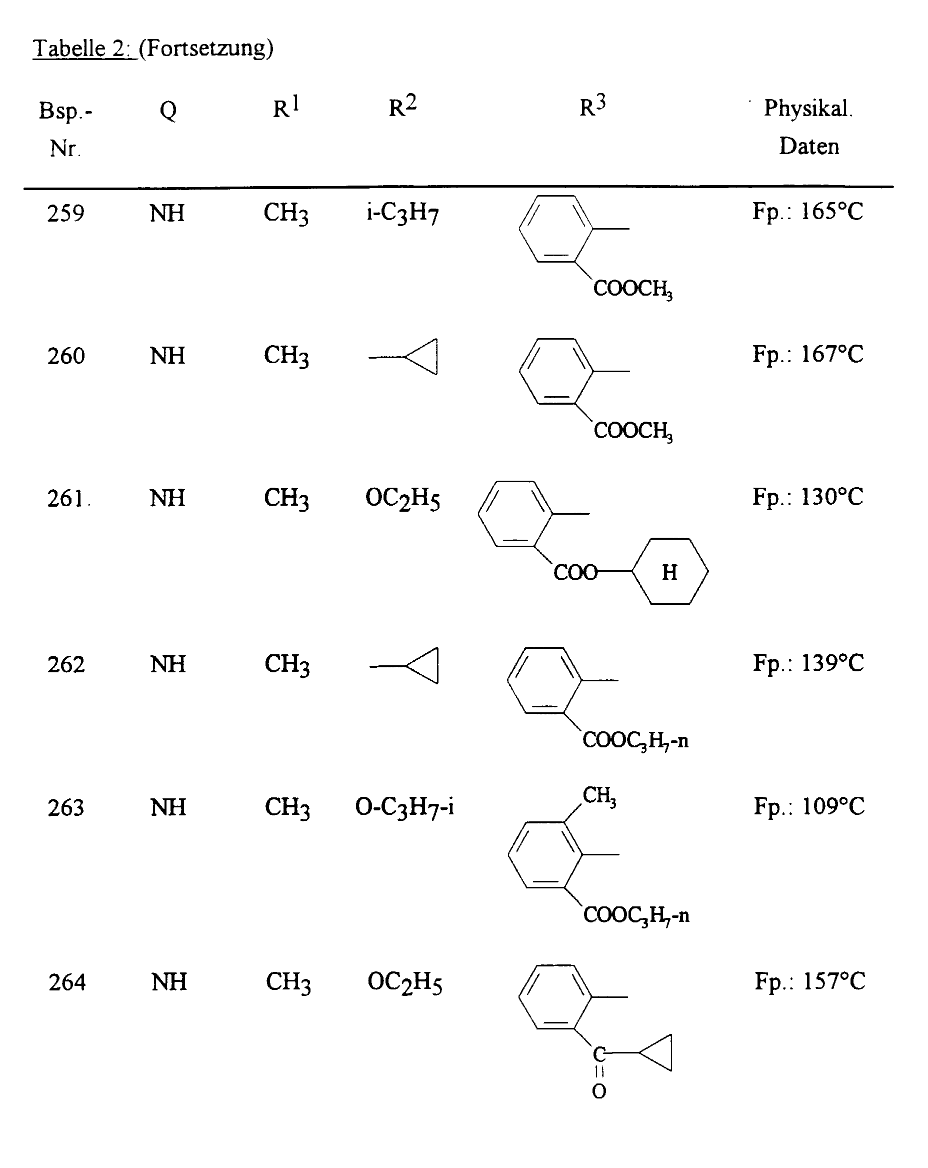

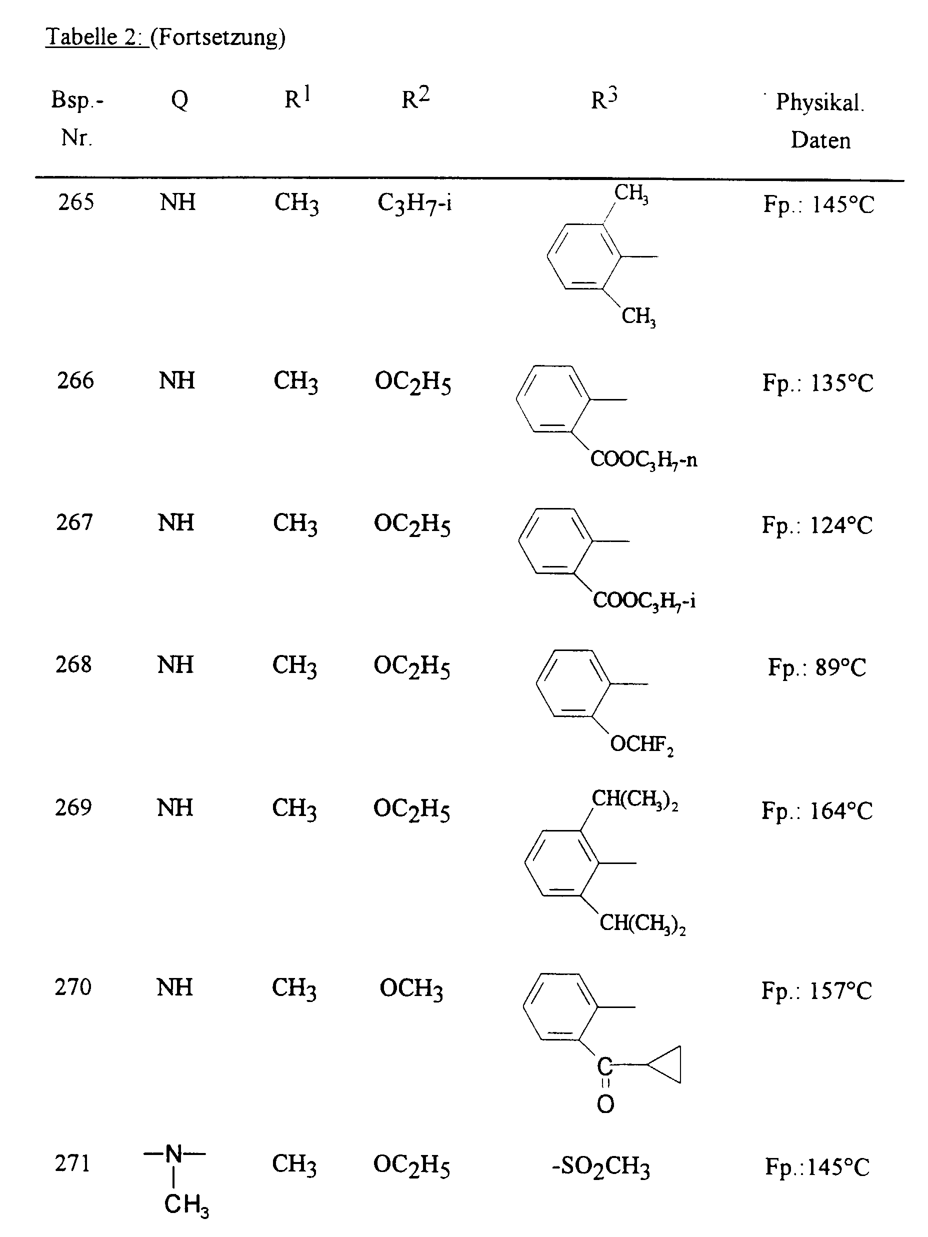

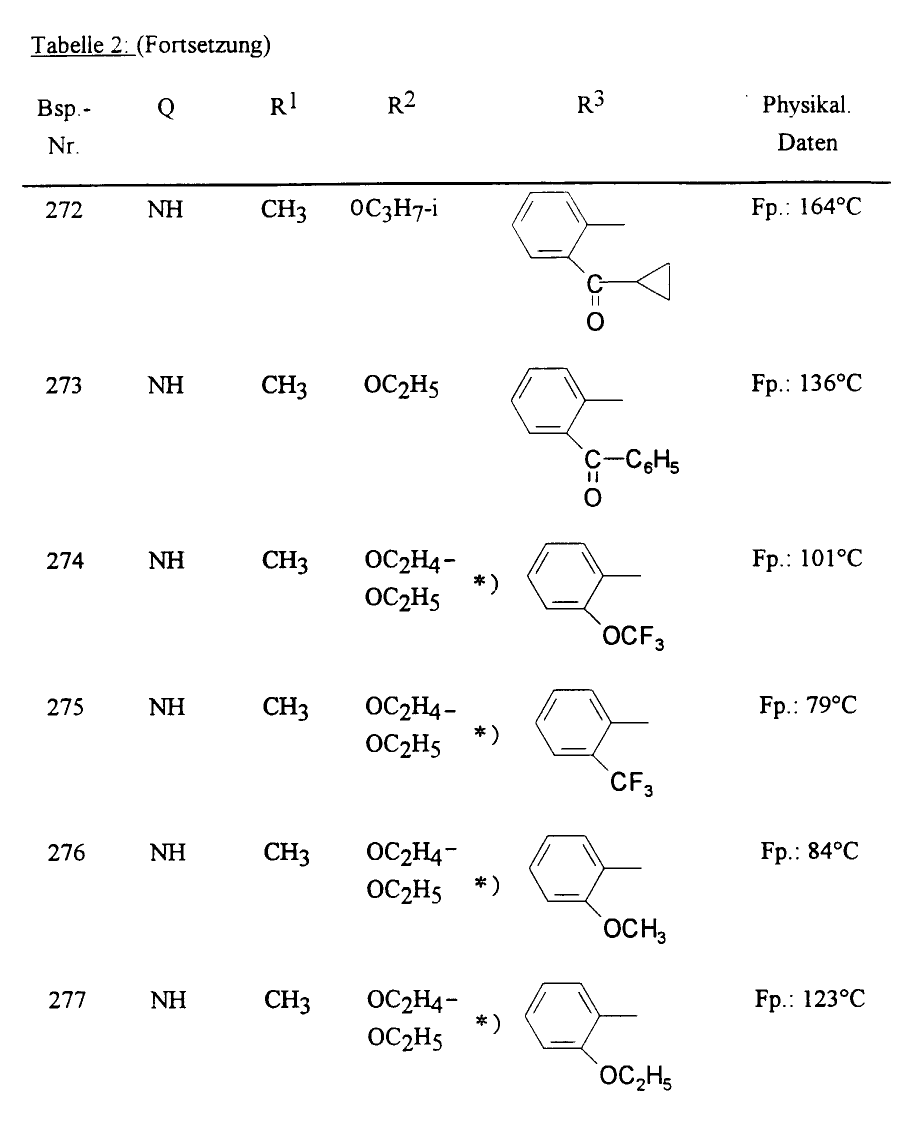

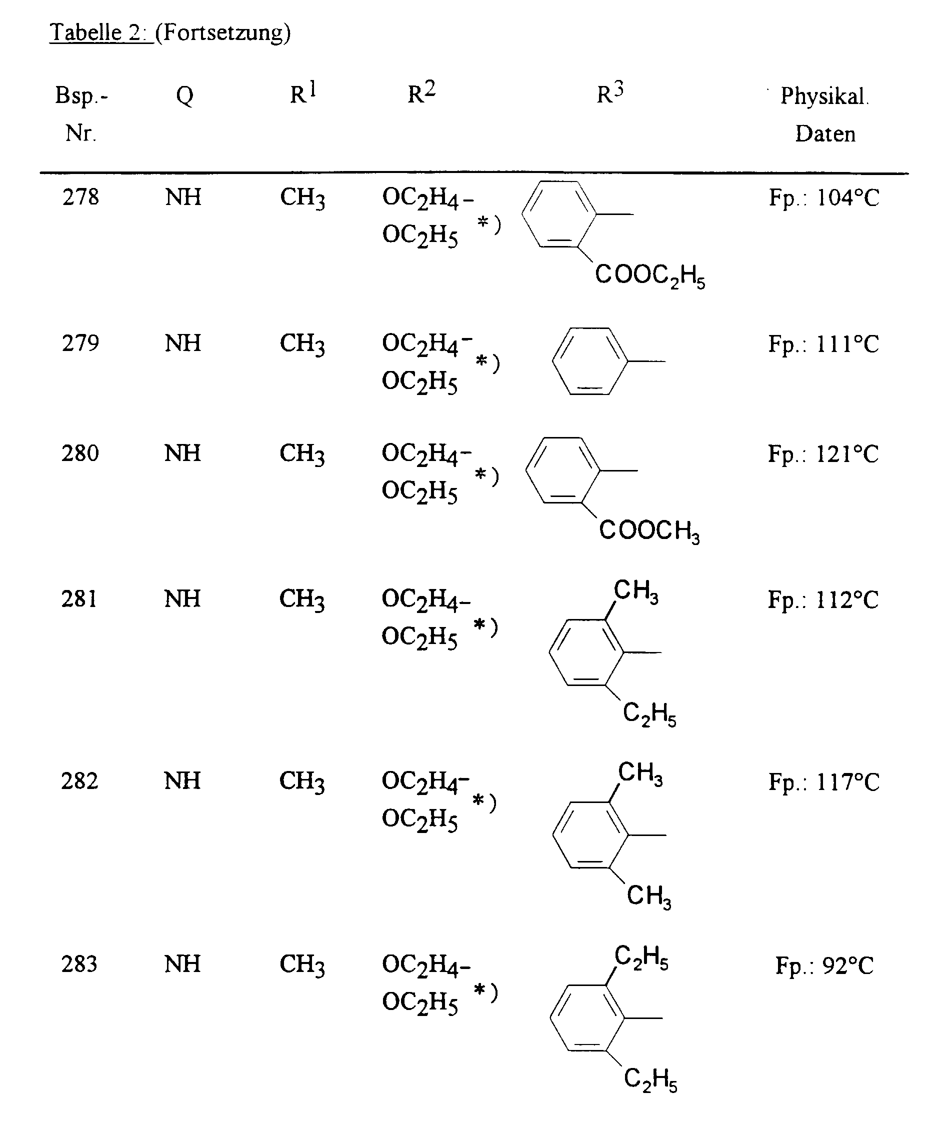

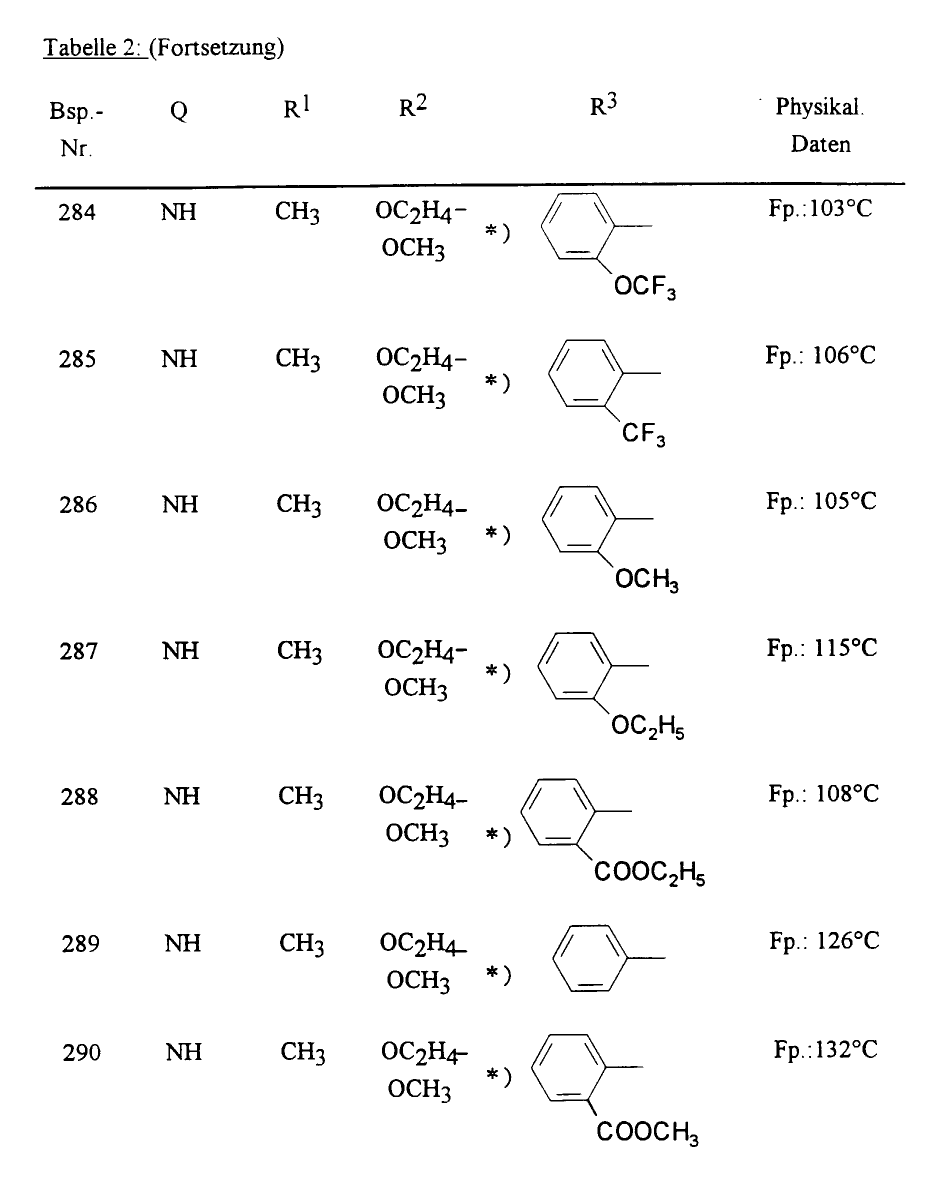

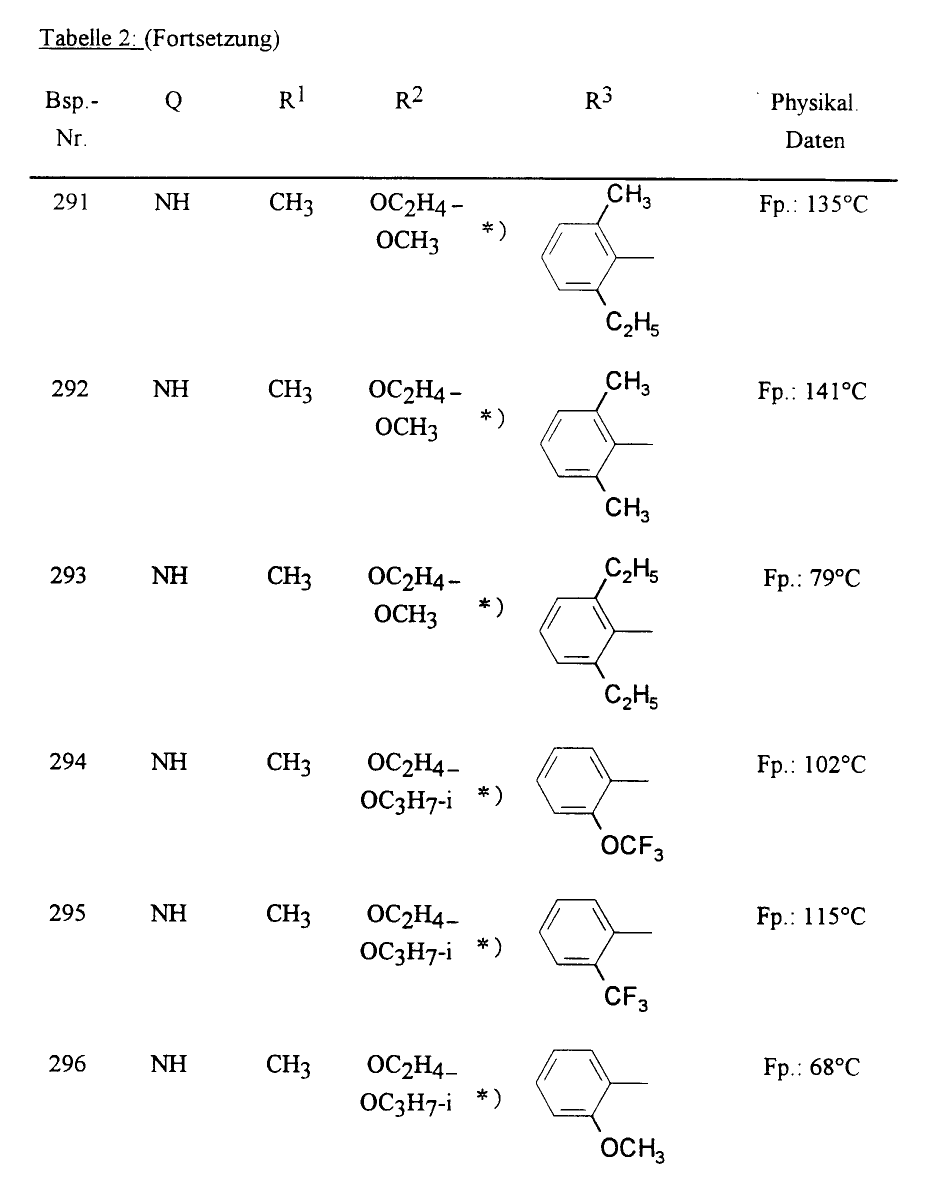

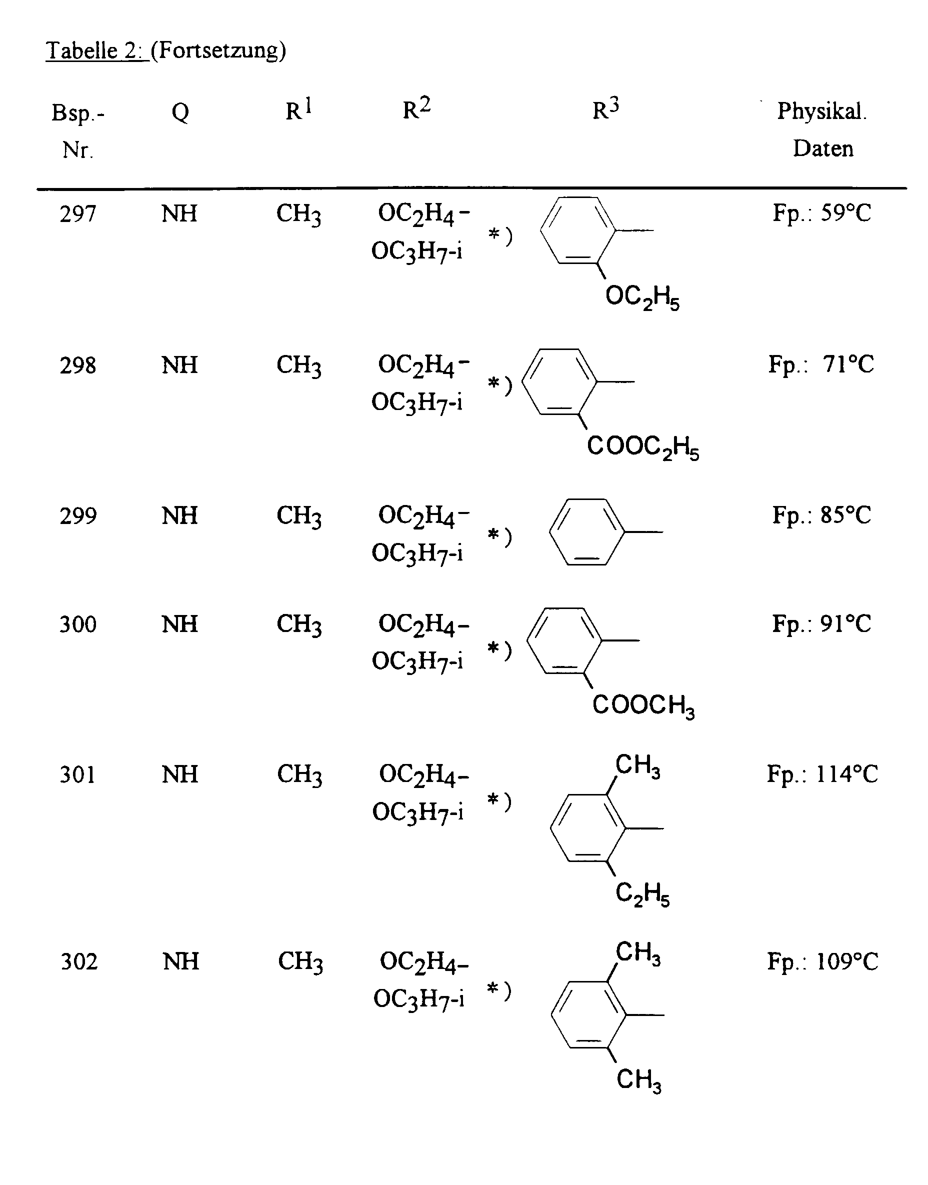

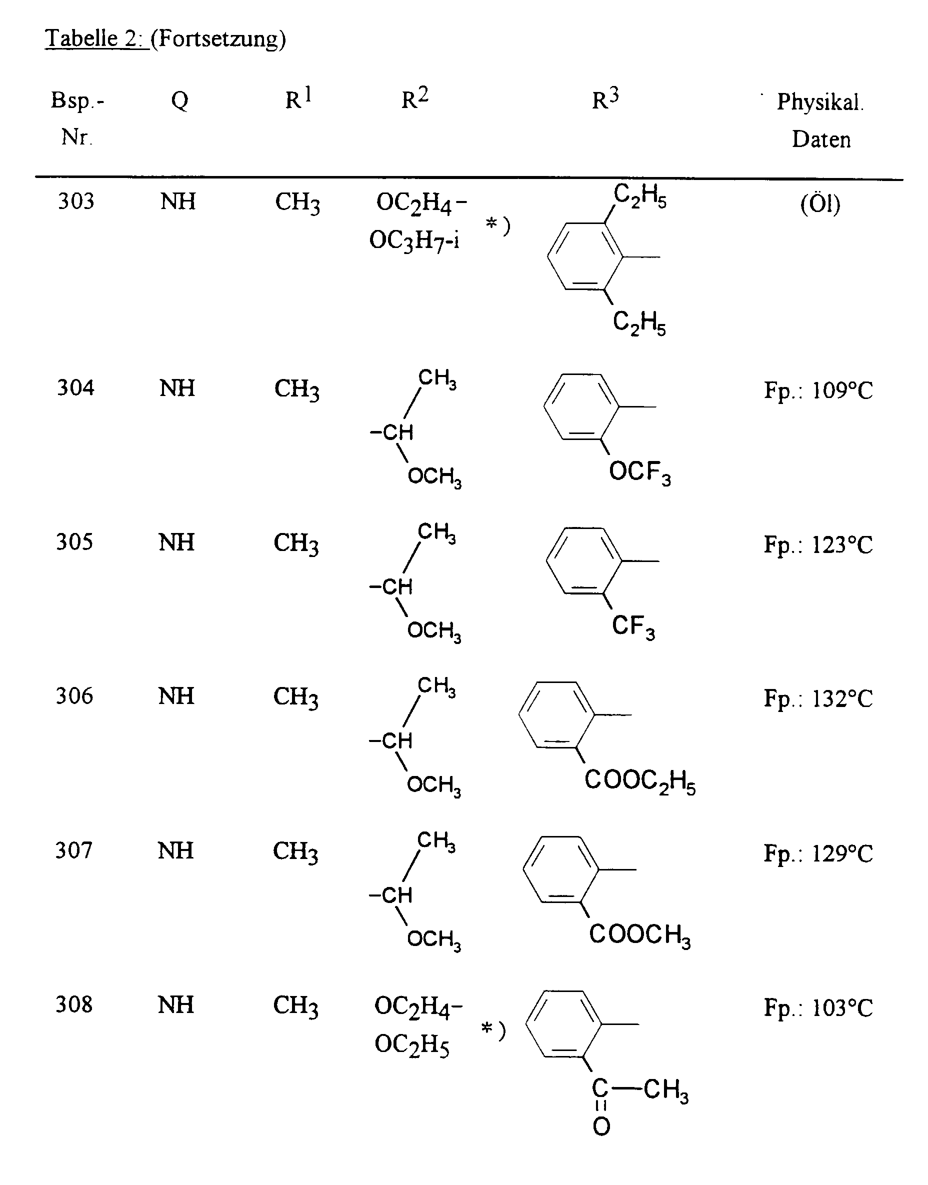

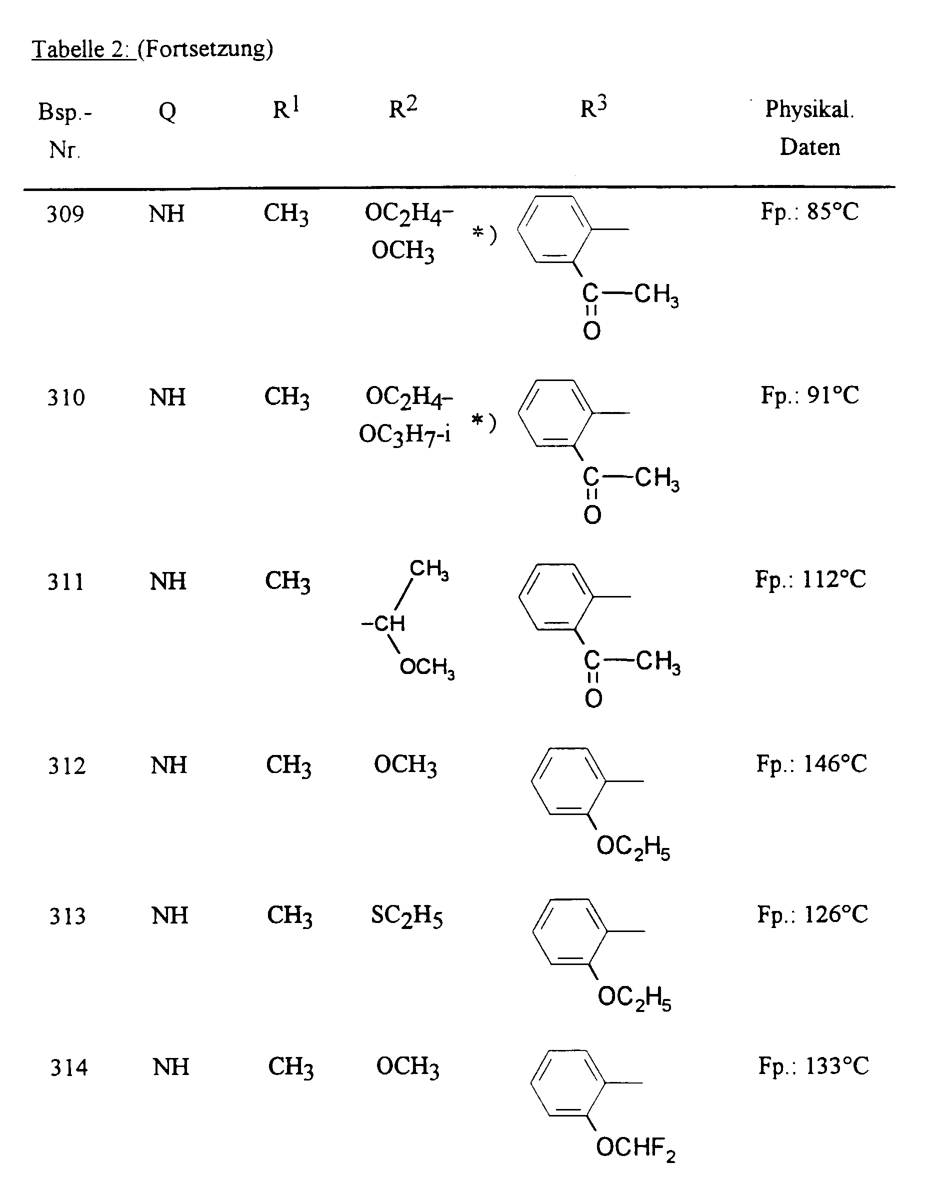

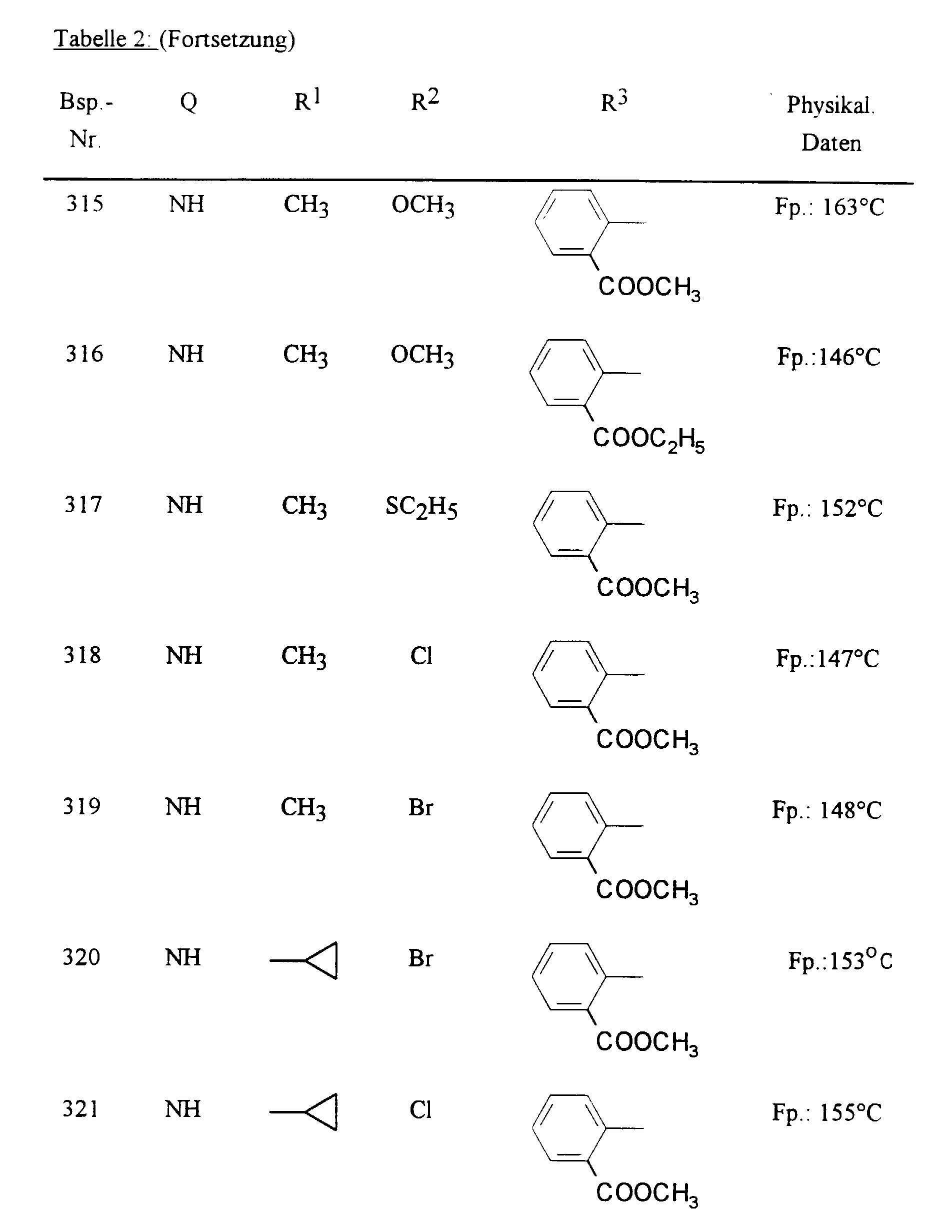

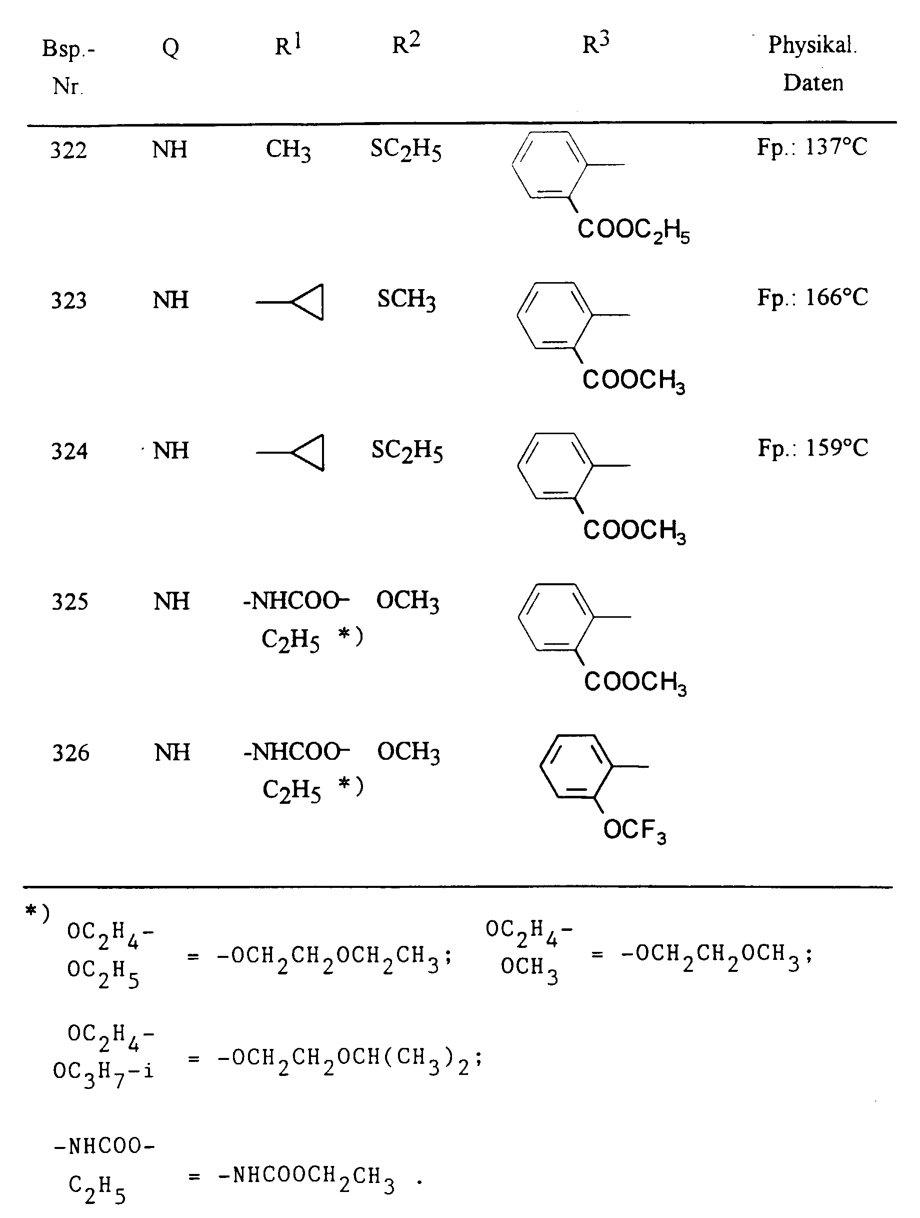

Die in Tabelle 2 als Beispiel 183 aufgeführte Verbindung kann beispielsweise auch wie folgt hergestellt werden [Verfahren(c)]: 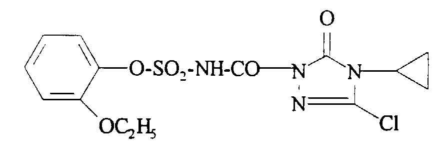 2,0 g (12,5 mMol) 4-Cyclopropyl-5-chlor-2,4-dihydro-3H-1,2,4-triazol-3-on werden in 40 ml Acetonitril gelöst und unter Rühren mit 4,3 g (26,9 mMol) (2-Ethoxy-phenoxy)-sulfonylisocyanat versetzt. Das Reaktionsgemisch wird 6 Stunden bei 20°C gerührt. Dann wird im Vakuum eingeengt, der Rückstand mit Diethylether verrührt und das kristalline Produkt durch Absaugen isoliert.

Man erhält 4,9 g (97% der Theorie) 2-(2-Ethoxy-phenyloxy)-sulfonylaminocarbonyl-4-cyclopropyl-5-chlor-2,4-dihydro-3H-1,2,4-triazol-3-on vom Schmelzpunkt 121°C.

### Ausgangsstoffe der Formel (II):

### Beispiel (II-1)

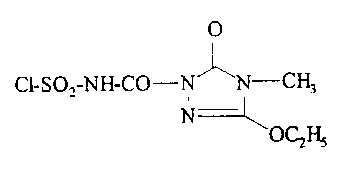

28,8 g (0,20 Mol) 5-Ethoxy-4-methyl-2,4-dihydro-3H-1,2,4-triazol-3-on werden in 250 ml Methylenchlorid vorgelegt und auf -10°C abgekühlt. Zu dieser Mischung werden 28,3 g (0,20 Mol) Chlorsulfonylisocyanat gegeben und das Reaktionsgemisch wird ohne Kühlen 30 Minuten gerührt. Dann wird das Lösungsmittel im Wasserstrahlvakuum sorgfältig abdestilliert.

Man erhält 53 g (93% der Theorie) 2-Chlorsulfonylaminocarbonyl-5-ethoxy-4-methyl-2,4-dihydro-3H-1,2,4-triazol-3-on als kristallinen Rückstand vom Schmelzpunkt 106°C.

### Beispiel (II-2)

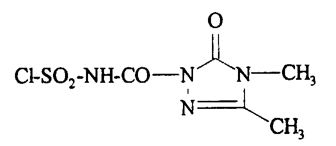

22,6 g (0,20 Mol) 4,5-Dimethyl-2,4-dihydro-3H-1,2,4-triazol-3-on werden in 250 ml Methylenchlorid vorgelegt und auf -10°C abgekühlt. Dann werden 28,3 g (0,20 Mol) Chlorsulfonylisocyanat dazu gegeben und die Mischung wird 20 Minuten bei -5°C bis -10°C gerührt, wobei zunächst eine klare Lösung entsteht und dann das Produkt sich kristallin abscheidet. Es wird durch Absaugen isoliert.

Man erhält 45 g (88% der Theorie) 2-Chlorsulfonylaminocarbonyl-4,5-dimethyl-2,4-dihydro-3H-1,2,4-triazol-3-on vom Schmelzpunkt 150°C (unter Zersetzung).

### Beispiel (II-3)

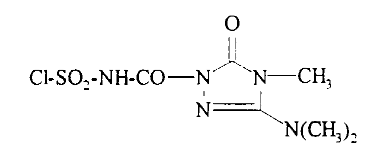

10 g (72 mMol) 5-Dimethylamino-4-methyl-2,4-dihydro-3H-1,2,4-triazol-3-on werden in 250 ml Methylenchlorid bei -10°C bis 0°C langsam mit 9,9 g (72 mMol) Chlorsulfonylisocyanat versetzt. Nach dreißigminütigem Rühren hat die Reaktionsmischung Raumtemperatur (20°C) erreicht. Das Lösungsmittel wird dann durch Destillation im Wasserstrahlvakuum entfernt.

Man erhält 19,1 g (94% der Theorie) 2-Chlorsulfonylaminocarbonyl-5-dimethylamino-4-methyl-2,4-dihydro-3H-1,2,4-triazol-3-on als amorphen Rückstand.

### Anwendungsbeispiele:

In den Anwendungsbeispielen wird die folgende Verbindung (A) als Vergleichssubstanz herangezogen: 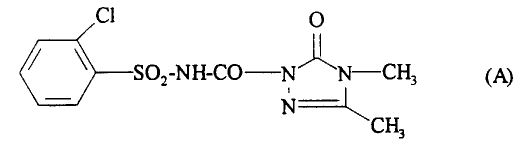 2-(2-Chlor-phenylsulfonylaminocarbonyl)-4,5-dimethyl-2,4-dihydro-3H-1,2,4-triazol-3-on (bekannt aus EP-A 341489).

### Beispiel A

### Pre-emergence-Test

### Lösungsmittel: 5 Gewichtsteile Aceton

### Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:
- O % =: keine Wirkung (wie unbehandelte Kontrolle)
- 100 % =: totale Vernichtung

Eine deutliche Überlegenheit in der Wirksamkeit gegenüber dem Stand der Technik zeigen in diesem Test z.B. die Verbindungen gemäß folgender Herstellungsbeispiele: 2, 4, 13, 33, 39, 41, 43, 45, 58, 64 und 65.

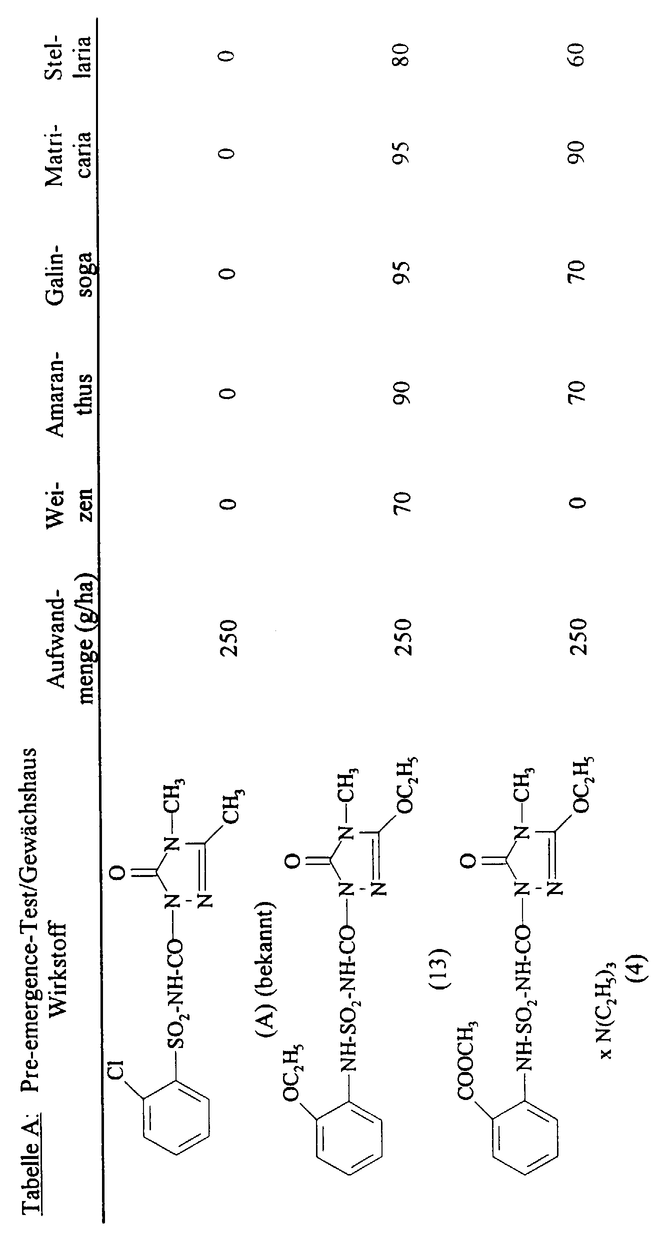

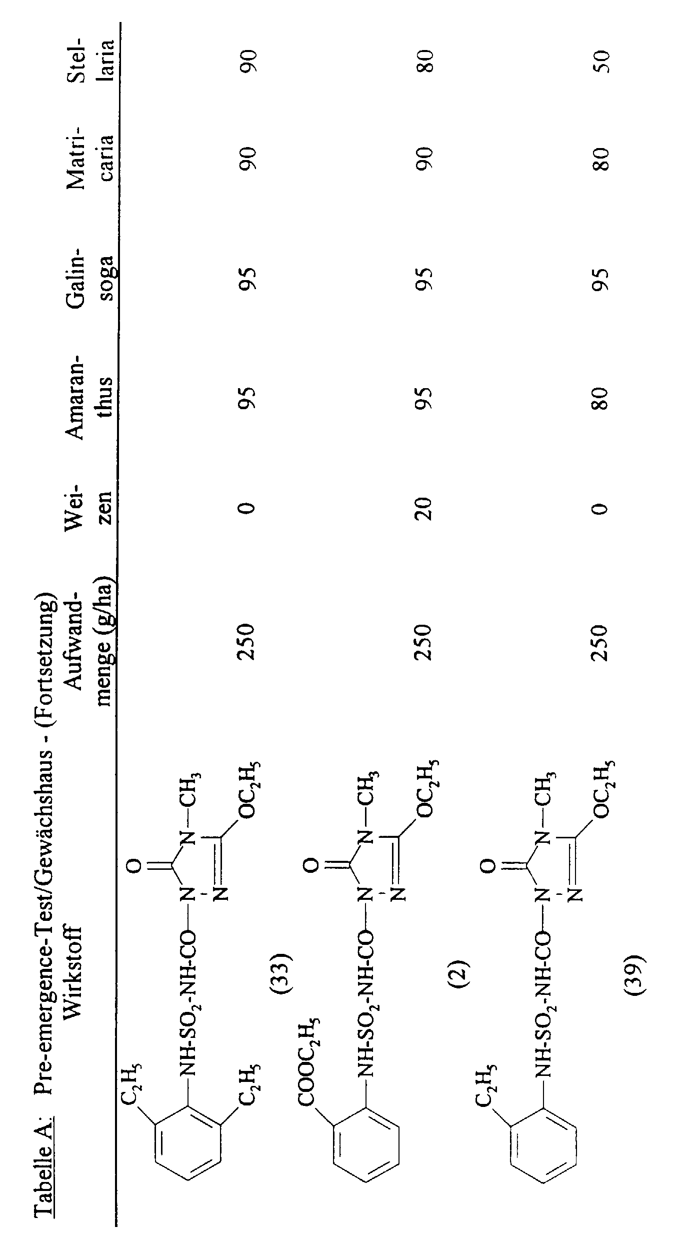

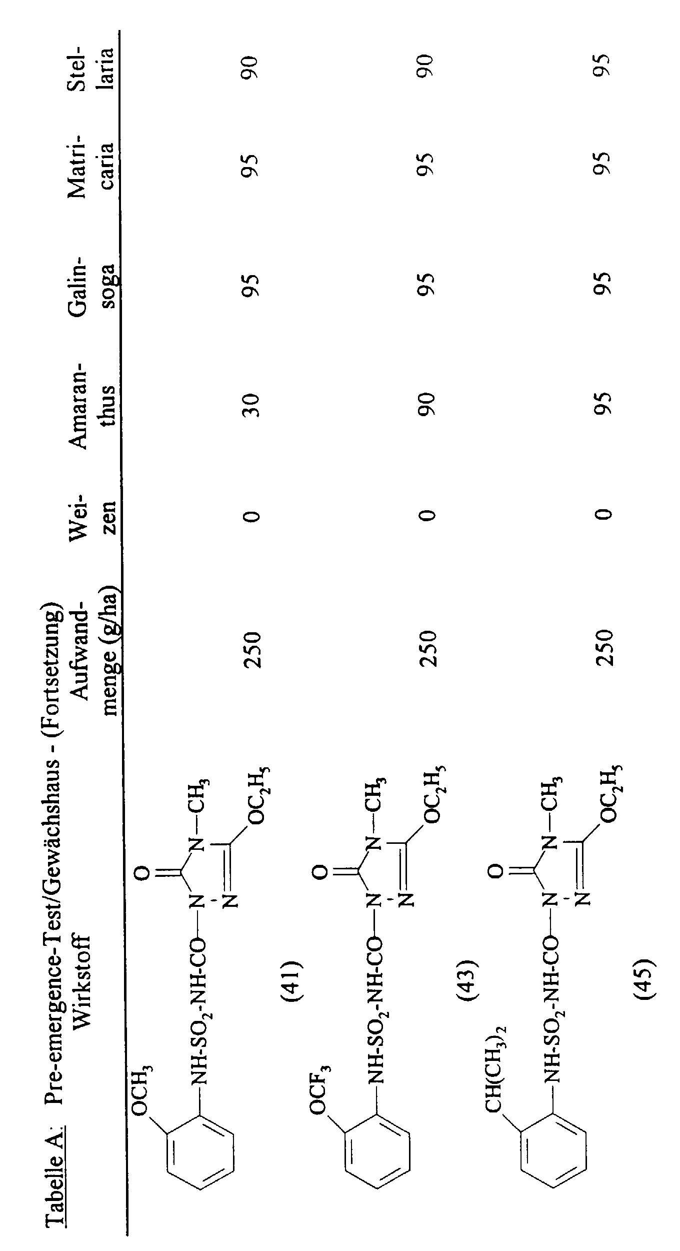

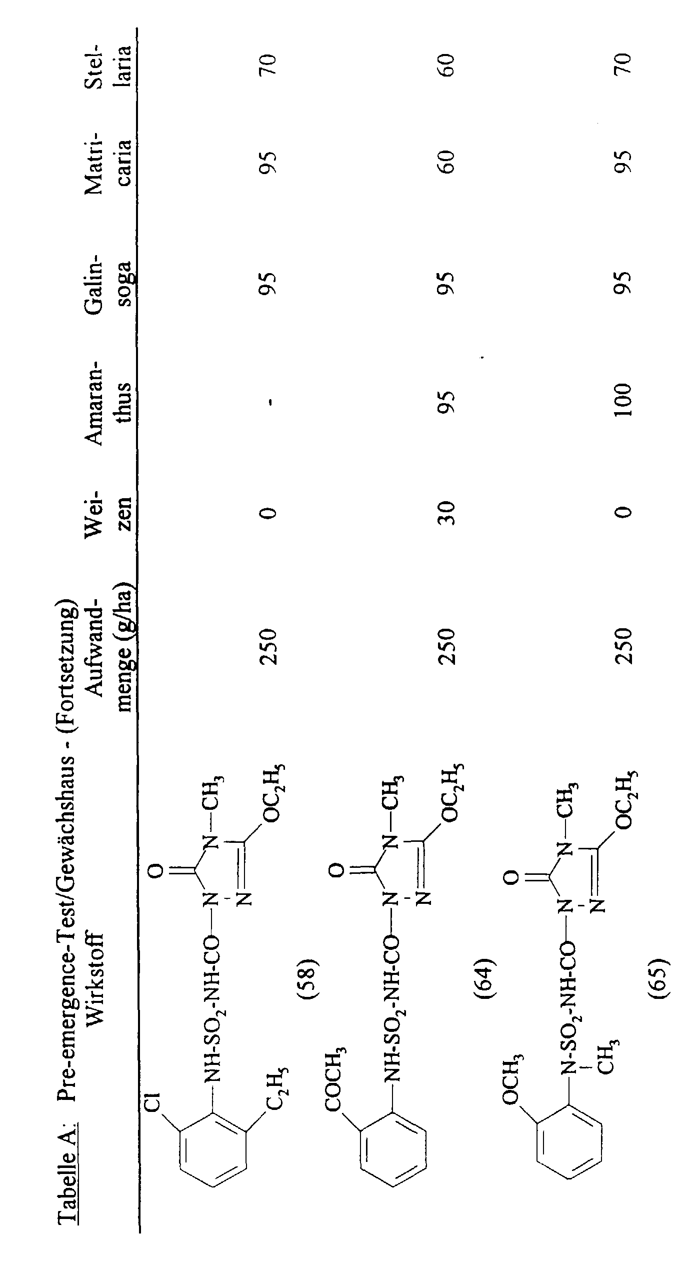

### Beispiel B

### Post-emergence-Test

### Lösungsmittel: 5 Gewichtsteile Aceton

### Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 1 Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:
- O % =: keine Wirkung (wie unbehandelte Kontrolle)
- 100 % =: totale Vernichtung

Eine deutliche Überlegenheit in der Wirksamkeit gegenüber dem Stand der Technik zeigen in diesem Test z.B. die Verbindungen gemäß folgender Herstellungsbeispiele: 2, 4, 13, 14, 33, 39, 41 und 43.

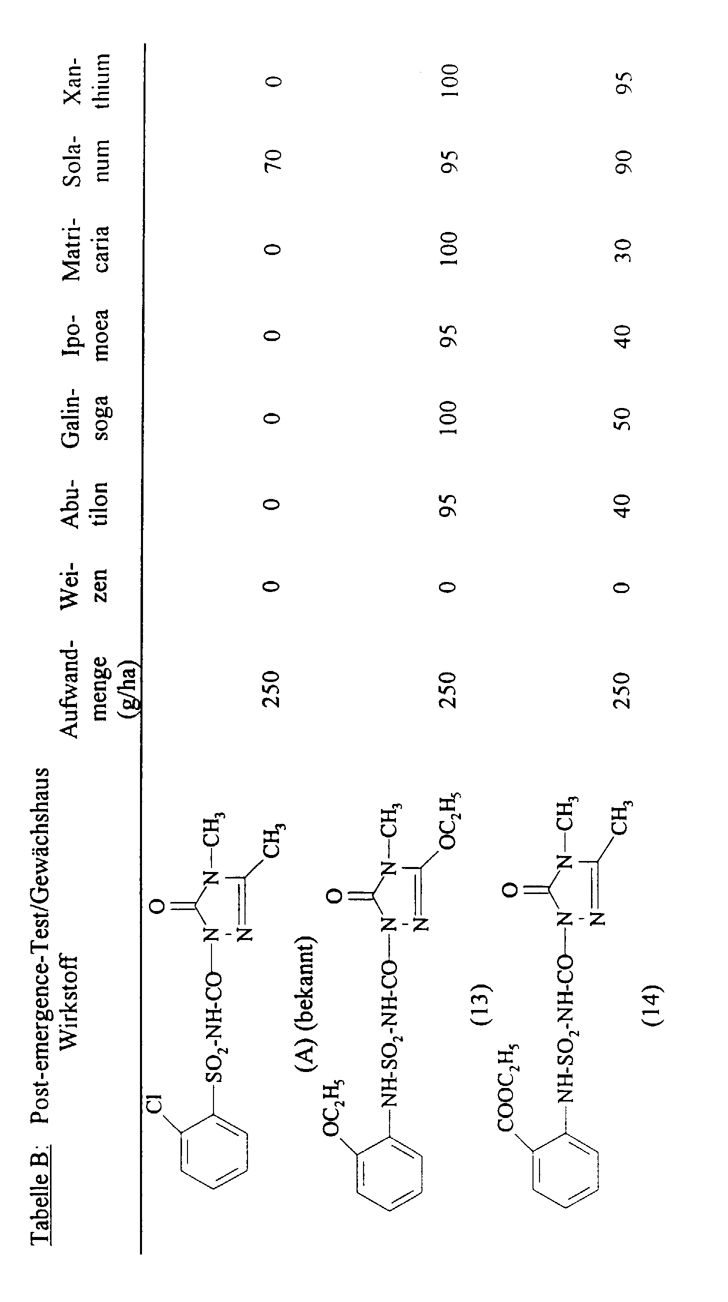

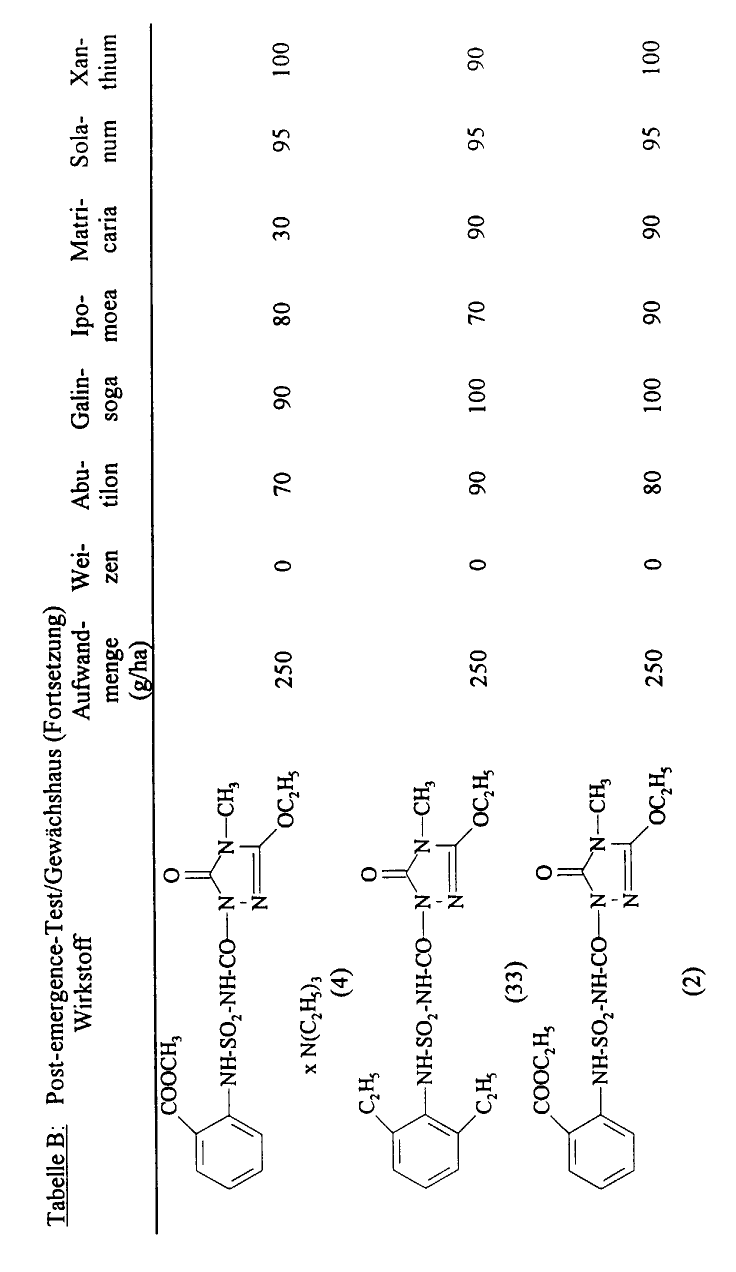

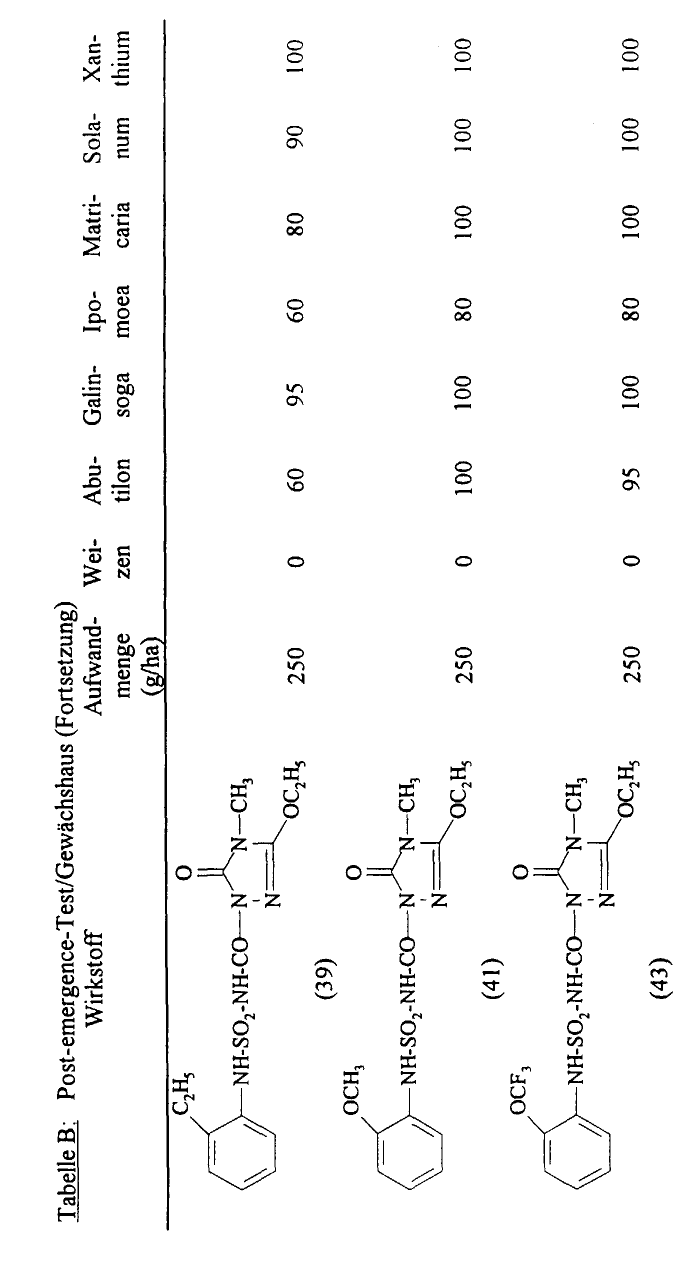

## Patentansprüche

1. Sulfonylaminocarbonyltriazolinone der allgemeinen Formel (I), 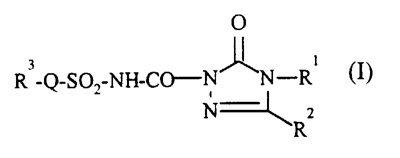 in welcher
Q für Sauerstoff, Schwefel oder eine der nachstehenden Gruppierungen steht 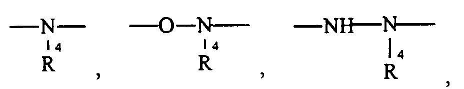
R¹ für Wasserstoff, Hydroxy, Amino, Alkylidenamino oder für einen jeweils gegebenenfalls substituierten Rest aus der Reihe Alkyl, Alkenyl, Alkinyl, Cycloalkyl, Aralkyl, Aryl, Alkoxy, Alkenyloxy, Alkylamino, Cycloalkylamino, Dialkylamino, Alkanoylamino, Alkoxycarbonylamino steht,
R² für Wasserstoff, Hydroxy, Mercapto, Amino, Halogen oder für einen jeweils gegebenenfalls substituierten Rest aus der Reihe Alkyl, Cycloalkyl, Alkenyl, Cycloalkenyl, Aralkyl, Aryl, Alkoxy, Alkenyloxy, Alkinyloxy, Cycloalkyloxy, Aryloxy, Aralkyloxy, Alkylthio, Alkylsulfinyl, Alkylsulfonyl, Alkenylthio, Alkinylthio, Cycloalkylthio, Arylthio, Aralkylthio, Alkylamino, Alkenylamino, Arylamino, Aralkylamino, Dialkylamino, Aziridino, Pyrrolidino, Piperidino oder Morpholino steht,
R³ für einen jeweils gegebenenfalls substituierten Rest aus der Reihe Alkyl, Alkenyl, Alkinyl, Cycloalkyl, Cycloalkylalkyl, Alkylsulfonyl, Alkylaminosulfonyl, Dialkylaminosulfonyl, Aryl, Aralkyl steht,
R⁴ für Wasserstoff, Hydroxy, Amino, Cyano, Alkoxycarbonyl, für jeweils gegebenenfalls substituiertes Alkyl, Alkenyl, Alkinyl, Alkoxy, Alkenyloxy, Aralkoxy, Aryloxy oder Dialkoxy(thio)phosphoryl steht oder für die nachstehende Gruppierung Q¹-R⁵ steht, worin
Q¹ für -CO- oder -SO₂- steht und
R⁵ für jeweils gegebenenfalls substituiertes Alkyl, Cycloalkyl oder Aryl steht,
sowie Salze von Verbindungen der Formel (I).

2. Verbindungen der Formel (I) gemäß Anspruch 1, dadurch gekennzeichnet, daß darin
Q für Sauerstoff, Schwefel oder eine der nachstehenden Gruppierungen steht 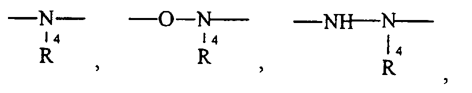
R¹ für Wasserstoff, Hydroxy, Amino, für C₂-C₁₀-Alkylidenamino, für gegebenenfalls durch Fluor, Chlor, Brom, Cyano, C₁-C₄-Alkoxy, C₁-C₄-Alkylcarbonyl oder C₁-C₄-Alkoxy-carbonyl substituiertes C₁-C₆-Alkyl, für jeweils gegebenenfalls durch Fluor, Chlor und/oder Brom substituiertes C₃-C₆-Alkenyl oder C₃-C₆-Alkinyl, für gegebenenfalls durch Fluor, Chlor, Brom und/oder C₁-C₄-Alkyl substituiertes C₃-C₆-Cycloalkyl, für gegebenenfalls durch Fluor, Chlor, Brom, Cyano, Nitro, C₁-C₄-Alkyl, Trifluormethyl, C₁-C₄-Alkoxy und/oder C₁-C₄-Alkoxy-carbonyl substituiertes Phenyl-C₁-C₃-alkyl, für gegebenenfalls durch Fluor, Chlor, Brom, Cyano, Nitro, C₁-C₄-Alkyl, Trifluormethyl, C₁-C₄-Alkoxy, Fluor- und/oder Chlor-substituiertes C₁-C₃-Alkoxy, C₁-C₄-Alkylthio, Fluor- und/oder Chlor-substituiertes C₁-C₃-Alkylthio, C₁-C₄-Alkylsulfinyl, C₁-C₄-Alkylsulfonyl und/oder C₁-C₄-Alkoxy-carbonyl substituiertes Phenyl, für gegebenenfalls durch Fluor, Chlor, Cyano, Phenyl, C₁-C₄-Alkoxy oder C₁-C₄-Alkoxy-carbonyl substituiertes C₁-C₆-Alkoxy, für C₃-C₆-Alkenyloxy, für gegebenenfalls durch Fluor, Cyano, C₁-C₄-Alkoxy oder C₁-C₄-Alkoxy-carbonyl substituiertes C₁-C₄-Alkylamino, C₃-C₆-Cycloalkylamino oder Di-(C₁-C₄-alkyl)-amino, für C₁-C₆-Alkanoylamino oder für C₁-C₆-Alkoxycarbonylamino steht,
R² für Wasserstoff, Hydroxy, Mercapto, Amino, Halogen, für gegebenenfalls durch Fluor, Chlor, Brom Cyano, C₃-C₆-Cycloalkyl, C₁-C₄-Alkoxy, C₁-C₄-Alkylthio oder C₁-C₄-Alkoxy-carbonyl substituiertes C₁-C₆-Alkyl oder C₂-C₆-Alkenyl, für gegebenenfalls durch Fluor, Chlor, Brom und/oder C₁-C₄-Alkyl substituiertes C₃-C₆-Cycloalkyl, für Cyclohexenyl, für gegebenenfalls durch Fluor, Chlor, Brom, Cyano, Nitro, C₁-C₄-Alkyl, Trifluormethyl, C₁-C₄-Alkoxy und/oder C₁-C₄-Alkoxy-carbonyl substituiertes Phenyl-C₁-C₃-alkyl, für gegebenenfalls durch Fluor, Chlor, Brom, Cyano, Nitro, C₁-C₄-Alkyl, Trifluormethyl, C₁-C₄-Alkoxy, Fluor- und/oder Chlor-substituiertes C₁-C₃-Alkoxy, C₁-C₄-Alkylthio, Fluor- und/oder Chlor-substituiertes C₁-C₃-Alkylthio, C₁-C₄-Alkyl-sulfinyl, C₁-C₄-Alkylsulfonyl und/oder C₁-C₄-Alkoxy-carbonyl substituiertes Phenyl, für gegebenenfalls durch Fluor, Chlor, Cyano, C₁-C₄-Alkoxy oder C₁-C₄-Alkoxy-carbonyl substituiertes C₁-C₆-Alkoxy, für C₃-C₆-Alkenyloxy, C₃-C₆-Alkinyloxy oder C₃-C₆-Cycloalkyloxy, für jeweils gegebenenfalls durch Fluor, Chlor, Brom, Cyano, Nitro, C₁-C₄-Alkyl, Trifluormethyl, C₁-C₄-Alkoxy, Difluormethoxy oder Trifluormethoxy substituiertes Phenoxy oder Benzyloxy, für jeweils gegebenenfalls durch Fluor, Chlor, Cyano, C₁-C₄-Alkoxy oder C₁-C₄-Alkoxy-carbonyl substituiertes C₁-C₆-Alkylthio, C₁-C₆-Alkylsulfinyl, C₁-C₆-Alkylsulfonyl, C₃-C₆-Alkenylthio, C₃-C₆-Alkinylthio oder C₃-C₆-Cycloalkylthio, für jeweils gegebenenfalls durch Fluor, Chlor, Brom, Cyano, Nitro, C₁-C₄-Alkyl, Trifluormethyl, C₁-C₄-Alkoxy, Difluormethoxy oder Trifluormethoxy substituiertes Phenylthio oder Benzylthio, für C₁-C₆-Alkylamino oder C₃-C₆-Alkenylamino, für jeweils gegebenenfalls durch Fluor, Chlor, Brom, Cyano, Nitro, C₁-C₄-Alkyl, Trifluormethyl, C₁-C₄-Alkoxy, Difluormethoxy oder Trifluormethoxy substituiertes Phenylamino oder Benzylamino, für Di-(C₁-C₄-alkyl)-amino, oder für jeweils gegebenenfalls durch C₁-C₄-Alkyl substituiertes Aziridino, Pyrrolidino oder Morpholino steht,
R³ für jeweils gegebenenfalls durch Halogen, Cyano, Carboxy, C₁-C₄-Alkoxy, C₁-C₄-alkylthio, C₁-C₄-Alkylsulfinyl, C₁-C₄₋Alkylsulfonyl oder C₁-C₄-Alkoxy-carbonyl substituiertes C₁-C₆-Alkyl, C₃-C₆-Alkenyl oder C₃-C₆-Alkinyl, für jeweils gegebenenfalls durch Halogen, Cyano, Carboxy, C₁-C₄-Alkyl, C₁-C₄-Halogenalkyl oder C₁-C₄-Alkoxycarbonyl substituiertes C₃-C₆-Cycloalkyl oder C₃-C₆-Cycloalkyl-C₁-C₄-alkyl, für jeweils gegebenenfalls durch Halogen substituiertes C₁-C₆-Alkylsulfonyl, C₁-C₆-Alkylaminosulfonyl oder Di-(C₁-C₄-alkyl)aminosulfonyl, für jeweils gegebenenfalls durch Halogen, Cyano, Carboxy, Nitro, durch C₁-C₄-Alkyl, C₁-C₄-Alkoxy, C₁-C₄-Alkylthio, C₁-C₄-Alkylsulfinyl oder C₁-C₄-Alkylsulfonyl (welche jeweils gegebenenfalls durch Halogen, Cyano, C₁-C₄-Alkoxy oder C₁-C₄-Alkoxy-carbonyl substituiert sind), durch C₁-C₄-Alkylsulfonyloxy, C₁-C₄-Alkylaminosulfonyloxy, Di-(C₁-C₄-alkyl)aminosulfonyloxy, C₁-C₄-Halogenalkylsulfonyloxy, Di-(C₁-C₄-alkyl)aminocarbonyl oder C₁-C₄-Alkylaminocarbonyloxy, durch C₃-C₆-Cycloalkyl oder C₃-C₆-Cycloalkyl-C₁-C₄-alkyl (welche jeweils gegebenenfalls durch Halogen oder C₁-C₄-Alkyl substituiert sind), durch Phenyl, Phenoxy, Phenylthio, Phenylsulfinyl, Phenylsulfonyl, Phenylsulfonyloxy, Phenylamino, Phenylcarbonyl oder Phenyl-C₁-C₄-alkyl (welche jeweils gegebenenfalls durch Halogen, Cyano, Nitro, C₁-C₄-Alkyl, C₁-C₄-Halogenalkyl, C₁-C₄-Alkoxy, C₁-C₄-Halogenalkoxy, C₁-C₄-Alkylthio, C₁-C₄-Halogenalkylthio, C₁-C₄-Alkoxy-carbonyl oder C₁-C₄-Alkoxy-amino substituiert sind), durch C₁-C₄-Alkyl-carbonyl, C₃-C₆-Cycloalkylcarbonyl oder C₁-C₄-Alkoxy-carbonyl (welche jeweils gegebenenfalls durch Halogen, C₃-C₆-Cycloalkyl oder C₁-C₄-Alkoxy substituiert sind) substituiertes Phenyl, Naphthyl, Tetralinyl, Phenyl-C₁-C₄-alkyl, Naphthyl-C₁-C₄-alkyl oder Tetralinyl-C₁-C₄-alkyl steht,
R⁴ für Wasserstoff, Hydroxy, Amino, Cyano, für jeweils gegebenenfalls durch Halogen, Cyano, Carboxy, C₁-C₄-Alkoxy, C₁-C₄-Alkylthio, C₁-C₄-Alkylsulfinyl, C₁-C₄-Alkylsulfonyl, C₁-C₄-Alkylamino, Di-(C₁-C₄-alkyl)-amino oder C₁-C₄-Alkoxy-carbonyl substituiertes C₁-C₆-Alkyl, C₃-C₆-Alkenyl oder C₃-C₆-Alkinyl, für C₁-C₄-Alkoxy-carbonyl, für C₁-C₆-Alkoxy, C₃-C₆-Alkenyloxy, Benzyloxy, Phenoxy oder Di-(C₁-C₄-alkoxy)-(thio)phosphoryl steht oder für die nachstehende Gruppierung Q¹-R⁵ steht, worin
Q¹ für -CO- oder -SO₂- steht und
R⁵ für gegebenenfalls durch Halogen oder C₁-C₄-Alkoxy substituiertes C₁-C₆-Alkyl, für gegebenenfalls durch Halogen oder C₁-C₄-Alkyl substituiertes C₃-C₆-Cycloalkyl oder für gegebenenfalls durch Halogen, C₁-C₄-Alkyl, C₁-C₄-Halogenalkyl oder C₁-C₄-Alkoxy substituiertes Phenyl steht.

3. Verbindungen der Formel (I) gemäß Anspruch 1, dadurch gekennzeichnet, daß darin
Q für Sauerstoff oder die Gruppierung 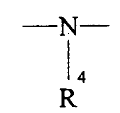 steht,
R¹ für Wasserstoff, Hydroxy, Amino, für C₃-C₈-Alkylidenamino, für Methyl, Ethyl, n- oder i-Propyl, n-, i-, s- oder t-Butyl, Dichlormethyl, Fluorethyl, Chlorethyl, Difluorethyl, Dichlorethyl, Trifluorethyl, Trichlorethyl, Chlordifluorethyl, Tetrafluorethyl, Cyanomethyl, Cyanoethyl, Methoxymethyl, Ethoxymethyl, Methoxyethyl, Ethoxyethyl, für Allyl, Chlorallyl, Dichlorallyl, Propargyl, für Cyclopropyl, Benzyl oder Phenyl, für Methoxy, Ethoxy, n- oder i-Propoxy, für Allyloxy, n- oder i- oder s-Butoxy, für Methylamino, Ethylamino, n- oder i-Propylamino, für Cyclopropylamino, Dimethylamino, Diethylamino, Acetylamino, Methoxycarbonylamino oder Ethoxycarbonylamino steht,
R² für Wasserstoff, Hydroxy, Mercapto, Amino, Fluor, Chlor, Brom, für Methyl, Ethyl, n- oder i-Propyl, n-, i-, s- oder t-Butyl, Difluormethyl, Trifluormethyl, Dichlormethyl, Trichlormethyl, Cyanomethyl, Cyanoethyl, Cyclopropylmethyl, Methoxymethyl, Ethoxymethyl, Methoxyethyl, Ethoxyethyl, Methylthiomethyl, Ethylthiomethyl, Methylthioethyl oder Ethylthioethyl, für Cyclopropyl, Difluorcyclopropyl oder Dichlorcyclopropyl, für Phenyl oder Benzyl, für Methoxy, Ethoxy, n- oder i-Propoxy, für Methoxymethoxy, Ethoxymethoxy, Methoxyethoxy oder Ethoxyethoxy, für Phenoxy oder Benzyloxy, für Methylthio, Ethylthio, n- oder i-Propylthio, Allylthio, Propargylthio, Cyclopropylmethylthio, Methylsulfinyl, Ethylsulfinyl, Methylsulfonyl, Ethylsulfonyl, Phenylthio oder Benzylthio, für Methylamino, Ethylamino, n- oder i-Propylamino, Phenylamino oder Benzylamino, für Dimethylamino, Diethylamino, Dipropylamino, Methylethylamino, Methylpropylamino, für Aziridino oder für jeweils gegebenenfalls durch Methyl oder Ethyl substituiertes Pyrrolidino, Piperidino oder Morpholino steht,
R³ für jeweils gegebenenfalls durch Fluor, Chlor, Cyano, Methoxy, Ethoxy, Methoxycarbonyl oder Ethoxycarbonyl substituiertes Methyl, Ethyl, n-oder i-Propyl, n-, i-, s- oder t-Butyl, für jeweils gegebenenfalls durch Fluor, Chlor, Cyano, Methyl, Ethyl, Trifluormethyl, Methoxycarbonyl oder Ethoxycarbonyl substituiertes Cyclopropyl, Cyclobutyl, Cyclopentyl, Cyclohexyl, Cyclopropylmethyl, Cyclobutylmethyl, Cyclopentylmethyl, Cyclohexylmethyl, Cyclopropylethyl, Cyclobutylethyl, Cyclopentylethyl oder Cyclohexylethyl, für gegebenenfalls durch Fluor oder Chlor substituiertes Methylsulfonyl, Ethylsulfonyl, Propylsulfonyl oder Butylsulfonyl, für gegebenenfalls durch Fluor, Chlor, Brom, Cyano, Carboxy, Nitro, durch Methyl, Ethyl, Methoxy, Ethoxy, Methylthio, Ethylthio, Methylsulfinyl, Ethylsulfinyl, Methylsulfonyl oder Ethylsulfonyl (welche jeweils gegebenenfalls durch Fluor, Chlor, Cyano, Methoxy, Ethoxy, Methoxycarbonyl oder Ethoxycarbonyl substituiert sind), durch Methylsulfonyloxy, Ethylsulfonyloxy, Methylaminosulfonyloxy, Ethylaminosulfonyloxy, Dimethylaminosulfonyloxy, Diethylaminosulfonyloxy, Trifluormethylsulfonyloxy, Dimethylaminocarbonyl oder Diethylaminocarbonyl, durch Cyclohexyl oder Cyclohexylmethyl (welche jeweils gegebenenfalls durch Chlor, Methyl oder Ethyl substituiert sind) durch Phenyl, Phenoxy, Phenylthio, Phenylsulfonyl, Phenylsulfonyloxy, Phenylamino, Phenylcarbonyl, Phenylmethyl oder Phenylethyl (welche jeweils gegebenenfalls durch Fluor, Chlor, Cyano, Nitro, Methyl, Ethyl, Trifluormethyl, Methoxy, Ethoxy, Difluormethoxy, Trifluormethoxy, Methylthio, Ethylthio, Trifluormethylthio, Methoxycarbonyl oder Ethoxycarbonyl substituiert sind), durch Acetyl, Cyclopropylcarbonyl, Methoxycarbonyl oder Ethoxycarbonyl (welche gegebenenfalls durch Fluor, Chlor, Cyclopentyl, Cyclohexyl, Methoxy oder Ethoxy substituiert sind) substituiertes Phenyl, für jeweils gegebenenfalls durch Fluor, Chlor, Cyano, Nitro, Methyl, Ethyl, Methoxy oder Ethoxy substituiertes Naphthyl oder Tetralinyl, oder für jeweils gegebenenfalls durch Fluor, Chlor, Methyl, Ethyl, Methoxy oder Ethoxy substituiertes Naphthylmethyl, Naphthylethyl, Tetralinylmethyl oder Tetralinylethyl steht,
R⁴ für Wasserstoff, Cyano, Cyanomethyl, Cyanoethyl, Difluormethyl, Methoxymethyl, Ethoxymethyl, Methoxyethyl, Ethoxyethyl, Methoxycarbonylmethyl, Ethoxycarbonylmethyl, Methoxycarbonylethyl, Ethoxycarbonylethyl, Allyl, Propargyl, Methoxycarbonyl, Ethoxycarbonyl, Methoxy, Ethoxy, Allyloxy oder Benzyloxy steht oder für die Gruppierung Q¹-R⁵ steht, worin
Q¹ für -CO- oder -SO₂- steht und
R⁵ für jeweils gegebenenfalls durch Fluor, Chlor, Methoxy oder Ethoxy substituiertes Methyl, Ethyl oder Propyl, für jeweils gegebenenfalls durch Fluor, Chlor, Methyl oder Ethyl substituiertes Cyclopropyl, Cyclobutyl, Cyclopentyl oder Cyclohexyl, oder für gegebenenfalls durch Fluor, Chlor, Brom, Methyl, Ethyl, Trifluormethyl, Methoxy oder Ethoxy substituiertes Phenyl steht.

4. Die Natrium-, Kalium-, Magnesium-, Calcium-, Ammonium-, C₁-C₄-Alkylammonium-, Di-(C₁-C₄-alkyl)-ammonium-, Tri-(C₁-C₄-alkyl)-ammonium-, C₅- oder C₆-Cycloalkyl-ammonium- und Di-(C₁-C₂-alkyl)-benzyl-ammonium-Salze der Verbindungen der Formel (I) gemäß den Ansprüchen 1, 2 und 3.

5. Verfahren zur Herstellung von Sulfonylaminocarbonyltriazolinonen der Formel (I) gemäß Anspruch 1, dadurch gekennzeichnet, daß man
(a) Chlorsulfonylaminocarbonyltriazolinone der allgemeinen Formel (II) 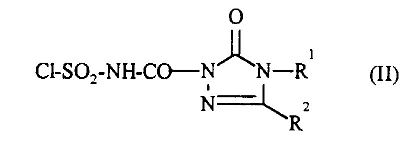 in welcher
R¹ und R² die in Anspruch 1 angegebene Bedeutung haben,
mit nucleophilen Verbindungen der allgemeinen Formel (III)
R³-Q-H (III)
in welcher
Q und R³ die in Anspruch 1 angegebene Bedeutung haben,
gegebenenfalls in Gegenwart eines Säureakzeptors und gegebenenfalls in Gegenwart eines Verdünnungsmittels umsetzt,
oder daß man
(b) Triazolinone der allgemeinen Formel (IV) 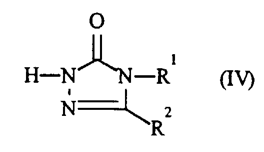 in welcher
R¹ und R² die in Anspruch 1 angegebene Bedeutung haben, mit Chlorsulfonylisocyanat, gegebenenfalls in Gegenwart eines Verdünnungsmittels umsetzt und die hierbei gebildeten Chlorsulfonylaminocarbonyltriazolinone der Formel (II) ohne Zwischenisolierung mit nucleophilen Verbindungen der allgemeinen Formel (III) gegebenenfalls in Gegenwart eines Säureakzeptors und gegebenenfalls in Gegenwart eines Verdünnungsmittels umsetzt,
und gegebenenfalls die nach Verfahren (a) oder (b) erhaltenen Verbindungen der Formel (I) nach üblichen Methoden in Salze überführt.

6. Herbizide Mittel, gekennzeichnet durch einen Gehalt an mindestens einer Verbindung der Formel (I) gemäß Anspruch 1.

7. Verwendung von Verbindungen der allgemeinen Formel (I) gemäß Anspruch 1 zur Bekämpfung von unerwünschtem Pflanzenwachstum.

8. Verfahren zur Bekämpfung von Unkräutern, dadurch gekennzeichnet, daß man Verbindungen der allgemeinen Formel (I) gemäß Anspruch 1 auf die Unkräuter oder ihren Lebensraum einwirken läßt.

9. Verfahren zur Herstellung von herbiziden Mitteln, dadurch gekennzeichnet, daß man Verbindungen der allgemeinen Formel (I) gemäß Anspruch 1 mit Streckmitteln und/oder oberflächenaktiven Mitteln vermischt.

10. Chlorsulfonylaminocarbonyltriazolinone der allgemeinen Formel (II), 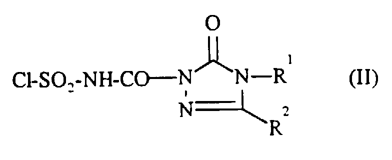 in welcher
R¹ für Wasserstoff, Hydroxy, Amino, Alkylidenamino oder für einen jeweils gegebenenfalls substituierten Rest aus der Reihe Alkyl, Alkenyl, Alkinyl, Cycloalkyl, Aralkyl, Aryl, Alkoxy, Alkenyloxy, Alkylamino, Cycloalkylamino, Dialkylamino, Alkanoylamino, Alkoxycarbonylamino steht und
R² für Wasserstoff, Hydroxy, Mercapto, Amino, Halogen oder für einen jeweils gegebenenfalls substituierten Rest aus der Reihe Alkyl, Cycloalkyl, Alkenyl, Cycloalkenyl, Aralkyl, Aryl, Alkoxy, Alkenyloxy, Alkinyloxy, Cycloalkyloxy, Aryloxy, Aralkyloxy, Alkylthio, Alkylsulfinyl Alkylsulfonyl, Alkenylthio, Alkinylthio, Cycloalkylthio, Arylthio, Aralkylthio, Alkylamino, Alkenylamino, Arylamino, Aralkylamino, Dialkylamino, Aziridino, Pyrrolidino, Piperidino oder Morpholino steht.

## Claims

1. Sulphonylaminocarbonyltriazolinones of the general formula (I) 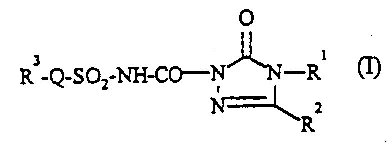 in which
Q represents oxygen, sulphur or one of the following groups 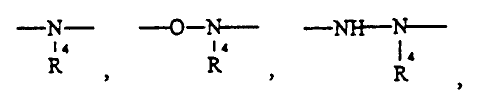
R¹ represents hydrogen, hydroxyl, amino, alkylideneamino, or represents a radical from the series consisting of alkyl, alkenyl, alkinyl, cycloalkyl, aralkyl, aryl, alkoxy, alkenyloxy, alkylamino, cycloalkylamino, dialkylamino, alkanoylamino and alkoxycarbonylamino, each of which is optionally substituted,
R² represents hydrogen, hydroxyl, mercapto, amino, halogen, or represents a radical from the series consisting of alkyl, cycloalkyl, alkenyl, cycloalkenyl, aralkyl, aryl, alkoxy, alkenyloxy, alkinyloxy, cycloalkyloxy, aryloxy, aralkyloxy, alkylthio, alkylsulphinyl, alkylsulphonyl, alkenylthio, alkinylthio, cycloalkylthio, arylthio, aralkylthio, alkylamino, alkenylamino, arylamino, aralkylamino, dialkylamino, aziridino, pyrrolidino, piperidino or morpholino, each of which is optionally substituted,
R³ represents a radical from the series consisting of alkyl, alkenyl, alkinyl, cycloalkyl, cycloalkylalkyl, alkylsulphonyl, alkylaminosulphonyl, dialkylaminosulphonyl, aryl and aralkyl, each of which is optionally substituted,
R⁴ represents hydrogen, hydroxyl, amino, cyano, alkoxycarbonyl, or represents alkyl, alkenyl, alkinyl, alkoxy, alkenyloxy, aralkoxy, aryloxy or dialkoxy(thio)phosphoryl, each of which is optionally substituted, or the following group Q¹-R⁵, in which
Q¹ represents -CO- or -SO₂- and
R⁵ represents alkyl, cycloalkyl or aryl, each of which is optionally substituted,
and salts of compounds of the formula (I).

2. Compounds of the formula (I) according to Claim 1, characterized in that, in this formula,
Q represents oxygen, sulphur or one of the following groups 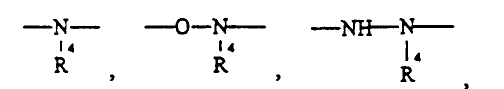
R¹ represents hydrogen, hydroxyl, amino, or represents C₂-C₁₀-alkylideneamino, or represents C₁-C₆-alkyl which is optionally substituted by fluorine, chlorine, bromine, cyano, C₁-C₄-alkoxy, C₁-C₄-alkylcarbonyl or C₁-C₄-alkoxy-carbonyl, or represents C₃-C₆-alkenyl or C₃-C₆-alkinyl, each of which is optionally substituted by fluorine, chlorine and/or bromine, or represents C₃-C₆-cycloalkyl which is optionally substituted by fluorine, chlorine, bromine and/or C₁-C₄-alkyl, or represents phenyl-C₁-C₃-alkyl which is optionally substituted by fluorine, chlorine, bromine, cyano, nitro, C₁-C₄-alkyl, trifluoromethyl, C₁-C₄-alkoxy and/or C₁-C₄-alkoxy-carbonyl, or represents phenyl which is optionally substituted by fluorine, chlorine, bromine, cyano, nitro, C₁-C₄-alkyl, trifluoromethyl, C₁-C₄-alkoxy, fluorine- and/or chlorine-substituted C₁-C₃-alkoxy, C₁-C₄-alkylthio, fluorine- and/or chlorine-substituted C₁-C₃-alkylthio, C₁-C₄-alkylsulphinyl, C₁-C₄-alkylsulphonyl and/or C₁-C₄-alkoxycarbonyl, or represents C₁-C₆-alkoxy which is optionally substituted by fluorine, chlorine, cyano, phenyl, C₁-C₄-alkoxy or C₁-C₄-alkoxycarbonyl, or represents C₃-C₆-alkenyloxy, or represents C₁-C₄-alkylamino, C₃-C₆-cycloalkylamino or di-(C₁-C₄-alkyl)-amino, each of which is optionally substituted by fluorine, cyano, C₁-C₄-alkoxy or C₁-C₄-alkoxy-carbonyl, or represents C₁-C₆-alkanoylamino, or represents C₁-C₆-alkoxycarbonylamino,
R² represents hydrogen, hydroxyl, mercapto, amino, halogen, or represents C₁-C₆-alkyl or C₂-C₆-alkenyl, optionally substituted by fluorine, chlorine, bromine cyano, C₃-C₆-cycloalkyl, C₁-C₄-alkoxy, C₁-C₄-alkylthio or C₁-C₄-alkoxy-carbonyl, or represents C₃-C₆-cycloalkyl which is optionally substituted by fluorine, chlorine, bromine and/or C₁-C₄-alkyl, or represents cyclohexenyl, or represents phenyl-C₁-C₃-alkyl which is optionally substituted by fluorine, chlorine, bromine, cyano, nitro, C₁-C₄-alkyl, trifluoromethyl, C₁-C₄-alkoxy and/or C₁-C₄-alkoxy-carbonyl, or represents phenyl which is optionally substituted by fluorine, chlorine, bromine, cyano, nitro, C₁-C₄-alkyl, trifluoromethyl, C₁-C₄-alkoxy, fluorine- and/or chlorine-substituted C₁-C₃-alkoxy, C₁-C₄-alkylthio, fluorine- and/or chlorine-substituted C₁-C₃-alkylthio, C₁-C₄-alkyl-sulphinyl, C₁-C₄-alkylsulphonyl and/or C₁-C₄-alkoxycarbonyl, or represents C₁-C₆-alkoxy which is optionally substituted by fluorine, chlorine, cyano, C₁-C₄-alkoxy or C₁-C₄-alkoxycarbonyl, or represents C₃-C₆-alkenyloxy, C₃-C₆-alkinyloxy or C₃-C₆-cycloalkyloxy, or represents phenoxy or benzyloxy, each of which is optionally substituted by fluorine, chlorine, bromine, cyano, nitro, C₁-C₄-alkyl, trifluoromethyl, C₁-C₄-alkoxy, difluoromethoxy or trifluoromethoxy, or represents C₁-C₆-alkylthio, C₁-C₆-alkylsulphinyl, C₁-C₆-alkylsulphonyl, C₃-C₆-alkenylthio, C₃-C₆-alkinylthio or C₃-C₆-cycloalkylthio, each of which is optionally substituted by fluorine, chlorine, cyano, C₁-C₄-alkoxy or C₁-C₄-alkoxy-carbonyl, or represents phenylthio or benzylthio, each of which is optionally substituted by fluorine, chlorine, bromine, cyano, nitro, C₁-C₄-alkyl, trifluoromethyl, C₁-C₄-alkoxy, difluoromethoxy or trifluoromethoxy, or represents C₁-C₆-alkylamino or C₃-C₆-alkenylamino, or represents phenylamino or benzylamino, each of which is optionally substituted by fluorine, chlorine, bromine, cyano, nitro, C₁-C₄-alkyl, trifluoromethyl, C₁-C₄-alkoxy, difluoromethoxy or trifluoromethoxy, or represents di-(C₁-C₄-alkyl)-amino, or represents aziridino, pyrrolidino or morpholino, each of which is optionally substituted by C₁-C₄-alkyl,
R³ represents C₁-C₆-alkyl, C₃-C₆-alkenyl or C₃-C₆-alkinyl, each of which is optionally substituted by halogen, cyano, carboxyl, C₁-C₄-alkoxy, C₁-C₄-alkylthio, C₁-C₄-alkylsulphinyl, C₁-C₄-alkylsulphonyl or C₁-C₄-alkoxy-carbonyl, or represents C₃-C₆-cycloalkyl or C₃-C₆-cycloalkyl-C₁-C₄-alkyl, each of which is optionally substituted by halogen, cyano, carboxyl, C₁-C₄-alkyl, C₁-C₄-halogenoalkyl or C₁-C₄-alkoxy-carbonyl, or represents C₁-C₆-alkylsulphonyl, C₁-C₆-alkylaminosulphonyl or di-(C₁-C₄-alkyl)-aminosulphonyl, each of which is optionally substituted by halogen, or represents phenyl, naphthyl, tetralinyl, phenyl-C₁-C₄-alkyl, naphthyl-C₁-C₄-alkyl or tetralinyl-C₁-C₄-alkyl, each of which is optionally substituted by halogen, cyano, carboxyl, nitro, by C₁-C₄-alkyl, C₁-C₄-alkoxy, C₁-C₄-alkylthio, C₁-C₄-alkylsulphinyl or C₁-C₄-alkylsulphonyl (in each case optionally substituted by halogen, cyano, C₁-C₄-alkoxy or C₁-C₄-alkoxy-carbonyl), by C₁-C₄-alkylsulphonyloxy, C₁-C₄-alkylaminosulphonyloxy, di-(C₁-C₄-alkyl)aminosulphonyloxy, C₁-C₄-halogenoalkylsulphonyloxy, di-(C₁-C₄-alkyl)aminocarbonyl or C₁-C₄-alkylaminocarbonyloxy, by C₃-C₆-cycloalkyl or C₃-C₆-cycloalkyl-C₁-C₄-alkyl (in each case optionally substituted by halogen or C₁-C₄-alkyl), by phenyl, phenoxy, phenylthio, phenylsulphinyl, phenylsulphonyl, phenylsulphonyloxy, phenylamino, phenylcarbonyl or phenyl-C₁-C₄-alkyl (in each case optionally substituted by halogen, cyano, nitro, C₁-C₄-alkyl, C₁-C₄-halogenoalkyl, C₁-C₄-alkoxy, C₁-C₄-halogenoalkoxy, C₁-C₄-alkylthio, C₁-C₄-halogenoalkylthio, C₁-C₄-alkoxycarbonyl or C₁-C₄-alkoxy-amino), by C₁-C₄-alkyl-carbonyl, C₃-C₆-cycloalkylcarbonyl or C₁-C₄-alkoxy-carbonyl (in each case optionally substituted by halogen, C₃-C₆-cycloalkyl or C₁-C₄-alkoxy),
R⁴ represents hydrogen, hydroxyl, amino, cyano, or represents C₁-C₆-alkyl, C₃-C₆-alkenyl or C₃-C₆-alkinyl, each of which is optionally substituted by halogen, cyano, carboxyl, C₁-C₄-alkoxy, C₁-C₄-alkylthio, C₁-C₄-alkylsulphinyl, C₁-C₄-alkylsulphonyl, C₁-C₄-alkylamino, di-(C₁-C₄-alkyl)-amino or C₁-C₄-alkoxy-carbonyl, or represents C₁-C₄-alkoxy-carbonyl, or represents C₁-C₆-alkoxy, C₃-C₆-alkenyloxy, benzyloxy, phenoxy or di-(C₁-C₄-alkoxy)-(thio)-phosphoryl, or represents the following group Q¹-R⁵ in which
Q¹ represents -CO- or -SO₂- and
R⁵ represents C₁-C₆-alkyl which is optionally substituted by halogen or C₁-C₄-alkoxy, or represents C₃-C₆-cycloalkyl which is optionally substituted by halogen or C₁-C₄-alkyl, or represents phenyl which is optionally substituted by halogen, C₁-C₄-alkyl, C₁-C₄-halogenoalkyl or C₁-C₄-alkoxy.

3. Compounds of the formula (I) according to Claim 1, characterized in that, in this formula,
Q represents oxygen or the group 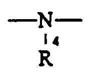
R¹ represents hydrogen, hydroxyl, amino, or represents C₃-C₈-alkylideneamino, or represents methyl, ethyl, n- or i-propyl, n-, i-, s- or t-butyl, dichloromethyl, fluoroethyl, chloroethyl, difluoroethyl, dichloroethyl, trifluoroethyl, trichloroethyl, chlorodifluoroethyl, tetrafluoroethyl, cyanomethyl, cyanoethyl, methoxymethyl, ethoxymethyl, methoxyethyl, ethoxyethyl, or represents allyl, chloroallyl, dichloroallyl, propargyl, or represents chloropropyl, benzyl or phenyl, or represents methoxy, ethoxy, n- or i-propoxy, or represents allyloxy, n- or i- or s-butoxy, or represents methylamino, ethylamino, n- or i-propylamino, or represents cyclopropylamino, dimethylamino, diethylamino, acetylamino, methoxycarbonylamino or ethoxycarbonylamino,
R² represents hydrogen, hydroxyl, mercapto, amino, fluorine, chlorine, bromine, or represents methyl, ethyl, n- or i-propyl, n-, i-, s- or t-butyl, difluoromethyl, trifluoromethyl, dichloromethyl, trichloromethyl, cyanomethyl, cyanoethyl, cyclopropylmethyl, methoxymethyl, ethoxymethyl, methoxyethyl, ethoxyethyl, methylthiomethyl, ethylthiomethyl, methylthioethyl or ethylthioethyl, or represents cyclopropyl, difluorocyclopropyl or dichlorocyclopropyl, or represents phenyl or benzyl, or represents methoxy, ethoxy, n- or i-propoxy, or represents methoxymethoxy, ethoxymethoxy, methoxyethoxy or ethoxyethoxy, or represents phenoxy or benzyloxy, or represents methylthio, ethylthio, n- or i-propylthio, allylthio, propargylthio, cyclopropylmethylthio, methylsulphinyl, ethylsulphinyl, methylsulphonyl, ethylsulphonyl, phenylthio or benzylthio, or represents methylamino, ethylamino, nor i-propylamino, phenylamino or benzylamino, or represents dimethylamino, diethylamino, dipropylamino, methylethylamino, methylpropylamino, or represents aziridino, or represents pyrrolidino, piperidino or morpholino, each of which is optionally substituted by methyl or ethyl,
R³ represents methyl, ethyl, n- or i-propyl or n-, i-, s- or t- butyl, each of which is optionally substituted by fluorine, chlorine, cyano, methoxy, ethoxy, methoxycarbonyl or ethoxycarbonyl, or represents cyclopropyl, cyclobutyl, cyclopentyl, cyclohexyl, cyclopropylmethyl, cyclobutylmethyl, cyclopentylmethyl, cyclohexylmethyl, cyclopropylethyl, cyclobutylethyl, cyclopentylethyl or cyclohexylethyl, each of which is optionally substituted by fluorine, chlorine, cyano, methyl, ethyl, trifluoromethyl, methoxycarbonyl or ethoxycarbonyl, or represents methylsulphonyl, ethylsulphonyl, propylsulphonyl or butylsulphonyl, optionally substituted by fluorine or chlorine, or represents phenyl which is optionally substituted by fluorine, chlorine, bromine, cyano, carboxyl, nitro, by methyl, ethyl, methoxy, ethoxy, methylthio, ethylthio, methylsulphinyl, ethylsulphinyl, methylsulphonyl or ethylsulphonyl (in each case optionally substituted by fluorine, chlorine, cyano, methoxy, ethoxy, methoxycarbonyl or ethoxycarbonyl), by methylsulphonyloxy, ethylsulphonyloxy, methylaminosulphonyloxy, ethylaminosulphonyloxy, dimethylaminosulphonyloxy, diethylaminosulphonyloxy, trifluoromethylsulphonyloxy, dimethylaminocarbonyl or diethylaminocarbonyl, by cyclohexyl or cyclohexylmethyl (in each case optionally substituted by chlorine, methyl or ethyl), by phenyl, phenoxy, phenylthio, phenylsulphonyl, phenylsulphonyloxy, phenylamino, phenylcarbonyl, phenylmethyl or phenylethyl (in each case optionally substituted by fluorine, chlorine, cyano, nitro, methyl, ethyl, trifluoromethyl, methoxy, ethoxy, difluoromethoxy, trifluoromethoxy, methylthio, ethylthio, trifluoromethylthio, methoxycarbonyl or ethoxycarbonyl), by acetyl, cyclopropylcarbonyl, methoxycarbonyl or ethoxycarbonyl (optionally substituted by fluorine, chlorine, cyclopentyl, cyclohexyl, methoxy or ethoxy), or represents naphthyl or tetralinyl, each of which is optionally substituted by fluorine, chlorine, cyano, nitro, methyl, ethyl, methoxy or ethoxy, or represents naphthylmethyl, naphthylethyl, tetralinylmethyl or tetralinylethyl, each of which is optionally substituted by fluorine, chlorine, methyl, ethyl, methoxy or ethoxy,
R⁴ represents hydrogen, cyano, cyanomethyl, cyanoethyl, difluoromethyl, methoxymethyl, ethoxymethyl, methoxyethyl, ethoxyethyl, methoxycarbonylmethyl, ethoxycarbonylmethyl, methoxycarbonylethyl, ethoxycarbonylethyl, allyl, propargyl, methoxycarbonyl, ethoxycarbonyl, methoxy, ethoxy, allyloxy or benzyloxy, or represents the group Q¹-R⁵ in which
Q¹ represents -CO- or -SO₂- and
R⁵ represents methyl, ethyl or propyl, each of which is optionally substituted by fluorine, chlorine, methoxy or ethoxy, or represents cyclopropyl, cyclobutyl, cyclopentyl or cyclohexyl, each of which is optionally substituted by fluorine, chlorine, methyl or ethyl, or represents phenyl which is optionally substituted by fluorine, chlorine, bromine, methyl, ethyl, trifluoromethyl, methoxy or ethoxy.

4. The sodium, potassium, magnesium, calcium, ammonium, C₁-C₄-alkylammonium, di-(C₁-C₄-alkyl)-ammonium, tri-(C₁-C₄-alkyl)-ammonium, C₅-or C₆-cycloalkyl-ammonium and di-(C₁-C₂-alkyl)-benzyl-ammonium salts of the compounds of the formula (I) according to Claims 1, 2 and 3.

5. Process for the preparation of sulphonylaminocarbonyltriazolinones of the formula (I) according to Claim 1, characterized in that
(a) chlorosulphonylaminocarbonyltriazolinones of the general formula (II) 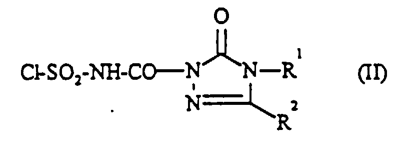 in which
R¹ and R² have the meaning given in Claim 1,
are reacted with nucleophilic compounds of the general formula (III)
R³-Q-H (III)
in which
Q and R³ have the meaning given in Claim 1, if appropriate in the presence of an acid acceptor and if appropriate in the presence of a diluent,
or in that
(b) triazolinones of the general formula (IV) 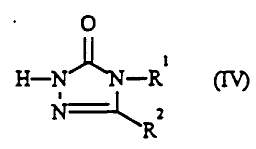 in which
R¹ and R² have the meaning given in Claim 1,
are reacted with chlorosulphonyl isocyanate, if appropriate in the presence of a diluent, and the resulting chlorosulphonylaminocarbonyltriazolinones of the formula (II) are reacted, without intermediate isolation, with nucleophilic compounds of the general formula (III) if appropriate in the presence of an acid acceptor and if appropriate in the presence of a diluent,
and, if appropriate, the compounds of the formula (I) obtained by process (a) or (b) are converted to salts by customary methods.

6. Herbicidal compositions, characterized in that they contain at least one compound of the formula (I) according to Claim 1.

7. Use of compounds of the general formula (I) according to Claim 1 for combating undesired plant growth.

8. Method of combating weeds, characterized in that compounds of the general formula (I) according to Claim 1 are allowed to act on the weeds or their environment.

9. Process for the preparation of herbicidal compositions, characterized in that compounds of the general formula (I) according to Claim 1 are mixed with extenders and/or surface-active agents.

10. Chlorosulphonylaminocarbonyltriazolinones of the general formula (II) 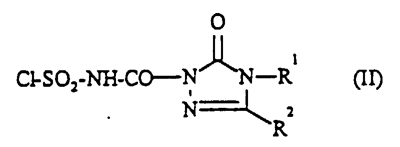 in which
R¹ represents hydrogen, hydroxyl, amino, alkylideneamino, or represents a radical from the series consisting of alkyl, alkenyl, alkinyl, cycloalkyl, aralkyl, aryl, alkoxy, alkenyloxy, alkylamino, cycloalkylamino, dialkylamino, alkanoylamino and alkoxycarbonylamino, each of which is optionally substituted, and
R² represents hydrogen, hydroxyl, mercapto, amino, halogen, or represents a radical from the series consisting of alkyl, cycloalkyl, alkenyl, cycloalkenyl, aralkyl, aryl, alkoxy, alkenyloxy, alkinyloxy, cycloalkyloxy, aryloxy, aralkyloxy, alkylthio, alkylsulphinyl, alkylsulphonyl, alkenylthio, alkinylthio, cycloalkylthio, arylthio, aralkylthio, alkylamino, alkenylamino, arylamino, aralkylamino, dialkylamino, aziridino, pyrrolidino, piperidino or morpholino, each of which is optionally substituted.

## Revendications

1. Sulfonylaminocarbonyltriazolinones répondant à la formule générale (I) 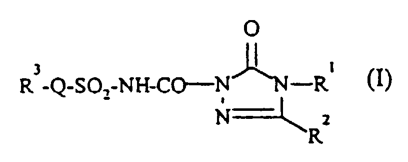 dans laquelle
Q représente un atome d'oxygène, un atome de soufre ou un des groupements ci-après: 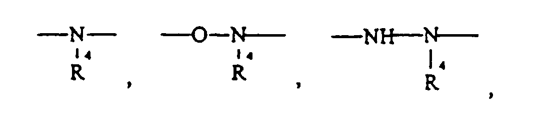
R¹ représente un atome d'hydrogène, un groupe hydroxyle, un groupe amino, un groupe alkylidène-amino ou encore un radical choisi parmi la série comprenant un radical alkyle, un radical alcényle, un radical alcynyle, un radical cycloalkyle, un radical aralkyle, un radical aryle, un radical alcoxy, un radical alcényloxy, un radical alkylamino, un radical cycloalkylamino, un radical dialkylamino, un radical alcanoylamino, un radical alcoxycarbonylamino, chacun de ces radicaux portant éventuellement un ou plusieurs substituants identiques ou différents,
R² représente un atome d'hydrogène, un groupe hydroxyle, un groupe mercapto, un groupe amino, un atome d'halogène ou encore un radical choisi parmi la série comprenant un radical alkyle, un radical cycloalkyle, un radical alcényle, un radical cycloalcényle, un radical aralkyle, un radical aryle, un radical alcoxy, un radical alcényloxy, un radical alcynyloxy, un radical cycloalkyloxy, un radical aryloxy, un radical aralkyloxy, un radical alkylthio, un radical alkylsulfinyle, un radical alkylsulfonyle, un radical alcénylthio, un radical alcynylthio, un radical cycloalkylthio, un radical arylthio, un radical aralkylthio, un radical alkylamino, un radical alcénylamino, un radical arylamino, un radical aralkylamino, un radical dialkylamino, un radical aziridino, un radical pyrrolidino, un radical pipéridino ou un radical morpholino, chacun de ces radicaux portant éventuellement un ou plusieurs substituants identiques ou différents,
R³ représente un radical choisi parmi la série comprenant un radical alkyle, un radical alcényle, un radical alcynyle, un radical cycloalkyle, un radical cycloalkylalkyle, un radical alkylsulfonyle, un radical alkylaminosulfonyle, un radical dialkylaminosulfonyle, un radical aryle, un radical aralkyle, chacun de ces radicaux portant éventuellement un ou plusieurs substituants identiques ou différents,
R⁴ représente un atome d'hydrogène, un groupe hydroxyle, un groupe amino, un groupe cyano, un groupe alcoxycarbonyle; un groupe alkyle, un groupe alcényle, un groupe alcynyle, un groupe alcoxy, un groupe alcényloxy, un groupe aralcoxy, un groupe aryloxy ou un groupe dialcoxy(thio)phosphoryle, chacun de ces groupes étant éventuellement substitué, ou encore le groupement Q¹-R⁵ ci-après dans lequel
Q¹ représente -CO- ou -SO₂-, et
R⁵ représente un groupe alkyle, un groupe cycloalkyle ou un groupe aryle, chacun de ces groupes étant éventuellement substitué,
ainsi que des sels des composés de formule (I).

2. Composés de formule (I) selon la revendication 1, caractérisés en ce que, dans ces composés,
Q représente un atome d'oxygène, un atome de soufre ou encore un des groupements ci-après: 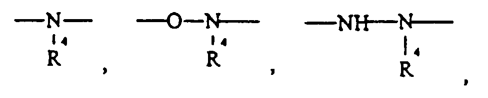
R¹ représente un atome d'hydrogène, un groupe hydroxyle, un groupe amino; un groupe alkylidène(en C₂-C₁₀)amino; un groupe alkyle en C₁-C₆ portant un ou plusieurs substituants identiques ou différents fluoro, chloro, bromo, cyano, alcoxy en C₁-C₄, alkyl(en C₁-C₄)carbonyle ou alcoxy(en C₁-C₄)carbonyle; un groupe alcényle en C₃-C₆ ou un groupe alcynyle en C₃-C₆, chacun de ces groupes portant éventuellement un ou plusieurs substituants identiques ou différents fluoro, chloro et/ou bromo; un groupe cycloalkyle en C₃-C₆ portant éventuellement un ou plusieurs substituants identiques ou différents fluoro, chloro, bromo et/ou alkyle en C₁-C₄; un groupe phénylalkyle en C₁-C₃ portant éventuellement un ou plusieurs substituants identiques ou différents fluoro, chloro, bromo, cyano, nitro, alkyle en C₁-C₄, trifluorométhyle, alcoxy en C₁-C₄ et/ou alcoxy(en C₁-C₄)carbonyle; un groupe phényle portant éventuellement un ou plusieurs substituants identiques ou différents fluoro, chloro, bromo, cyano, nitro, alkyle en C₁-C₄, trifluorométhyle, alcoxy en C₁-C₄, alcoxy en C₁-C₃ portant éventuellement un ou plusieurs substituants identiques ou différents fluoro et/ou chloro, alkyl(en C₁-C₄)thio, alkyl(en C₁-C₃)thio portant éventuellement un ou plusieurs substituants identiques ou différents fluoro et/ou chloro, alkyl(en C₁-C₄)sulfinyle, alkyl(en C₁-C₄)sulfonyle et/ou alcoxy(en C₁-C₄)carbonyle; un groupe alcoxy en C₁-C₆ portant éventuellement un ou plusieurs substituants identiques ou différents fluoro, chloro, cyano, phényle, alcoxy en C₁-C₄ ou alcoxy(en C₁-C₄)carbonyle; un groupe alcényl(en C₃-C₆)oxy; un groupe alkyl(en C₁-C₄)amino, un groupe cycloalkyl(en C₃-C₆)amino ou di(alkyl en C₁-C₄)amino, chacun de ces groupes portant éventuellement un ou plusieurs substituants identiques ou différents fluoro, cyano, alcoxy en C₁-C₄ ou alcoxy(en C₁-C₄)carbonyle; un groupe alcanoyl(en C₁-C₆)amino ou encore un groupe alcoxy(en C₁-C₆)carbonylamino,
R² représente un atome d'hydrogène, un groupe hydroxyle, un groupe mercapto, un groupe amino, un atome d'halogène; un groupe alkyle en C₁-C₆ ou un groupe alcényle en C2-C6 portant éventuellement un ou plusieurs substituants identiques ou différents fluoro, chloro, bromo, cyano, cycloalkyle en C₃-C₆, alcoxy en C₁-C₄, alkyl(en C₁-C₄)thio ou alcoxy(en C₁-C₄)carbonyle; un groupe cycloalkyle en C₃-C₆ portant éventuellement un ou plusieurs substituants identiques ou différents fluoro, chloro, bromo et/ou alkyle en C₁-C₄; un groupe cyclohexényle; un groupe phénylalkyle en C₁-C₃ portant éventuellement un ou plusieurs substituants identiques ou différents fluoro, chloro, bromo, cyano, nitro, alkyle en C₁-C₄, trifluorométhyle, alcoxy en C₁-C₄ et/ou alcoxy(en C₁-C₄)carbonyle; un groupe phényle portant éventuellement un ou plusieurs substituants identiques ou différents fluoro, chloro, bromo, cyano, nitro, alkyle en C₁-C₄, trifluorométhyle, alcoxy en C₁-C₄, alcoxy en C₁-C₃ portant éventuellement un ou plusieurs substituants identiques ou différents fluoro et/ou chloro, alkyl(en C₁-C₄)thio, alkyl(en C₁-C₃)thio portant éventuellement un ou plusieurs substituants identiques ou différents fluoro et/ou chloro, alkyl(en C₁-C₄)sulfinyle, alkyl(en C₁-C₄)sulfonyle et/ou alcoxy(en C₁-C₄)carbonyle; un groupe alcoxy en C₁-C₆ portant éventuellement un ou plusieurs substituants identiques ou différents fluoro, chloro, cyano, alcoxy en C₁-C₄ ou alcoxy(en C₁-C₄)carbonyle; un groupe alcényl(en C₃-C₆)oxy, un groupe alcynyl(en C₃-C₆)oxy ou un groupe cycloalkyl(en C₃-C₆)oxy; un groupe phénoxy ou un groupe benzyloxy, chacun de ces groupes portant éventuellement un ou plusieurs substituants identiques ou différents fluoro, chloro, bromo, cyano, nitro, alkyle en C₁-C₄, trifluorométhyle, alcoxy en C₁-C₄, difluorométhoxy ou trifluoro-méthoxy; un groupe alkyl(en C₁-C₆)thio, un groupe alkyl(en C₁-C₆)sulfinyle, un groupe alkyl(en C₁-C₆)sulfonyle, un groupe alcényl(en C₃-C₆)thio, un groupe alcynyl(en C₃-C₆)thio ou un groupe cycloalkyl(en C₃-C₆)thio, chacun de ces groupes portant éventuellement un ou plusieurs substituants identiques ou différents fluoro, chloro, cyano, alcoxy en C₁-C₄ ou alcoxy(en C₁-C₄)carbonyle; un groupe phénylthio ou un groupe benzylthio, chacun de ces groupes portant éventuellement un ou plusieurs substituants identiques ou différents fluoro, chloro, bromo, cyano, nitro, alkyle en C₁-C₄, trifluorométhyle, alcoxy en C₁-C₄, difluorométhoxy ou trifluorométhoxy; un groupe alkyl (en C₁-C₆) amino ou un groupe alcényl (en C₃-C₆) amino; un groupe phénylamino ou un groupe benzylamino, chacun de ces groupes portant éventuellement un ou plusieurs substituants identiques ou différents fluoro, chloro, bromo, cyano, nitro, alkyle en C₁-C₄, trifluorométhyle, alcoxy en C₁-C₄, difluorométhoxy ou trifluorométhoxy; un groupe di(alkyl en C₁-C₄) amino; ou encore un groupe aziridino, un groupe pyrrolidino ou un groupe morpholino, chacun de ces groupes portant éventuellement un ou plusieurs substituants identiques ou différents alkyle en C₁-C₄,
R³ représente un groupe alkyle en C₁-C₆, un groupe alcényle en C₃-C₆ ou un groupe alcynyle en C₃-C₆, chacun de ces groupes portant éventuellement un ou plusieurs substituants identiques ou différents halogéno, cyano, carboxyle, alcoxy en C₁-C₄, alkyl (en C₁-C₄) thio, alkyl (en C₁-C₄) sulfinyle, alkyl(en C₁-C₄) sulfonyle ou alcoxy(en C₁-C₄)carbonyle; un groupe cycloalkyle en C₃-C₆ ou un groupe cycloalkyl(en C₃-C₆)alkyle en C₁-C₄, chacun de ces groupes portant éventuellement un ou plusieurs substituants identiques ou différents halogéno, cyano, carboxyle, alkyle en C₁-C₄, halogénalkyle en C₁-C₄ ou alcoxy(en C₁-C₄) carbonyle; un groupe alkyl(en C₁-C₆) sulfonyle, un groupe alkyl(en C₁-C₆)aminosulfonyle ou un groupe di(alkyl en C₁-C₄)aminosulfonyle, chacun de ces groupes portant éventuellement un ou plusieurs substituants identiques ou différents halogéno; un groupe phényle, un groupe naphtyle, un groupe tétralinyle, un groupe phénylalkyle en C₁-C₄, un groupe naphtylalkyle en C₁-C₄ ou un groupe tétralinylalkyle en C₁-C₄, chacun de ces groupes portant éventuellement un ou plusieurs substituants identiques ou différents halogéno, cyano, carboxyle, nitro, un ou plusieurs substituants identiques ou différents alkyle en C₁-C₄, alcoxy en C₁-C₄, alkyl (en C₁-C₄) thio, alkyl(en C₁-C₄) sulfinyle ou alkyl(en C₁-C₄)sulfonyle (chacun de ces substituants portant éventuellement un ou plusieurs substituants identiques ou différents halogéno, cyano, alcoxy en C₁-C₄ ou alcoxy(en C₁-C₄)carbonyle)), un ou plusieurs substituants identiques ou différents alkyl (en C₁-C₄)sulfonyloxy, alkyl (en C₁-C₄) aminosulfonyloxy, di (alkyl en C₁-C₄) aminosulfonyloxy, halogénalkyl(en C₁-C₄) sulfonyloxy, di (alkyl en C₁-C₄)aminocarbonyle ou alkyl (en C₁-C₄)aminocarbonyloxy, un ou plusieurs substituants identiques ou différents cycloalkyle en C₃-C₆ ou cycloalkyl(en C₃-C₆)alkyle en C₁-C₄ (chacun de ces substituants portant éventuellement un ou plusieurs substituants identiques ou différents halogéno ou alkyle en C₁-C₄), un ou plusieurs substituants identiques ou différents phényle, phénoxy, phénylthio, phénylsulfinyle, phénylsulfonyle, phénylsulfonyloxy, phénylamino, phénylcarbonyle ou phénylalkyle en C₁-C₄ (chacun de ces substituants portant éventuellement un ou plusieurs substituants identiques ou différents halogéno, cyano, nitro, alkyle en C₁-C₄, halogénalkyle en C₁-C₄, alcoxy en C₁-C₄, halogénalcoxy en C₁-C₄, alkyl (en C₁-C₄) thio, halogénalkyl(en C₁-C₄) thio, alcoxy(en C₁-C₄)-carbonyle ou alcoxy(en C₁-C₄) amino), un ou plusieurs substituants identiques ou différents alkyl(en C₁-C₄) carbonyle, cycloalkyl(en C₃-C₆) carbonyle ou alcoxy(en C₁-C₄) carbonyle (chacun de ces substituants portant éventuellement un ou plusieurs substituants identiques ou différents halogéno, cycloalkyle en C₃-C₆ ou alcoxy(en C₁-C₄)),
R⁴ représente un atome d'hydrogène, un groupe hydroxyle, un groupe amino, un groupe cyano; un groupe alkyle en C₁-C₆, un groupe alcényle en C₃-C₆ ou un groupe alcynyle en C₃-C₆, chacun de ces groupes portant éventuellement un ou plusieurs substituants identiques ou différents halogéno, cyano, carboxyle, alcoxy en C₁-C₄, alkyl (en C₁-C₄) thio, alkyl (en C₁-C₄) sulfinyle, alkyl(en C₁-C₄)sulfonyle, alkyl(en C₁-C₄) amino, di(alkyl en C₁-C₄) amino ou alcoxy(en C₁-C₄) carbonyle; un groupe alcoxy(en C₁-C₄)carbonyle; un groupe alcoxy en C₁-C₆, un groupe alcényloxy en C₃-C₆, un groupe benzyloxy, un groupe phénoxy ou un groupe di(alcoxy en C₁-C₄)(thio)phosphoryle ou encore le groupement ci-après Q¹-R⁵, où
Q¹ représente -CO- ou -SO₂-, et
R⁵ représente un groupe alkyle en C₁-C₆ portant éventuellement un ou plusieurs substituants identiques ou différents halogéno ou alcoxy en C₁-C₄; un groupe cycloalkyle en C₃-C₆ portant éventuellement un ou plusieurs substituants identiques ou différents halogéno ou alkyle en C₁-C₄; ou encore un groupe phényle portant éventuellement un ou plusieurs substituants identiques ou différents halogéno, alkyle en C₁-C₄, halogénalkyle en C₁-C₄ ou alcoxy en C₁-C₄.

3. Composés de formule (I) selon la revendication 1, caractérisés en ce que, dans ces composés,
Q représente un atome d'oxygène ou le groupement: 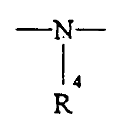
R¹ représente un atome d'hydrogène, un groupe hydroxyle, un groupe amino; un groupe alkylidène(en C₃-C₈)amino; un groupe méthyle, un groupe éthyle, un groupe n- ou i-propyle, un groupe n-, i-, s- ou t-butyle, un groupe dichlorométhyle, un groupe fluoréthyle, un groupe chloréthyle, un groupe difluoréthyle, un groupe dichloréthyle, un groupe trifluoréthyle, un groupe trichloréthyle, un groupe chlorodifluoréthyle, un groupe tétrafluoréthyle, un groupe cyanométhyle, un groupe cyanoéthyle, un groupe méthoxyméthyle, un groupe éthoxyméthyle, un groupe méthoxyéthyle, un groupe éthoxyéthyle; un groupe allyle, un groupe chlorallyle, un groupe dichlorallyle, un groupe propargyle; un groupe cyclopropyle, un groupe benzyle ou un groupe phényle; un groupe méthoxy, un groupe éthoxy, un groupe n- ou i-propoxy; un groupe allyloxy, un groupe n- ou i- ou s-butoxy; un groupe méthylamino, un groupe éthylamino, un groupe n- ou i-propylamino; un groupe cyclopropylamino, un groupe diméthylamino, un groupe diéthylamino, un groupe acétylamino, un groupe méthoxycarbonylamino ou un groupe éthoxycarbonylamino,
R² représente un atome d'hydrogène, un groupe hydroxyle, un groupe mercapto, un groupe amino, un atome de fluor, un atome de chlore, un atome de brome; un groupe méthyle, un groupe éthyle, un groupe n- ou i-propyle, un groupe n-, i-, s- ou t-butyle, un groupe difluorométhyle, un groupe trifluorométhyle, un groupe dichlorométhyle, un groupe trichlorométhyle, un groupe cyanométhyle, un groupe cyanoéthyle, un groupe cyclopropylméthyle, un groupe méthoxyméthyle, un groupe éthoxyméthyle, un groupe méthoxyéthyle, un groupe éthoxyéthyle, un groupe méthylthiométhyle, un groupe éthylthiométhyle, un groupe méthylthioéthyle ou un groupe éthylthioéthyle; un groupe cyclopropyle, un groupe difluorocyclopropyle ou un groupe dichlorocyclopropyle; un groupe phényle ou un groupe benzyle; un groupe méthoxy, un groupe éthoxy, un groupe n- ou i-propoxy; un groupe méthoxyméthoxy, un groupe éthoxyméthoxy, un groupe méthoxyéthoxy ou un groupe éthoxyéthoxy; un groupe phénoxy ou un groupe benzyloxy; un groupe méthylthio, un groupe éthylthio, un groupe n- ou i-propylthio, un groupe allylthio, un groupe propargylthio, un groupe cyclopropylméthylthio, un groupe méthylsulfinyle, un groupe éthylsulfinyle, un groupe méthylsulfonyle, un groupe éthylsulfonyle, un groupe phénylthio ou un groupe benzylthio; un groupe méthylamino, un groupe éthylamino, un groupe n- ou i-propylamino, un groupe phénylamino ou un groupe benzylamino; un groupe diméthylamino, un groupe diéthylamino, un groupe dipropylamino, un groupe méthyléthylamino, un groupe méthylpropylamino; un groupe aziridino; ou encore un groupe pyrrolidino, un groupe pipéridino ou un groupe morpholino, chacun de ces groupes portant éventuellement un ou plusieurs substituants méthyle ou éthyle,
R³ représente un groupe méthyle, un groupe éthyle, un groupe n- ou i-propyle, un groupe n-, i-, s- ou t-butyle, chacun de ces groupes portant éventuellement un ou plusieurs substituants identiques ou différents fluoro, chloro, cyano, méthoxy, éthoxy, méthoxycarbonyle ou éthoxycarbonyle; un groupe cyclopropyle, un groupe cyclobutyle, un groupe cyclopentyle, un groupe cyclohexyle, un groupe cyclopropylméthyle, un groupe cyclobutylméthyle, un groupe cyclopentylméthyle, un groupe cyclohexylméthyle, un groupe cyclopropyléthyle, un groupe cyclobutyléthyle, un groupe cyclopentyléthyle ou un groupe cyclohexyléthyle, chacun de ces groupes portant éventuellement un ou plusieurs substituants identiques ou différents fluoro, chloro, cyano, méthyle, éthyle, trifluorométhyle, méthoxycarbonyle ou éthoxycarbonyle; un groupe méthylsulfonyle, un groupe éthylsulfonyle, un groupe propylsulfonyle ou un groupe butylsulfonyle, chacun de ces groupes portant éventuellement un ou plusieurs substituants identiques ou différents fluoro ou chloro; un groupe phényle portant éventuellement un ou plusieurs substituants identiques ou différents fluoro, chloro, bromo, cyano, carboxyle, nitro, un ou plusieurs substituants identiques ou différents méthyle, éthyle, méthoxy, éthoxy, méthylthio, éthylthio, méthylsulfinyle, éthylsulfinyle, méthylsulfonyle ou éthylsulfonyle (chacun de ces substituants portant éventuellement un ou plusieurs substituants identiques ou différents fluoro, chloro, cyano, méthoxy, éthoxy, méthoxycarbonyle ou éthoxycarbonyle), un ou plusieurs substituants identiques ou différents méthylsulfonyloxy, éthylsulfonyloxy, méthylaminosulfonyloxy, éthylaminosulfonyloxy, diméthylaminosulfonyloxy, diéthylaminosulfonyloxy, trifluorométhylsulfonyloxy, diméthylaminocarbonyle ou diéthylamino-carbonyle, un ou plusieurs substituants identiques ou différents cyclohexyle ou cyclohexylméthyle (chacun de ces substituants portant éventuellement un ou plusieurs substituants identiques ou différents chloro, méthyle ou éthyle), un ou plusieurs substituants identiques ou différents phényle, phénoxy, phénylthio, phénylsulfonyle, phénylsulfonyloxy, phénylamino, phénylcarbonyle, phénylméthyle ou phényléthyle (chacun de ces substituants portant éventuellement un ou plusieurs substituants identiques ou différents fluoro, chloro, cyano, nitro, méthyle, éthyle, trifluorométhyle, méthoxy, éthoxy, difluorométhoxy, trifluorométhoxy, méthylthio, éthylthio, trifluorométhylthio, méthoxycarbonyle ou éthoxycarbonyle, un ou plusieurs substituants identiques ou différents acétyle, cyclopropylcarbonyle, méthoxycarbonyle ou éthoxycarbonyle (chacun de ces substituants portant éventuellement un ou plusieurs substituants identiques ou différents fluoro, chloro, cyclopentyle, cyclohexyle, méthoxy ou éthoxy); un groupe naphtyle ou un groupe tétralinyle, chacun de ces groupes portant éventuellement un ou plusieurs substituants identiques ou différents fluoro, chloro, cyano, nitro, méthyle, éthyle, méthoxy ou éthoxy; ou encore un groupe naphtylméthyle, un groupe naphtyléthyle, un groupe tétralinylméthyle ou un groupe tétralinyléthyle, chacun de ces groupes portant éventuellement un ou plusieurs substituants identiques ou différents fluoro, chloro, méthyle, éthyle, méthoxy ou éthoxy,
R⁴ représente un atome d'hydrogène, un groupe cyano, un groupe cyanométhyle, un groupe cyanoéthyle, un groupe difluorométhyle, un groupe méthoxyméthyle, un groupe éthoxyméthyle, un groupe méthoxyéthyle, un groupe éthoxyéthyle, un groupe méthoxycarbonylméthyle, un groupe éthoxycarbonylméthyle, un groupe méthoxycarbonyléthyle, un groupe éthoxycarbonyléthyle, un groupe allyle, un groupe propargyle, un groupe méthoxycarbonyle, un groupe éthoxycarbonyle, un groupe méthoxy, un groupe éthoxy, un groupe allyloxy ou un groupe benzyloxy ou encore le groupement Q¹-R⁵, où
Q¹ représente -CO- ou -SO₂-, et
R⁵ représente un groupe méthyle, un groupe éthyle ou un groupe propyle, chacun de ces groupes portant éventuellement un ou plusieurs substituants identiques ou différents fluoro, chloro, méthoxy ou éthoxy; un groupe cyclopropyle, un groupe cyclobutyle, un groupe cyclopentyle ou un groupe cyclohexyle, chacun de ces groupes portant éventuellement un ou plusieurs substituants fluoro, chloro, méthyle, éthyle; ou encore un groupe phényle portant éventuellement un ou plusieurs substituants identiques ou différents fluoro, chloro, bromo, méthyle, éthyle, trifluorométhyle, méthoxy ou éthoxy.

4. Les sels de sodium, de potassium, de magnésium, de calcium, d'ammonium, d'alkyl (en C₁-C₄) ammonium, de di (alkyl en C₁-C₄)ammonium, de tri (alkyl en C₁-C₄)ammonium, de cycloalkyl(en C₅ ou en C₆)ammonium et de di(alkyl en C₁-C₂)benzylammonium des composés de formule (I) selon les revendications 1, 2 et 3.

5. Procédé pour la préparation de sulfonylaminocarbonylthiazolinones de formule (I) selon la revendication 1, caractérisé en ce qu'on fait réagir
(a) des chlorosulfonylaminocarbonyltriazolinones répondant à la formule générale (II) 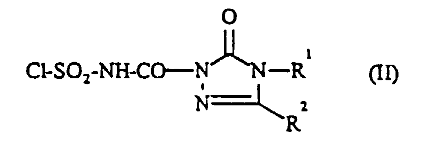 dans laquelle
R¹ et R² ont la signification indiquée à la revendication 1,
avec des composés nucléophiles répondant à la formule générale (III)
R³-Q-H (III)
dans laquelle
Q et R³ ont la signification indiquée à la revendication 1,
éventuellement en présence d'un agent neutralisant les acides et éventuellement en présence d'un diluant,
ou bien en ce qu'on fait réagir
(b) des triazolinones répondant à la formule générale (IV) 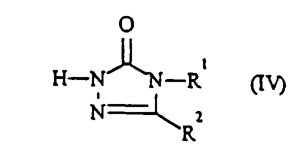 dans laquelle
R¹ et R² ont la signification indiquée à la revendication 1,
avec du chlorosulfonylisocyanate, éventuellement en présence d'un diluant, et on fait réagir les chlorosulfonylaminocarbonyltriazolinones de formule (II) que l'on obtient en l'occurrence, sans isolation intermédiaire, avec des composés nucléophiles répondant à la formule générale (III), éventuellement en présence d'un agent neutralisant les acides et éventuellement en présence d'un diluant,
et on transforme éventuellement en sels les composés de formule (I) obtenus conformément au procédé (a) ou (b), conformément à des procédés habituels.

6. Agents herbicides caractérisés par une teneur en au moins un composé de formule (I) selon la revendication 1.

7. Utilisation de composés répondant à la formule générale (I) selon la revendication 1, pour lutter contre une croissance de plantes indésirable.

8. Procédé pour lutter contre les mauvaises herbes, caractérisé en ce qu'on laisse agir des composés répondant à la formule générale (I) selon la revendication 1, sur les mauvaises herbes ou sur leur biotope.

9. Procédé pour la préparation d'agents herbicides, caractérisé en ce qu'on mélange des composés répondant à la formule générale (I) selon la revendication 1, avec des diluants et/ou avec des agents tensioactifs.

10. Chlorosulfonylaminocarbonyltriazolinones répondant à la formule générale (II) 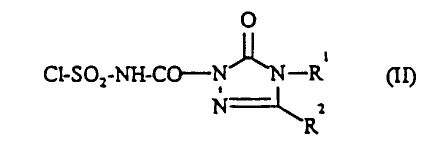 dans laquelle
R¹ représente un atome d'hydrogène, un groupe hydroxyle, un groupe amino, un groupe alkylidène-amino; ou encore un radical choisi parmi la série comprenant un radical alkyle, un radical alcényle, un radical alcynyle, un radical cycloalkyle, un radical aralkyle, un radical aryle, un radical alcoxy, un radical alcényloxy, un radical alkylamino, un radical cycloalkylamino, un radical dialkylamino, un radical alcanoylamino, un radical alcoxycarbonylamino, chacun de ces radicaux portant éventuellement un ou plusieurs substituants identiques ou différents, et
R² représente un atome d'hydrogène, un groupe hydroxyle, un groupe mercapto, un groupe amino, un atome d'halogène; ou encore un radical choisi parmi la série comprenant un radical alkyle, un radical cycloalkyle, un radical alcényle, un radical cycloalcényle, un radical aralkyle, un radical aryle, un radical alcoxy, un radical alcényloxy, un radical alcynyloxy, un radical cycloalkyloxy, un radical aryloxy, un radical aralkyloxy, un radical alkylthio, un radical alkylsulfinyle, un radical alkylsulfonyle, un radical alcénylthio, un radical alcynylthio, un radical cycloalkylthio, un radical arylthio, un radical aralkylthio, un radical alkylamino, un radical alcénylamino, un radical arylamino, un radical aralkylamino, un radical dialkylamino, un radical aziridino, un radical pyrrolidino, un radical pipéridino ou un radical morpholino, chacun de ces radicaux portant éventuellement un ou plusieurs substituants identiques ou différents.
